(19) Europäisches Patentamt
European Patent Office
Office européen des brevets

(11) Publication number: 0 217 142 A2

(12) EUROPEAN PATENT APPLICATION

(21) Application number: 86111938.6

(22) Date of filing: 28.08.86

(51) Int. Cl.⁴: C 07 D 487/04
A 61 K 31/505
//(C07D487/04, 239:00, 231:00),
(C07D487/04, 239:00, 235:00),
(C07D487/04, 249:00, 239:00)

(30) Priority: 30.09.85 JP 218306/85
28.04.86 JP 98499/86

(43) Date of publication of application: 08.04.87 Bulletin 87/15

(84) Designated Contracting States: AT BE CH DE FR GB IT LI NL SE

(71) Applicant: Yoshitomi Pharmaceutical Industries, Ltd.
35 Hiranomachi 3-chome Higashi-ku
Osaka-shi Osaka 541(JP)

(72) Inventor: Tsuda, Yoshinao
645-3, Higashisayamagaoka 1-chome
Tokorozawa-shi Saitama(JP)

(72) Inventor: Mishina, Tadashi
2-102, 1-20, Koyodai 1-chome
Iruma-shi Saitama(JP)

(72) Inventor: Obata, Minoru
4-36, Okidaimachi 1-chome
Nakatsu-shi Oita(JP)

(72) Inventor: Araki, Kazuhiko
1212-4, Aza-Azema Oaza-Kamiikenaga
Nakatsu-shi Oita(JP)

(72) Inventor: Inui, Jun
1129-78, Oaza-Noda
Iruma-shi Saitama(JP)

(72) Inventor: Nakamura, Tadao
468-1, Shimomiyanaga 2-chome
Nakatsu-shi Oita(JP)

(74) Representative: von Kreisler, Alek, Dipl.-Chem. et al,
Deichmannhaus am Hauptbahnhof
D-5000 Köln 1(DE)

(54) A polyazaheterocyclic compound.

(57) A polyazaheterocycle compound of the formula:

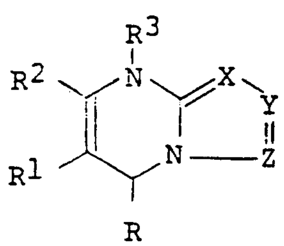

or a pharmaceutically acceptable salt thereof, wherein each symbol is as defined in the specification.

Said compounds exhibit calcium antagonistic and or calcium agonistic activities

EP 0 217 142 A2

Croydon Printing Company Ltd.

0217142

# A polyazaheterocycle compound

## Field of the Invention

The present invention relates to novel and pharmaceutically useful polyazaheterocycle compounds and pharmaceutical composition containing said compounds.

## Prior Art

G. Auzzi et al. disclose a method for preparing 2-phenyl-3-chloro/bromo-4,7-dimethyl-4,7-dihydropyrazolo [1,5-a] pyrimidine-6-carboxylic acid in Il Farmaco, Ed. Sc., vol. 34, 751-758; 1979. European Patent Application No. 183848 discloses polyazaheterocycle compounds.

## Disclosure of the Invention

The present invention relates to polyazaheterocycle compounds of the formula:

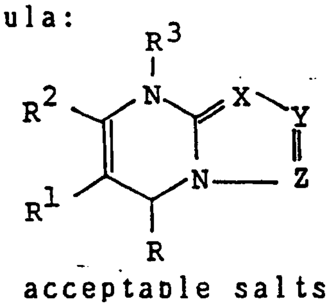

(I)

or a pharmaceutically acceptable salts thereof and pharmaceutical compositions containing said compounds, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and -X-$R^4$ (wherein X is oxygen or sulfur and $R^4$ is $C_{1-8}$ alkyl, $C_{2-10}$ alkenyl, $C_{2-10}$ alkynyl,

haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl, $C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) hetero-aryl, (9) substituted hetero-aryl, (10) aralkyl, (11) substituted aralkyl, (12) arylalkenyl, (13) substituted arylalkenyl, (14) cycloalkylalkyl,(15) cycloalkylalkenyl, (16) cycloalkylalkynyl, (17)heteroarylalkyl, (18) substituted hetero-arylalkyl, (19) benzoyl, (20) substituted benzoyl, $R^1$ is (1) hydrogen, (2) nitro, (3) cyano, (4) acyl, (5) carbamoyl, (6) mono or di-substituted carbamoyl, (7) thiocarbamoyl, (8) monoor di-substituted thiocarbamoyl, (9) $C_{1-8}$ alkylthiocarbonyl, (10) $C_{1-8}$ alkoxythiocarbonyl, (11) $C_{1-8}$ alkyldithiocarbonyl,or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl- $C_{1-8}$ alkyl, halo-alkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, heteroaryl-$C_{1-8}$ alkyl, alkylthio-$C_{1-8}$ alkyl, alkoxycarbonyl-$C_{1-8}$ alkyl, 2,3-epoxypropyl, $-(CH_2)m-$ 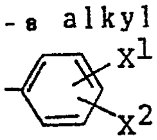 (wherein $X^1$ and $X^2$ are same or different

and each is hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or

$$-(CH_2)_pCH(CH_2)_nR^7$$
$$|$$
$$R^6$$

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$ alkoxy, heteroaryl or $C_{1-8}$ alkoxycarbonyl, and each of n and p is 0 or 1 to 4) ;

$R^2$ is (1) halogen, (2) cyano, (3) $C_{1-8}$ alkyl, (4) haloalkyl, (5) hydroxyalkyl, (6) acyloxyalkyl, (7) alkoxyalkyl, (8) dialkoxyalkyl, (9) aminoalkyl, (10) acylaminoalkyl, (11) formyl, (12) carbamoyloxyalkyl, (13) thiocarbamoyloxyalkyl, (14) amino, (15) substituted amino, (16) aralkyloxyalkyl, (17) aryloxyalkyl, (18) hydroxyiminomethyl, (19) hydroxyalkoxyalkyl, (20) aminoalkoxyalkyl, (21) substituted aminoalkoxyalkyl, (22) $C_{3-8}$ cycloalkyl, (23) cycloalkylalkyl, (24) phenyl, (25) substituted phenyl by at least one substituent selected from halogen, $C_{1-8}$ alkyl, $C_{1-8}$ alkoxy, nitro, cyano, hydroxy, amino and trifluoromethyl, (26) aralkyl, or (27) substituted aralkyl by at least one substituent selected from halogen, $C_{1-8}$ alkyl, $C_{1-8}$ alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl; and at least one of X, Y and Z is N, and other(s) is (are) C-$R^8$, wherein $R^8$ is (1) hydrogen, (2) halogen, (3) nitro, (4) amino, (5) acylamino, (6) $C_{1-8}$ alkyl, (7) $C_{3-8}$ cycloalkyl, (8) cyano, (9) carboxyl, (10) alkoxycarbonyl, (11) carbamoyl, (12) mono or di-substituted carbamoyl, (13) formyl, (14) hydroxyalkyl, (15) aminoalkyl, (16) acyl, (17) haloalkyl, (18) $C_{2-10}$ alkenyl, (19) $C_{2-10}$ alkynyl, (20) cycloalkylalkyl, (21) alkoxyalkyl, (22) acyloxyalkyl, (23) cyanoalkyl, (24) nitroalkyl, (25) carbamoyloxyalkyl, (26) thiocarbamoyloxyalkyl, (27) aralkyloxy, (28) aralkylthio, (29) alkylthio, (30) $C_{1-8}$ alkoxy, or (31) aralkyl;
with the following provisos: (1) when two of X, Y and Z are C-$R^8$, two of $R^8$ are the same or different and each is as defined above, (2) when X and Z are N, $R^2$ is not halogen, amino or substituted amino, (3) when only X is N, $R^8$ is not nitro, amino, $C_{1-8}$ alkyl, carboxyl, formyl, hydroxylalkyl, aminoalkyl, alkoxyalkyl, acyloxyalkyl, nitroalkyl, aralkyloxy, aralkylthio, alkylthio or $C_{1-8}$ alkoxy, and

0217142

(4) each of R, $R^1$, $R^2$, $R^3$ and $R^8$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and $-X-R^4$ (wherein X is oxygen or sulfur, and $R^4$ is $C_{1-4}$ alkyl, aralkyl), $C_{3-8}$ cycloalkyl, furyl, thienyl or pyridyl;

$R^1$: hydrogen, cyano, acyl, carbamoyl or $-COOR^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$ alkyl, heteroaryl-$C_{1-4}$ alkyl, $-(CH_2)m-$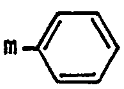 (wherein m is 1 to 4) or $-(CH_2)p\underset{R^6}{\underset{|}{CH}}(CH_2)nR^7$ (wherein $R^6$ is phenyl or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen;

$R^8$: hydrogen.

More specifically stated with the aforementioned generic formula, $C_{1-8}$ alkyl includes, e.g. methyl, ethyl, propyl, isopropyl, butyl, sec-butyl, tert-butyl, pentyl,

neopentyl, 1-ethylpropyl, 1-methylbutyl, tert-pentyl, hexyl, heptyl, octyl, etc.; $C_{2-10}$ alkenyl includes, e.g. vinyl, 2-propenyl, 2-butenyl, 3-butenyl, 1-methyl-2-propenyl, 2-methyl-2-propenyl, 2-pentenyl, 1-methyl-2-butenyl, 1-ethyl-2-propenyl, 1,1-dimethyl-2-propenyl, 3-methyl-2-butenyl, geranyl, etc.; $C_{2-10}$ alkynyl includes, e.g. ethynyl, propargyl, propynyl, 2-butynyl, 1-methyl-2-propynyl, 2-pentynyl, 1-ethyl-2-propynyl, 1,1-dimethyl-2-propynyl, etc.; aryl includes, e.g. phenyl, naphthyl, anthranyl, etc.; halogen includes fluorine, chlorine, bromine, iodine; (mono or di-substituted) amino includes, e.g. amino, methylamino, ethylamino, propylamino, dimethylamino, diethylamino, dipropylamino, 1-pyrrolidinyl, piperidino, morpholino, 1-piperazinyl, 4-methyl-piperazinl-yl, N-methyl-N-benzylamino, etc.; acylamino includes, e.g. acetylamino, propionylamino, butyrylamino, isobutyrylamino, N-methylacetylamino, benzoylamino, etc.; acyl includes, e.g. acetyl, propionyl, butyryl, isobutyryl, benzoyl, etc.; alkoxycarbonyl includes, e.g. methoxycarbonyl, ethoxycarbonyl, propoxycarbonyl, isopropoxycarbonyl, butoxycarbonyl, etc.; haloalkyl includes, e.g. fluoromethyl, bromomethyl, iodomethyl, trifluoromethyl, fluoroethyl, trifluoroethyl, chloromethyl, chloroethyl, chloropropyl, bromopropyl, difluoromethyl, dichloromethyl, dibromomethyl, difluoroethyl, trifluoropropyl, trifluorobutyl, iodoethyl,

chlorobutyl, fluorobutyl, etc.; $C_{3-8}$ cycloalkyl includes, e.g. cyclopropyl, cyclobutyl, cyclopentyl, cyclohexyl, cycloheptyl, etc.; cycloalkylalkyl includes, e.g. cyclopropylmethyl, cyclobutylmethyl, cyclopropylethyl, cyclopentylmethyl, cyclohexylmethyl, cyclopentylethyl, cycloheptylmethyl, cyclohexylethyl, cyclopentylbutyl, cyclohexylpropyl, cyclopropylbutyl, etc.; $C_{1-8}$ alkoxy includes, e.g. methoxy, ethoxy, propoxy, isopropoxy, butoxy, etc.; aralkyl includes,e.g. benzyl, phenylethyl, phenylpropyl, etc.; heteroaryl includes, e.g. pyridyl, thienyl, furyl, pyryl,quinolyl, isoquinolyl, pyrimidinyl, pyridazinyl, quinazolyl, quinoxalyl, benzothienyl, pyrazolyl, imidazolyl, triazolyl, oxadiazolyl, pyrazinyl, thiazolyl, oxazolyl, isothiazolyl,isoxazolyl, thiadiazolyl, indolizinyl, indolyl, benzofuranyl, indazolyl, benzothienyl, benzimidazolyl, benzoxazolyl, benzisoxazolyl, benzthiazolyl, benztriazolyl, 2,1,3-benzoxadiazolyl, cinnolyl, phthalazynyl, naphthyri- dinyl, benztriazinyl, etc.; arylalkenyl includes, e.g. styryl, cinnamyl, phenylbutenyl, etc.; arylalkynyl includes, e.g. phenylpropynyl, phenylbutynyl, etc.; mono or di-substituted carbamoyl includes, e.g. N-methylcarbamoyl, N-ethylcarbamoyl, N-propylcarbamoyl, N-butylcarbamoyl, N,N-dimethylcarbamoyl, N,N-diethylcarbamoyl, N-isopropylcarbamoyl, N-hexylcarbamoyl, N-methyl-N-benzylcarbamoyl, N-benzylcarbamoyl, N-phenylcarbamoyl, pyrrolidinyl-

carbamoyl, piperidinocarbamoyl, morpholinocarbamoyl, N-tert-butylcarbamoyl, N-cyclopentylcarbamoyl, etc.; mono or di-substituted thiocarbamoyl includes, e.g. N-methylthiocarbamoyl, N-ethylthiocarbamoyl, N-propylthiocarbamoyl, N-butylthiocarbamoyl, N,N-dimethylthiocarbamoyl, N,N-diethylthiocarbamoyl, N-isopropylthiocarbamoyl, N-methyl-N-benzylthiocarbamoyl, N-benzylthiocarbamoyl, N-phenylthiocarbamoyl, pyrrolidinylthiocarbamoyl, piperidino- thiocarbamoyl, morpholinothiocarbamoyl, S-methylthiocarbamoyl, S-ethylthiocarbamoyl, S-propylthiocarbamoyl, S-butylthiocarbamoyl, S-isopropylthiocarbamoyl, S-benzylthiocarbamoyl, S-phenylthiocarbamoyl, N-tert-butylthiocarbamoyl, S-tert-butylthiocarbamoyl, N-cyclopentylthiocarbamoyl, S-cyclopentylthiocarbamoyl, etc.; $C_{1-8}$ alkylthiocarbonyl includes, e.g. methylthiocarbonyl, ethylthiocarbonyl, propylthiocarbonyl, isopropylthiocarbonyl, butylthiocarbonyl, isobutylthiocarbonyl, sec-butylthiocarbonyl, tert-butylthiocarbonyl, pentylthiocarbonyl, etc.; $C_{1-8}$ alkoxythiocarbonyl includes, e.g. methoxythiocarbonyl, ethoxythiocarbonyl, propoxythiocarbonyl, isopropoxythiocarbonyl, butoxythiocarbonyl, tert-butoxythiocarbonyl, cyclopentyloxythiocarbonyl, etc.; $C_{1-8}$ alkyldithiocarbonyl includes, e.g. methyldithiocarbonyl, ethyldithiocarbonyl, propyldithiocarbonyl, isopropyldithiocarbonyl, butyldithiocarbonyl,

tert-butyldithiocarbonyl, cyclopentyldithiocarbonyl, etc.; $C_{1-25}$ alkyl includes, e.g. methyl, ethyl, propyl, isopropyl, butyl, isobutyl, sec-butyl, tert-butyl, pentyl, neopentyl, 1-ethylpropyl, 1-methylbutyl, tert-pentyl, hexyl, heptyl, octyl, decyl, cetyl, stearyl, behenyl, etc.; amino-$C_{1-8}$ alkyl includes, e.g. aminomethyl, methylaminomethyl, ethylaminomethyl, propylaminomethyl, isopropylaminomethyl, dimethylaminomethyl, diethylaminomethyl, dipropylaminomethyl, pyrrolidinylmethyl, piperidinomethyl, morpholinomethyl, 4-methylpiperazin-1-ylmethyl, N-methyl-N-benzylaminomethyl, 2-aminoethyl, 2-methylaminoethyl, 2-ethylaminoethyl, 2-propylaminoethyl, 2-dimethylaminoethyl, 2-diethylaminoethyl, 2-dipropylaminoethyl, 2-(1-pyrrolidinyl)ethyl, 2-piperidinoethyl, 2-morpholinoethyl, 2-(1-piperazinyl)ethyl, 2-(4-methylpiperazin-1-yl)ethyl, 2-(N-methyl-N-benzylamino)ethyl, 2-diethylamino-1-methylethyl, 2-piperidino-1-methylethyl, 2-(N-methyl-N-benzylamino)-1-methylethyl, 2-piperidino-1,1-dimethylethyl, 3-diethylaminopropyl, 3-piperidinopropyl, 3-(N-methyl-N-benzylamino)propyl, 3-morpholinopropyl, 4-dimethylaminobutyl, 4-diethylaminobutyl, 4-piperidinobutyl, 4-(2-methoxyphenyl)piperazin-1-ylmethyl, N-methyl-N-[2-(3,4-dimethoxyphenyl)ethyl]aminoethyl, N-[2-(3,4-dimethoxyphenyl)ethyl]aminomethyl, N-methyl-N-[2-(3,4-dihydroxyphenyl)ethyl]aminomethyl, etc.;

alkylthio-$C_{1-8}$ alkyl includes, e.g. 2-methylthioethyl, 2-ethylthioethyl, 2-propylthioethyl, 2-isopropylthioethyl, 3-methylthiobutyl, 3-ethylthiobutyl, 2-methylthio-1-methylethyl, 2-methylthio-1,1-dimethylethyl, etc.; alkoxycarbonyl-$C_{1-8}$ alkyl includes, e.g. methoxycarbonylmethyl, ethoxycarbonylmethyl, propoxycarbonylmethyl, isopropoxycarbonylmethyl, 2-methoxycarbonylethyl, 2-ethoxycarbonylethyl, 1-methoxycarbonylethyl, 1-ethoxycarbonylethyl, 1-methoxycarbonyl-1-methylethyl, 1-ethoxycarbonyl-1-methylethyl, etc.; hydroxyalkyl includes, e.g. hydroxymethyl, hydroxyethyl, hydroxypropyl, hydroxybutyl, etc.; acyloxyalkyl includes, e.g. acetoxymethyl, acetoxyethyl, acetoxypropyl, acetoxybutyl, propionyloxymethyl, butyryloxymethyl, isobutyryloxymethyl, benzoyloxymethyl, etc.; alkoxyalkyl includes, e.g. methoxymethyl, ethoxymethyl, propoxymethyl, isopropoxymethyl, butoxymethyl, methoxyethyl, methoxypropyl, methoxybutyl, propoxypropyl, isopropoxymethyl, etc.; dialkoxyalkyl includes, e.g. dimethoxymethyl, diethoxymethyl, dipropoxymethyl, dibutoxymethyl, etc.; acylaminoalkyl includes, e.g. acetylaminomethyl, acetylaminoethyl, acetylaminopropyl, acetylaminobutyl, acetylaminopentyl, propionylaminomethyl, butyrylaminomethyl, isobutyrylaminoethyl, N-methyl-acetylaminomethyl, etc.; carbamoyloxyalkyl includes, e.g. carbamoyloxymethyl, carbamoyloxyethyl, carbamoyloxypropyl,

cabamoyloxybutyl, N-methylcarbamoyloxymethyl, N-ethylcarbamoyloxymethyl, N-propylcarbamoyloxymethyl, N-butylcarbamoyloxymethyl, N-methylcarbamoyloxyethyl, N-methylcarbamoyloxypropyl, N-methylcarbamoyloxybutyl, N,N-dimethylcarbamoyloxymethyl, N,N-diethylcarbamoyloxyethyl, piperidinocarbonyloxymethyl, etc.; thiocarbamoyloxyalkyl includes, e.g. thiocarbamoyloxymethyl, thiocarbamoyloxyethyl, thiocarbamoyloxypropyl, thiocarbamoyloxybutyl, N-methylthiocarbamoyloxymethyl, N-ethylthiocarbamoyloxymethyl, N-propylthiocarbamoyloxymethyl, N-butylthiocarbamoyloxymethyl, N-methylthiocarbamoyloxyethyl, N-methylthiocarbamoyloxypropyl, N-methylthiocarbamoyloxybutyl, N,N-dimethylthiocarbamoyloxymethyl, N,N-diethylthiocarbamoyloxyethyl, piperidinothiocarbonyloxymethyl, etc.; substituted amino includes, e.g. methylamino, ethylamino, propylamino, dimethylamino, diethylamino, dipropylamino, 1-pyrrolidinyl, piperidino, morpholino, 1-piperazinyl, 4-methylpiperazin-1-yl, N-methyl-N-benzylamino, etc.; aralkyloxyalkyl includes, e.g. benzyloxymethyl, benzyloxyethyl, benzyloxypropyl, etc.; aryloxyalkyl includes, e.g. phenoxymethyl, phenoxyethyl, phenoxypropyl, tolyloxymethyl, tolyloxyethyl, tolyloxypropyl, tolyloxybutyl, xylyloxymethyl, xylyloxyethyl, xylyloxypropyl, etc.; hydroxyalkoxyalkyl includes, e.g. hydroxyethoxymethyl, hydroxyethoxyethyl, hydroxyethoxypropyl, hydroxyethoxy-

butyl, hydroxypropoxymethyl, hydroxybutoxymethyl, etc.; aminoalkoxyalkyl includes, e.g. aminoethoxymethyl, aminoethoxyethyl, aminoethoxypropyl, aminoethoxybutyl, aminopropoxymethyl, aminobutoxymethyl, etc.; substituted aminoalkoxyalkyl includes, e.g. N-methylaminoethoxymethyl, N-methylaminoethoxyethyl, N-butylaminoethoxypropyl, N,N-dimethylaminoethoxybutyl, N-benzylaminopropoxymethyl, N-methyl-N-benzylaminobutoxymethyl, pyrrolidinoethoxymethyl, piperidinoethoxyethyl, morpholinoethoxypropyl, etc.; heteroaroyl includes, e.g. furoyl, thenoyl, nicotinoyl, isonicotinoyl, etc.; arenesulfonyloxyalkyl includes, e.g. tosyloxyethyl, tosyloxypropyl, tosyloxybutyl, etc.; alkanesulfonyloxyalkyl includes, e.g. mesyloxyethyl, mesyloxypropyl, mesyloxybutyl, etc.; cyanoalkyl includes, e.g. cyanomethyl, cyanoethyl, cyanopropyl, cyanobutyl, etc.; nitroalkyl includes, e.g. nitromethyl, nitroethyl, nitropropyl, nitrobutyl, etc.; aralkyloxy includes, e.g. benzyloxy, phenylethyloxy, phenylpropyloxy, phenylbutyloxy, etc.; aralkylthio includes, e.g. benzylthio, phenylethylthio, phenylpropylthio, etc.; alkylthio includes, e.g. methylthio, ethylthio, propylthio, isopropylthio, butylthio, pentylthio, hexylthio, heptylthio, octylthio, etc.; and substituent includes, e.g. halogen, amino, nitro, cyano, $C_{1-8}$ alkyl, trifluoromethyl, hydroxy, $C_{1-8}$ alkoxy, haloalkyl, etc.

The ring in the formula (I) and in the specification of the present invention represented by the formula:

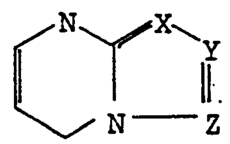

means a 4,7-dihydrotetrazolo [1,5-a] pyrimidine of the formula (a):

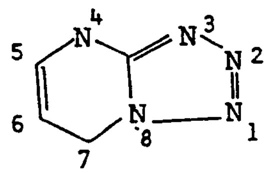

a 4,7-dihydrotriazolo [1,5-a] pyrimidine of the formula (b):

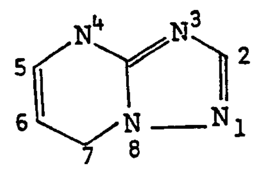

a 5,8-dihydroimidazo [1,2-a] pyrimidine of the formula (c):

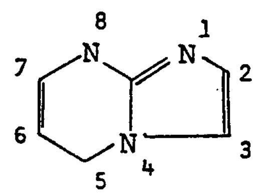

a 1,4-dihydroimidazo [1,5-a] pyrimidine of the formula (d):

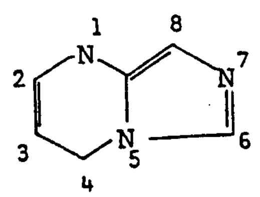

or a 4,7-dihydropyrazolo [1,5-a] pyrimidine of the formula (e):

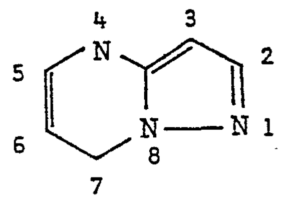

The compounds of formula (I) of the present invention can be, for example, prepared by the following methods.

(1) A method which comprises subjecting a compound of the formula:

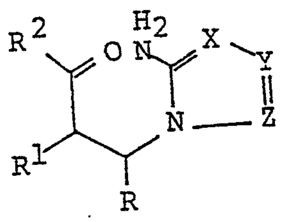

(II)

to dehydration condensation at room temperature or under heating in a suitable solvent or without it.

(2) A method which comprises subjecting a compound of the formula:

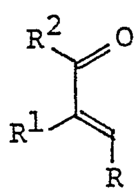

(III)

to condensation with a compound of the formula:

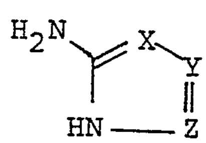

(IV)

at room temperature or under heating in a suitable solvent or without it.

(3) A method which comprises subjecting a compound of the formula:

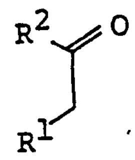

(V)

to dehydration condensation with a compound of the formula:

R-CHO (VI)

at room temperature or under heating in a suitable solvent or without it, if necessary in the presence of a suitable acid catalyst, a base catalyst or both, and then subjecting the compound obtained to condensation with a compound of the formula (IV) at room temperature or under heating in a suitable solvent or without it.

(4) A method which comprises reacting a compound of the formula:

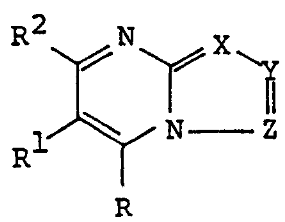

(VII)

with hydrogen gas in the presence of a suitable metal catalyst or a complex metal hydride (e.g. lithium aluminum hydride or sodium borohydride) at room temperature or under heating in a suitable solvent or without it.

(5) A method which comprises converting the substituents

R, $R^1$, $R^2$ and $R^8$ of the compound obtained by the above-mentioned methods (1)-(4) according to usual manners of organic chemical synthesis.

The compounds of the formula (I) wherein $R^3$ is the substituent other than hydrogen can, for example, be prepared by the following method.

(6) A method which comprises reacting a compound of the formula:

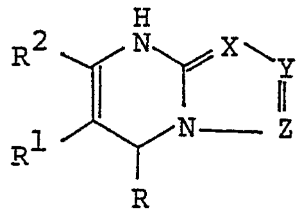

(Ⅷ)

with a compound of the formula:

$R^3$-Y (Ⅸ)

wherein Y is halogen (e.g. chlorine, bromine or iodine) or active ester group (e.g. methanesulfonyloxy, toluenesulfonyloxy or trifluoromethanesulfonyloxy) in a suitable solvent in the presence of a suitable inorganic base (e.g. sodium hydride, sodium amide, sodium methoxide, sodium hydroxide or potassium carbonate) or a suitable organic base (e.g. triethylamine or diisopropylethylamine).

(7) A method which comprises converting the substituents R, $R^1$, $R^2$ and $R^8$ of the compound obtained by the above-mentioned methods (6) according to usual manners of organic chemical synthesis.

Any inert solvent can be employed as a suitable solvent in the methods (1)-(4) and (6), and includes, for example, a lower alkanol (e.g. methanol, ethanol, propanol, isopropanol, butanol, tert-butanol or ethylene glycol), an aromatic hydrocarbon (e.g. benzene, toluene or xylene), an ether (e.g. diethyl ether, diisopropyl ether, dimethoxyethane, diethylene glycol dimethyl ether, tetrahydrofuran or dioxane), a halogenated lower alkane (e.g. dichloromethane, dichloroethane, chloroform or tetrachloromethane), an acid amide (e.g. dimethylformamide, N-methylpyrrolidone or hexamethylphosphorotriamide), a lower alkanecarboxylic acid or an ester thereof (e.g. formic acid, acetic acid, propionic acid, methyl acetate, ethyl acetate, butyl acetate or methyl propionate), a nitrated lower alkane (e.g. nitromethane or nitroethane), dimethylsulfoxide, sulforane, water, or a mixture thereof.

The reactions of the methods (1)-(4) perform at a temperature under 200 ℃, and preferably at temperature of from room temperature to 150℃.

The usual manners of organic chemical synthesis in the methods (5) and (7) include, for example, substitution reaction with an electrophilic reagent (e.g. Mannich reaction, Vilsmeyer reaction, Friedel-Crafts reaction, formation of methylol, halogenation or nitration);

reduction reaction (e.g. reduction of nitro to amino, reduction of a cyano to an aminomethyl, reduction of a carboxylic acid or its ester to a methylol, or reduction of an aldehyde or a ketone to an alcohol); hydrolysis reaction (e.g. hydrolysis of an ester, hydrolysis of an acetal or a ketal, or hydrolysis of an amide or a nitrile); hydrogenolysis; decarboxylation; substitution reaction via a diazonium salt (e.g. Sandmeyer reaction or Schiemann reaction); substitution reaction with a nucleophilic agent (e.g. amination, acetoxylation, halogenation, cyanation, alkoxylation, formation of thioether or thioester, formation of an azide or carbon-carbon bond formation using a carbanion); esterification; thioesterification; transesterification; acylation; sulfonylation; formation of alkylcarbamoyl; formation of alkylthiocarbamoyl; dehydration of an amide to a nitrile; dehydration of an alcohol or a halide to an olefine; rearrangement of an acid azide or an acid amide to an amino derivative; rearrangement of an oxime to an amino derivative; halogenation of an alkyl carbon; and formation of an iminoether from a nitrile.

The compound of formula (I) having an asymmetric carbon atom can be prepared as a racemate or an optically active isomer. The racemate can be devided into a desired optical isomer by means of, for example, a fractional recrystallization of a salt with an optically active base or acid; passing through the column filled with an optically active carrier; producing an ester bond or amide bond with an optically active amine, alcohol or carboxylic acid, and then separating the formed diastereomer by using a fractional recrystallization or column chromatography; carring out the synthetic methodsof (1), (2) and (3) using the starting compounds (the compounds of formulas (Ⅱ), (Ⅲ), (Ⅳ), (Ⅴ), (Ⅵ) and so on), which are combined with an optically active amine, alcohol or carboxylic acid; or reducing the starting compound using an asymmetric reduction reagent or asymmetric reduction catalyst in the synthetic method (4). Furthermore, the compounds of formula (I) having at least two asymmetric carbon atoms can be prepared as an individual diastereomer or a mixture thereof. The optical isomers thus obtained can be led to the optically active compounds of the present invention by the synthetic method (7). The individual diastereomer can be purified by means of fractional recrystallization or chromatography. In the compound (I) having an oxime of the present invention, syn- and anti-isomers can exist. The compounds of the present invention also include the syn- and anti-forms.

The compounds of the formula (I), optical isomers thereof or diastereomers thereof can, if desired, be

converted into acid addition salts thereof with an inorganic acid (e.g. hydrochloric acid, hydrobromic acid, sulfuric acid or phosphoric acid), an organic acid (e.g. acetic acid, propionic acid, glycollic acid, succinic acid, maleic acid, fumaric acid, malic acid, tartaric acid, oxalic acid, citric acid, benzoic acid, cinnamic acid, mandelic acid, salicylic acid, 2-acetoxybenzoic acid, nicotinic acid or isonicotinic acid), an organic sulfonic acid (e.g. methanesulfonic acid, ethanesulfonic acid, 2-hydroxyethanesulfonic acid, benzenesulfonic acid, p-toluenesulfonic acid or naphthalene-2-sulfonic acid), ascorbic acid, lysine or glutamic acid. Furthermore, the compounds (1) having a carboxyl group can also be converted into metal salts thereof (e.g. sodium salt, pottasium salt or aluminum salt) or salts with amines (e.g. a salt with triethylamine).

The compounds of the present invention clearly exhibited not only an increase in coronary blood flow and vertebral blood flow but also cardiotonic activity associated with mild hypotension by intravenous or intraduodenal administration to the anesthetized dogs. These compounds, however, did not influence heart rate. They also increased renal blood flow and exhibited diuretic activity. In the isolated myocardium, the compounds of the present invention prolonged the duration

of action potential.

Furthermore, the present inventors have found that the compounds of the present invention have shown calcium antagonistic and/or calcium agonistic activities depending upon the concentrations of extracellular potassium ion in cardiac muscle and blood vessel. Several compounds showed the organ-selective calcium antagonist and agonist activity, i.e., calcium antagonist in vascular system and calcium agonist in myocardium. Since the compounds possessing these specific pharmacological activities, they seem to be desirable as drugs for the treatment of ischemic heart disease (coronary vasodilators), heart failure (cardiotonics), arrhythmia and the prophylaxis or the treatment of myocardial infarction. In addition, the compounds of the present invention are recognized to have ideal characteristics to treat and improve the multiple cardiovascular disfunction, especially resulted from aging. Furthermore, for cerebral diseases, the compounds of the present inventin are ideal because they preferrentially increased the blood flow of damaged cerebral region with less steal phenomena in acute or chronic phases due to its dependency on extracellular potassium concentration. From the above-mentioned effects in brain, heart and kidney, it can be understood that the compounds of the present invention are also sufficient

as mild anti-hypertensive agents with increasing the blood flow in such vital organs as the heart, brain and kidney. Moreover, the comounds of the present invention can be applied as pressor or anti-shock agents.

The compounds of the present invention, when used as drugs, can be administered in the form of powder, tablets, capsules, granules, injectable solutions, suppositories, ointments or the like by mixing with a suitable carrier, excipient, diluent or the like. The dose for human adults usually ranges from 10 mg to 500 mg, but it may vary depending upon the age, body weight, and severity of the conditions to be treated.

Formulation Example 1 : Tablets

| | 25 mg tablets | 50 mg tablets |
|---|---|---|
| Compounds (I) | 25.0 mg | 50.0 mg |
| Lactose | 59.5 | 67.0 |
| Corn starch | 20.0 | 25.0 |
| Crystalline cellulose | 10.0 | 20.0 |
| Methyl cellulose | 1.0 | 1.5 |
| Talc | 4.0 | 6.0 |
| Magnesium stearate | 0.5 | 0.5 |
| | 120.0 mg | 170.0 mg |

Formulation Example 2 : 10 % Powder

| | |
|---|---|
| Compounds (I) | 10.0 % |
| Lactose | 59.5 |
| Corn starch | 30.0 |
| Talc | 0.5 |
| | 100.0 % |

The present invention will be explained by the following examples in more detail, but these examples are not to be construed as limiting the present invention:

Example 1

A mixture of 10.0 g of cyclopropylmethyl α-acetyl-2-trifluoromethylcinnamate and 2.66 g of 3-aminopyrazole in 30 ml of dimethylformamide is stirred at room temperature for an hour and heated at 90℃ for 5 hours. The resulting mixture is concentrated under reduced pressure and the residue is poured into water. After the solution is extracted with ethyl acetate, the extract is washed with sodium chloride solution and dried over magnesium sulfate and then pale red-orange oil (6.6 g) is purified by chromatography over 70 g of silica gel with chloroform-ethanol (100:1) eluent. The eluate is concentrated and the crystals are recrystallized from ethanol to give 1.7 g of 6-cyclopropylmethyloxycarbonyl-5-methyl-7-(2-trifluoromethylphenyl)-4,7-dihydropyrazolo [1,5-a] pyrimidine, melting at 182-183℃, as a white powder.

Cyclopropylmethyl α-acetyl-2-trifluoromethylcinnamate is synthesized according to the following manner.

A mixture of 10.0 g of cyclopropylmethanol and 11.7 g of diketene in 100 ml of benzene is refluxed under heating for 5 hours. The resulting mixture is concentrated under reduced pressure to give 15 g of cyclopropylmethyl acetoacetate as orange oil. To a mixture of 15 g of cyclopropylmethyl acetoacetate thus obtained and 16.7 g of 2-trifluoromethylbenzaldehyde in 80 ml of benzene are added 0.1 ml of piperidine and 0.1 ml of acetic acid, and the whole mixture is refluxed with heating for 5 hours with removing water. The resultant mixture is washed with sodium chloride solution, neutralized with sodium hydrogencarbonate solution and concentrated under reduced pressure to give 20 g of cyclopropylmethyl α-acetyl-2-trifluoromethylcinnamate as a red-orange oil.

Example 2

A mixture of 3.12 g of cyclopropylmethyl α-acetyl-2-trifluoromethylcinnamate and 1.08 g of 3-amino-4-cyanopyrazole in 27 ml of ethanol is heated at 50℃ for an hour and at 90℃ for 7 hours. The reaction mixture is concentrated under reduced pressure and the resulting oil is crystallized from a mixture of hexane and ethyl acetate to give white powdery cystals. The crystals are purified

by chromatography over silica gel with chloroform-methanol (100:1) eluents and the obtained white granular crystals are recrystallized from a mixture of chloroform and methanol to give 0.5 g of 3-cyano-6-cyclopropylmethyloxy-carbonyl-5-methyl-7-(2-trifluoromethylphenyl)-4,7-dihydropyrazolo (1,5-a) pyrimidine, melting at 223-225℃, as white powdery crystals.

Example 3

A mixture of 2.0 g of ethyl α-acetyl-2-trifluoro-methylcinnamate and 0.76 g of 3-amino-4-cyanopyrazole in 25 ml of dimethylformamide is heated at 40℃ for an hour and at 90℃ for 7 hours. After cooling, the solvent is distilled off and the residual red-orange oil is purified by chromatography over 35 g of silica gel with chloroform eluent. The eluate is concentrated under reduced pressure and 1.1 g of resulting yellow oil is recrystallized from ethanol to give 0.39 g of 3-cyanol-6-ethoxycarbonyl-5-methyl-7-(2-trifluoromethylphenyl)-4,7-dihydropyrazolo-(1,5-a) pyrimidine, melting at 252-254℃, as colorless crystals.

Example 4

A mixture of 0.79 g of 3-aminopyrazole and 3.1 g of cyclopentyl α-acetyl-2-trifluoromethylcinnamate, which is prepared from 1.74 g of 2-trifluoromethylbenzaldehyde and 1.7 g of cyclopentyl acetoacetate, in 25 ml of acetonitrile is heated at 50℃ for an hour and refluxed for 5 hours. The mixture is concentrated under reduced pressure and the resulting oil is purified by chromatography over 390 g of silica gel with chloroform-hexane (100:1) eluents. The eluate is concentrated and the resulting crystals are recrystallized from methanol to give 0.9 g of 6-cyclopentyloxycarbonyl-5-methyl-7-(2-trifluoromethylphenyl)-4,7-dihydropyrazolo [1,5-a] pyrimidine, melting at 159-160℃, as a white powder.

Cyclopentyl acetoacetate can be prepared from cyclopentanol and diketene.

Example 5

A mixture of 1.18 g of 3-amino-4-chloropyrazole and 2.86 g of ethyl α-acetyl-2-trifluoromethylcinnamate in 20 ml of acetonitrile is heated at 60℃ for 30 minutes. Furthermore, to the resulting mixture is added 20 ml of dimethylformamide and the whole mixture is refluxed under heating for 6 hours. After the solvent is distilled off, the residue is purified by column chromatography over silica gel with chloroform eluent. The eluate is concentrated and the residue is crystallized from a mixture of hexane and ethyl acetate to give 0.25 g of 3-chloro-6-ethoxycarbonyl-5-methyl-7-(2-trifluoromethylphenyl)-4,7-dihydropyrazolo [1,5-a] pyrimidine, melting at 199-200℃.

Example 6

A mixture of 1.46 g of ethyl α-acetyl-2-difluoromethoxycinnamate and 0.42 g of 3-aminopyrazole in 20 ml of acetonitrile is refluxed for 4 hours. After the mixture is concentrated, the residue is purified by column chromatography over 40 g of silica gel with hexane-ethyl acetate (2:1) eluent, and crystallized from diisopropyl ether to give 0.36 g of crude crystals.

The crystals are recrystallized from ethyl acetate to give 0.13 g of 7-(2-difluoromethoxyphenyl)-6-ethoxycarbonyl-5-methyl-4,7-dihydropyrazolo [1,5-a] pyrimidine, melting at 191-192℃.

Example 7

A mixture of 4.39 g of 2-(3,4-dimethoxyphenyl)ethyl α-acetyl-2-trifluoromethylcinnamate and 0.91 g of 3-aminopyrazole in 20 ml of dimethylformamide is stirred at 90℃ for 30 minutes and at 110℃ for an hour. The reaction mixture is poured into water and extracted with diethyl ether. The extract is washed with water and dried, and then the solvent is distilled off. The residue (4.69 g) is crystallized from a mixture of hexane and ethyl acetate to give 2.50 g of crystals. The obtained crystals are recrystallized from a mixture of ethyl acetate and diethyl ether to give 1.88 g of 6- [2-(3,4-dimethoxyphenyl)ethoxy] carbonyl-5-methyl-7-(2-trifluoromethylphenyl)-4,7-dihydropyrazolo [1,5-a] pyrimidine,

melting at 182-183.5℃, as white crystals.

Example 8

A mixture of 7 g of 3-pentyl α-acetyl-2-trifluoromethylcinnamate and 1.66 g of 3-aminopyrazole in 10 ml of acetonitrile is refluxed under heating for 8 hours. The solvent is distilled off and the residue is purified by column chromatography over silica gel. The obtained crystals are recrystallized twice from a mixture of diisopropyl ether and hexane to give 3.5 g of 5-methyl-6-(3-pentyloxycarbonyl)-7-(2-trifluoromethylphenyl)-4,7-dihydropyrazolo [1,5-a] pyrimidine, melting at 147-149℃, as colorless crystals.

3-Pentyl α-acetyl-2-trifluoromethylcinnamate can be prepared from 3-pentyl acetoacetate and 2-trifluorobenzaldehyde by using a similar procedure in Example 1.

Example 9

A mixture of 2.76 g of cyclopentyl α-acetyl-2-fluorocinnamate and 0.83 g of 3-aminopyrazole is warmed at 50℃ for 5 minutes in water bath and then to the mixture is added 40 ml of acetonitrile. The whole mixture is refluxed for 8.5 hours and concentrated. The residue is purified by column chromatography over 30 g of silica gel with chloroform eluent to give 0.97 g of crude crystals. The crystals are recrystallized from diisopropyl ether to give 0.66 g of 6-cyclopentyloxycarbonyl-7-(2-fluorophenyl)-5-methyl-4,7-dihydropyrazolo [1,5-a]-pyrimidine hemihydrate, melting at 115-117℃.

Example 10

A mixture of 2.25 g of isopropyl α-acetyl-2-fluorocinnamate and 0.97 g of 3-amino-4-cyanopyrazole is refluxed under heating for 7 hours. After the reaction mixture is concentrated, to the residue is added 35 ml of dimethylformamide and then the mixture is further refluxed for 6 hours. After the solvent is distilled off, the resulting red-orange oil is purified by column chromatography with dichloromethane eluent, and the eluate is concentrated. The residue is further purified by column chromatography with dichloromethane-hexane (100:1) eluent, and crystallized from diisopropyl ether to give 0.37 g of the crystals. The crystals are recrystallized from methanol to give 0.21 g of 3-cyano-7-(2-fluorophenyl)-6-isopropoxycarbonyl-5-methyl-4,7-dihydropyrazolo [1,5-a] pyrimidine, melting at 218-219℃, as colorless powdery crystals.

Example 11

A mixture of 1.5 g of isopropyl α-acetyl-2,3-difluorocinnamate and 0.6 g of 3-amino-4-cyanopyrazole in 25 ml of acetonitrile is refluxed under heating for 3 hours. After the mixture is concentrated under reduced pressure, the resulting red-orange oil is purified by

column chromatography over 25 g of silica gel with chloroform eluent. The pale yellow oil is crystallized from diethyl ether. The obtained crystals are collected by filtration and recrystallized from a mixture of ethyl acetate and hexane to give 0.1 g of 3-cyano-7-(2,3-difluorophenyl)-6-isopropoxycarbonyl-5-methyl-4,7-dihydropyrazolo [1,5-a] pyrimidine, melting at 191-193℃, as white powdery crystals.

Example 12

A mixture of 13 g of ethyl α-acetyl-3-nitrocinnamate and 4.0 g of 2-amino-4-methylimidazole in 80 ml of ethanol is heated at 50℃ for 2 hours and then at 60℃ for 4 hours. After cooling, the precipitated yellow crystals are recrystallized twice from a mixture of ethanol and dimethylformamide to give 1.2 g of 2,7-dimethyl-6-ethoxycarbonyl-5-(3-nitrophenyl)-5,8-dihydroimidazo [1,2-a] pyrimidine, melting at 246-247℃.

Example 13

A mixture of 1 g of ethyl 5-aminoimidazole-4-carboxylate and 1.5 g of methyl α-acetyl-3-nitrocinnamate in 50 ml of ethanol is stirred at 60-70℃ for 5 hours under nitrogen atmosphere. The mixture is concentrated under reduced pressure, and to the residue is added ethanol. The precipitated crystals are collected by filtration to give 1.5 g of 8-ethoxycarbonyl-3-methoxycarbonyl-2-methyl-4-(3-nitrophenyl)-1,4-dihydroimidazo [1,5-a] pyrimidine, melting at 110℃.

Thus obtained 8-ethoxycarbonyl-3-methoxycarbonyl-2-methyl-4-(3-nitrophenyl)-1,4-dihydroimidazo [1,5-a] pyrimidine (1.5 g) is added to a mixture of 0.15 g of paraformaldehyde, 0.43 g of diethylamine and 15 ml of acetic acid, and the mixture is stirred at 80℃ for 10 hours. The solution is concentrated under reduced pressure, and to the residue is added water and the solution is neutralized with sodium hydrogencarbonate. The mixture is extracted with chloroform, and then the extract is washed with water, dried over magnesium sulfate and concentrated under reduced pressure. The resulting oily substances are purified by column chromatography over silica gel and converted into the hydrochloride by adding ethanolic hydrochloric acid. The precipitated crystals are recrystallized from a mixture of acetone and ether to give 0.14 g of 6-diethylaminomethyl-8-ethoxycarbonyl-3-methoxycarbonyl-2-methyl-4-(3-nitrophenyl)-1,4-dihydroimidazo-[1,5-a] pyrimidine hydrochloride monohydrate, melting at 193-194℃ with decomposition, as pale yellow crystals.

Example 14

A mixture of 9.7 g of ethyl α-acetyl-3-nitrocinnamate and 3.4 g of 3-amino-1,2,4-triazole in 150 ml of ethanol is refluxed under heating with stirring for

10 hours. On cooling, the precipitated crystals are collected by filtration and recrystallized from methanol to give 6-ethoxycarbonyl-5-methyl-7-(3-nitrophenyl)-4,7-dihydro-1,2,4-triazolo [1,5-a] pyrimidine, melting at 222-224℃. The obtained 6-ethoxycarbonyl-5-methyl-7-(3-nitrophenyl)-4,7-dihydro-1,2,4-triazolo [1,5-a] pyrimidine (5.6 g) is dissolved in 50 ml of acetic acid under heating and to the mixture is added dropwise a solution of 3 g of bromine in 15 ml of acetic acid with stirring at 50-55℃ for 40 minutes. Furthermore, after stirring at 45-55℃ for 4.5 hours, the solvent is distilled off and to the residue is added water. The mixture is extracted with chloroform and the extract is washed with water, sodium hydrogencarbonate solution and ice. After drying over anhydrous magnesium sulfate, the solvent is distilled off under reduced pressure. A mixture of 8.2 g of 5-bromomethyl-6-ethoxycarbonyl-7-(3-nitrophenyl)-1,2,4-triazolo-[1,5-a] pyrimidine thus obtained and 4.4 g of diethylamine in 50 ml of ethanol is refluxed for an hour. After the solvent is distilled off under reduced pressure, to the residue is added water and the solution is extracted with ethyl acetate. The ethyl acetate layer is extracted with 3N-hydrochloric acid and the water layer is neutralized with potassium carbonate and then extracted with ethyl acetate. The extract is washed with water, dried

over anhydrous magnesium sulfate and then the solvent is distilled off under reduced pressure. The residue is treated with a mixture of diisopropyl ether and ethanol, and the precipitated crystals are converted into the hydrochloride by adding ethanolic hydrochloric acid. The crystals are recrystallized from ethanol to give 5-diethylaminomethyl-6-ethoxycarbonyl-7-(3-nitrophenyl)-1,2,4-triazolo [1,5-a] pyrimidine hydrochloride, melting at 199-200°C with decomposition.

Example 15

A mixture of 13.1 g of ethyl α-acetyl-3-nitro-cinnamate and 4.3 g of 5-aminotetrazole in 200 ml of ethanol is refluxed under heating for 24 hours. On cooling, white crystals are precipitated and collected by filtration to give 13.4 g of 6-ethoxycarbonyl-5-methyl-7-(3-nitrophenyl)-4,7-dihydrotetrazolo [1,5-a] pyrimidine, melting at 210-212℃. A solution of 5 g of the obtained crystals in 80 ml of acetic acid is heated to 80℃. To the solution is added dropwise a solution of 2.4 g of bromine in 10 ml of acetic acid with stirring. After stirring at 50℃ for 3 hours, the solvent is distilled off under reduced pressure. The resulting oil is dissolved in ethyl acetate and the organic layer is washed with sodium hydrogencarbonate solution and sodium chloride solution, and then dried over sodium sulfate. After the

solvent is distilled off, to the residue are added 30 ml of ethanol and 3.7 g of diethylamine, and the mixture is refluxed under heating for 2 hours. After the solvent is distilled off, a solution of the resulting oil in chloroform is extracted with 2N-hydrochloric acid seven times. The extracted water layers are combined, made alkaline with potassium carbonate, and then extracted with ethyl acetate. The organic layer is dried over sodium sulfate and the solvent is distilled off. The residue is converted into the hydrochloride by adding ethanolic hydrochloric acid and the hydrochloride is recrystallized from ethanol to give 2.7 g of 5-diethylaminomethyl-6-ethoxycarbonyl-7-(3-nitrophenyl)-4,7-dihydrotetrazolo [1,5-a]-pyrimidine, melting at 201-203℃ with decomposition, as pale yellow crystals.

The compounds summarized in the following tables can be also prepared in a similar manner as the above examples.

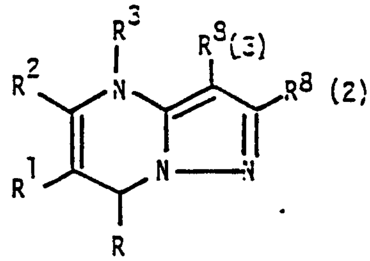

| No. | R | $R^1$ | $R^2$ | $R^3$ | $R^8$ (2) | $R^8$ (3) | M.p. (°C) |
|---|---|---|---|---|---|---|---|
| 16 | 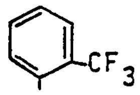 | $CH_3OOC$ | $CH_3$ | H | H | $COOC_2H_5$ | 118-120 |
| 17 | 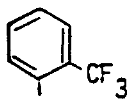 | $CH_3OOC$ | $CH_3$ | H | H | Br | 186-187 |
| 18 | 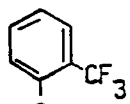 | $CH_3OOC$ | $CH_3$ | H | H | $NO_2$ | 247-249 |
| 19 | 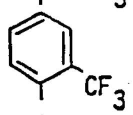 | $CH_3OOC$ | $CH_3$ | H | H | CN | 268-270 |
| 20 | 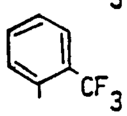 | $CH_3OOC$ | $CH_3$ | H | H | $CONH_2$ | 278-280 |
| 21 | 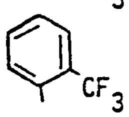 | $FCH_2CH_2OOC$ | $CH_3$ | H | H | H | 185-187 |
| 22 | 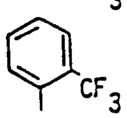 | $CH_3OOC$ | $CH_3$ | H | H | COOH | 216-217 |
| 23 | 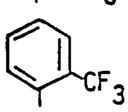 | $O_2N$ | $CH_3$ | H | H | $COOC_2H_5$ | 188-190 |
| 24 | 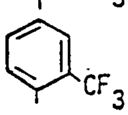 | $ClCH_2CH_2OOC$ | $CH_3$ | H | H | H | 189-190 |
| 25 | 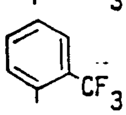 | $C_2H_5OOC$ | $CH_3$ | H | H | CO- 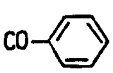 | 119-121 |
| 26 | 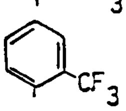 | $CF_3CH_2OOC$ | $CH_3$ | H | H | H | 191-193 |

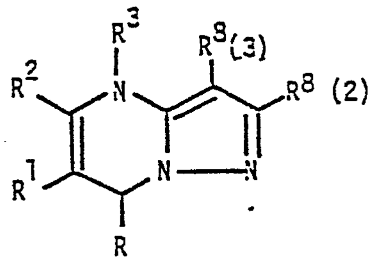

| No. | R | $R^1$ | $R^2$ | $R^3$ | $R^8$ (2) | $R^8$ (3) | M.p. (°C) |
|---|---|---|---|---|---|---|---|
| 27 | 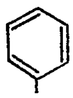 | 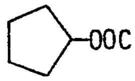 | $CH_3$ | H | H | H | 234-235 |
| 28 | 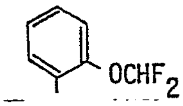 | $(CH_3)_3COOC$ | $CH_3$ | H | H | H | 138-140 |
| 29 | 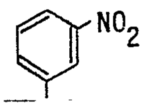 | $(CH_3)_2CHOOC$ | $CH_3$ | H | H | CN | 218-220 |
| 30 | 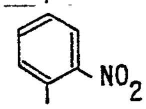 | $(CH_3)_2CHOOC$ | $CH_3$ | H | H | CN | 224-226 |
| 31 | 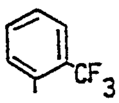 | $C_2H_5OOC$ | $CH_3$ | H | 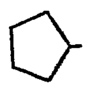 | H | 194-195 |
| 32 | 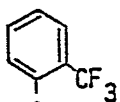 | $O_2N$ | $CH_3$ | H | H | H | 239-240 |
| 33 | 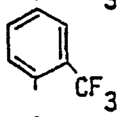 | 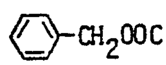 | $CH_3$ | $CH_2OC_2H_5$ | H | H | 79- 80 |
| 34 | 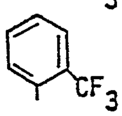 | HOOC | $CH_3$ | $CH_2OC_2H_5$ | H | H | 155-157(d) |
| 35 | 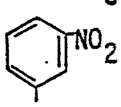 | $C_2H_5OOC$ | $(C_2H_5)_2NCH_2$ | H | H | H | 138-140 |
| 36 | 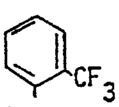 | $C_2H_5OOC$ | $CH_3$ | H | H | $CH_2N(C_2H_5)_2$ | 190-192(d) |
| 37 | 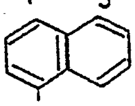 | $(CH_3)_2CHOOC$ | $CH_3$ | H | H | H | 194-195 |
| 38 | 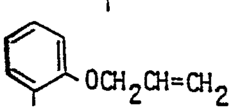 | $C_2H_5OOC$ | $CH_3$ | H | H | H | 203-204 |

(d: decomposition)

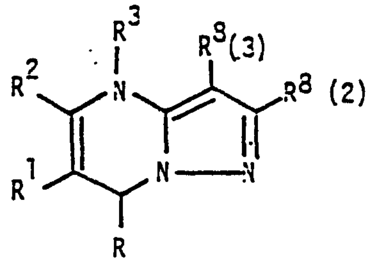

| No. | R | $R^1$ | $R^2$ | $R^3$ | $R^8$ (2) | $R^8$ (3) | M.p. (°C) |
|---|---|---|---|---|---|---|---|
| 39 | 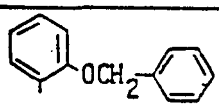 | 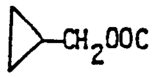 | $CH_3$ | H | H | H | 176-178 |
| 40 | 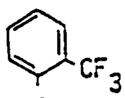 | 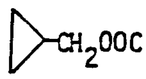 | $CH_3$ | H | H | $NO_2$ | 214-215 |
| 41 | 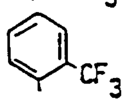 | $C_2H_5OOC$ | $CH_3$ | H | H | Br | 187-188 |
| 42 | 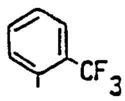 | $CH_3(CH_2)_7OOC$ | $CH_3$ | H | H | H | 104-105 |
| 43 | 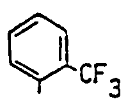 | $CH_3(CH_2)_5OOC$ | $CH_3$ | H | H | H | 105-107 |
| 44 | 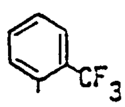 | $C_2H_5OOC$ | $CH_3$ | H | H | $COOC_2H_5$ | 116-118 |
| 45 | 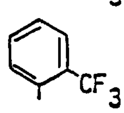 | $CH_2=CHCH_2OOC$ | $CH_3$ | H | H | H | 159-160 |
| 46 | 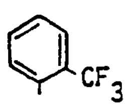 | 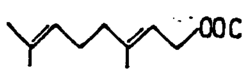 | $CH_3$ | H | H | H | 108-109 |
| 47 | 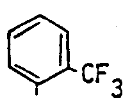 | $CH\equiv CCH_2OOC$ | $CH_3$ | H | H | H | 169-171 |
| 48 | 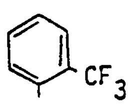 | $CH_3S(CH_2)_2OOC$ | $CH_3$ | H | H | H | 173-175 |
| 49 | 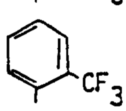 | $CH_3(CH_2)_4OOC$ | $CH_3$ | H | H | H | 1/2 $H_2O$ 115-117 |
| 50 | 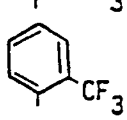 | 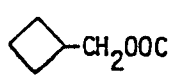 | $CH_3$ | H | H | H | 170-171 |

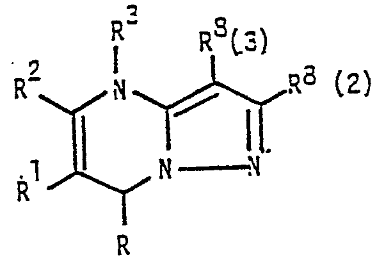

| No. | R | $R^1$ | $R^2$ | $R^3$ | $R^8$ (2) | $R^8$ (3) | M.p. (°C) |
|---|---|---|---|---|---|---|---|
| 51 | 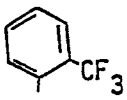 | $Cl(CH_2)_4OOC$ | $CH_3$ | H | H | H | 153-154 |
| 52 | 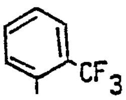 | 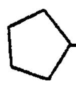 $-CH_2OOC$ | $CH_3$ | H | H | H | 158-159 |
| 53 | 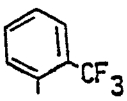 | 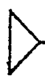 $-CH_2OOC$ | $CH_3$ | H | H | $NH_2$ | 169-171 |
| 54 | 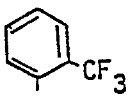 | 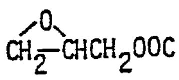 | $CH_3$ | H | H | H | 186-187 |
| 55 | 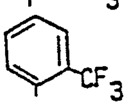 | $CH_3(CH_2)_{21}OOC$ | $CH_3$ | H | H | H | 107-110 |
| 56 | 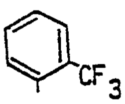 | 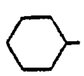 $-CH_2OOC$ | $CH_3$ | H | H | H | 178-180 |
| 57 | 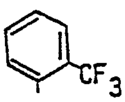 | $(CH_3)_3CCH_2OOC$ | $CH_3$ | H | H | H | 180-181 |
| 58 | 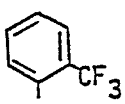 | $CH_3CH_2-C(CH_3)_2-OOC$ | $CH_3$ | H | H | H | 159-161 |
| 59 | 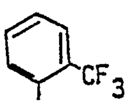 | 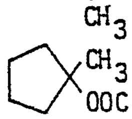 | $CH_3$ | H | H | H | 145-147 |
| 60 | 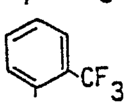 | $C_2H_5OOC$ | $CH_3$ | H | H | $NO_2$ | 195-198 |
| 61 | 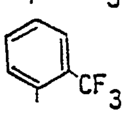 | $C_2H_5OOC$ | $CH_3$ | H | H | $CONH_2$ | 290-292 |
| 62 | 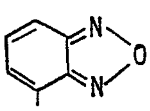 | $C_2H_5OOC$ | $CH_3$ | H | H | H | 204-205 |

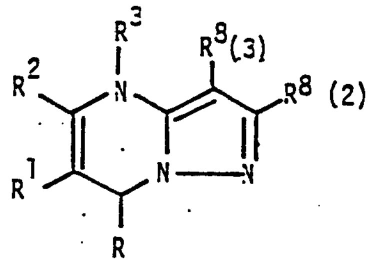

| No. | R | $R^1$ | $R^2$ | $R^3$ | $R^8$ (2) | $R^8$ (3) | M.p. (°C) |
|---|---|---|---|---|---|---|---|
| 63 | 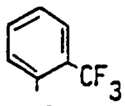 | $(CH_3)_2CHOOC$ | $CH_3$ | $CH_3$ | H | H | 136-137 |
| 64 | 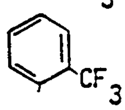 | $(CH_3)_2CHS\underset{O}{\underset{\|}{C}}$ | $CH_3$ | H | H | H | 183-185 |
| 65 | 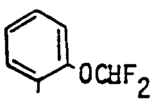 | $CH_3OOC$ | $CH_3$ | H | H | H | 175-177 |
| 66 | 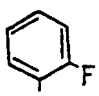 | $(CH_3)_3COOC$ | $CH_3$ | H | H | Br | 173-174 |
| 67 | 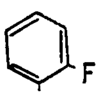 | $(CH_3)_3COOC$ | $CH_3$ | H | $CH_3$ | H | 185-186 |
| 68 | 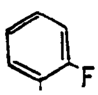 | $(CH_3)_3COOC$ | $CH_3$ | H | H | Cl | 163-164 |
| 69 | 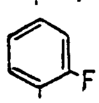 | $(CH_3)_2CHOOC$ | $CH_3$ | H | H | $CH_3$ | 219-220 |
| 70 | 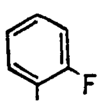 | $(CH_3)_2CHOOC$ | $CH_3$ | H | H | $CH_2OH$ | 155-156(d) |
| 71 | 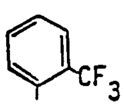 | $(CH_3)_2CHOOC$ | $CH_3$ | H | H | Cl | 171-172 |
| 72 | 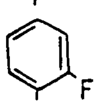 | $(CH_3)_2CHOOC$ | $CH_3$ | H | H | $CH(CH_3)_2$ | 186-188 |
| 73 | 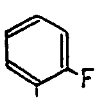 | $(CH_3)_2CHOOC$ | $CH_3$ | H | F | H | 167-168 |
| 74 | 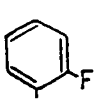 | $C_2H_5OOC$ | 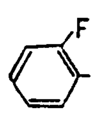 | H | H | H | 211-212 |

(d: decomposition)

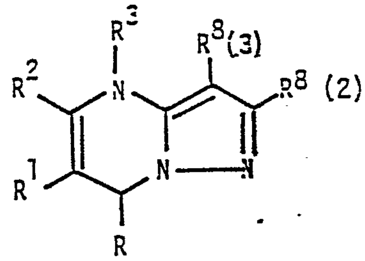

| No. | R | $R^1$ | $R^2$ | $R^3$ | $R^8$ (2) | $R^8$ (3) | M.p. (°C) |
|---|---|---|---|---|---|---|---|
| 75 | 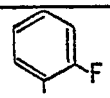 | $(CH_3)_2CHOOC$ | $CH_3$ | H | H | $NO_2$ | 175-177 |
| 76 | 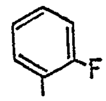 | 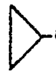 $-CH_2OOC$ | $CH_3$ | H | H | H | 177-179 |
| 77 | 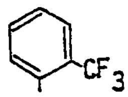 | $(CH_3)_2CHOOC$ | $CH_3$ | $COOC_2H_5$ | H | H | 60- 61 |
| 78 | 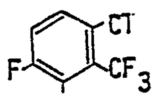 | $C_2H_5OOC$ | $CH_3$ | H | H | H | 204-205.5 |
| 79 | 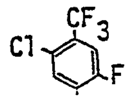 | $(CH_3)_2CHOOC$ | $CH_3$ | H | H | H | 201.5-202.5 |
| 80 | 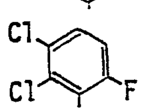 | $C_2H_5OOC$ | $CH_3$ | H | H | H | 196-198 |
| 81 | 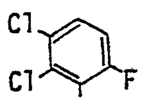 | $(CH_3)_2CHOOC$ | $CH_3$ | H | H | H | 178-181 |
| 82 | 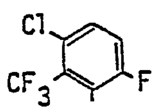 | $(CH_3)_2CHOOC$ | $CH_3$ | H | H | H | 211-212 |
| 83 | 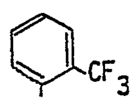 | $(ClCH_2)_2CHOOC$ | $CH_3$ | H | H | H | 186-187 |
| 84 | 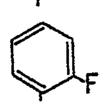 | $ClCH_2CH_2OOC$ | $CH_3$ | H | H | H | 142-144 |
| 85 | 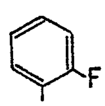 | $(CH_3)_2CHOOC$ | $CH_3$ | H | H | COOH | 198-200(d) |
| 86 | 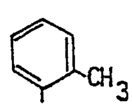 | 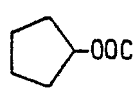 -OOC | $CH_3$ | H | H | H | 182-183 |

(d: decomposition)

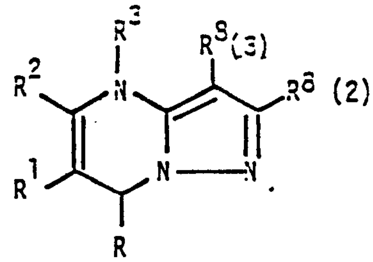

| No. | R | $R^1$ | $R^2$ | $R^3$ | $R^8$ (2) | $R^8$ (3) | M.p. (°C) |
|---|---|---|---|---|---|---|---|
| 87 | 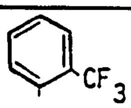 | 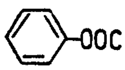 | $CH_3$ | H | H | H | 157-158 |
| 88 | 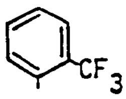 | 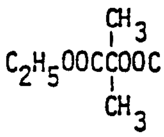 | $CH_3$ | H | H | H | 155-157.5 |
| 89 | 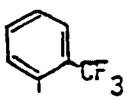 | $CH_3(CH_2)_{15}OOC$ | $CH_3$ | H | H | H | 91- 93 |
| 90 | 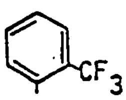 | 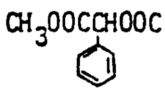 | $CH_3$ | H | H | H | α:187-189<br>β: |
| 91 | 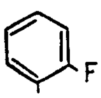 | $(CH_3)_2CHOOC$ | 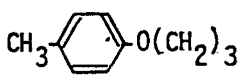 | H | H | H | 134-136 |
| 92 | 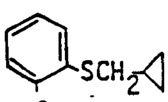 | $C_2H_5OOC$ | $CH_3$ | H | H | H | 173-175 |
| 93 | 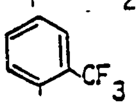 | $C_2H_5OOC$ | $CH_3$ | H | H | $C_3H_7$ | 131-133 |
| 94 | 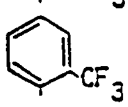 | $C_2H_5OOC$ | $CH_3$ | H | H | $CH_3$ | 203-204 |
| 95 | 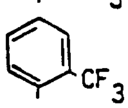 | 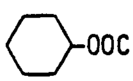 | $CH_3$ | H | H | H | 186-187 |
| 96 | 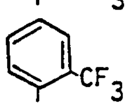 | $C_2H_5OOC$ | $CH_3$ | H | $CH_3$ | H | 207-208 |
| 97 | 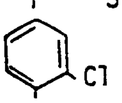 | 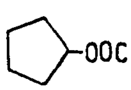 | $CH_3$ | H | H | H | 156-157 |
| 98 | 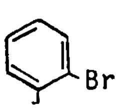 | 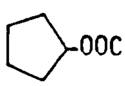 | $CH_3$ | H | H | H | 172-173 |

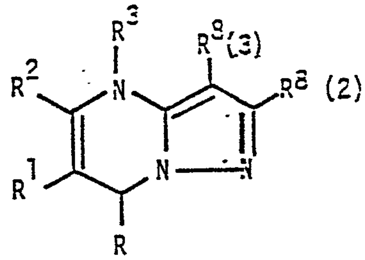

| No. | R | $R^1$ | $R^2$ | $R^3$ | $R^8$ (2) | $R^8$ (3) |
|---|---|---|---|---|---|---|
| 99 | 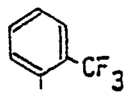 | $(CH_3)_2CHNHOC$ | $CH_3$ | H | H | H |
| 100 | 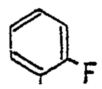 | $(C_2H_5)_2NOC$ | $CH_3$ | H | H | H |
| 101 | 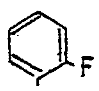 | $H_2NSC$ | $CH_3$ | H | H | H |
| 102 | 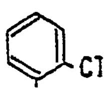 | 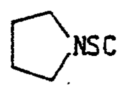 | $CH_3$ | H | H | H |
| 103 | 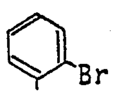 | $(CH_3)_3CNHSC$ | $CH_3$ | H | H | H |
| 104 | 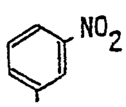 | 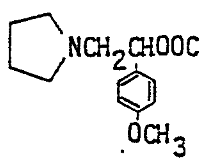 | $CH_3$ | H | H | H |
| 105 | 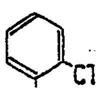 | $(CH_3)_2CHOOC$ | $CH_3$ | $CH_2CH_2Cl$ | H | H |
| 106 | 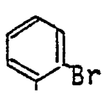 | $(CH_3)_3COOC$ | $CH_3$ | $(CH_2)_3OSO_2CH_3$ | H | H |
| 107 | 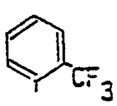 | 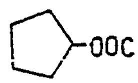 | $CH_3$ | 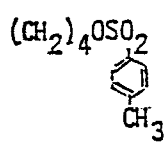 | H | H |
| 108 | 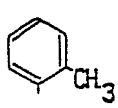 | $C_2H_5OOC$ | $CH_3$ | $CH_2CH_2OH$ | H | H |
| 109 | 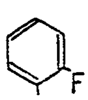 | $(CH_3)_2CHOOC$ | $CH_3$ | 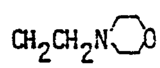 | H | H |
| 110 | 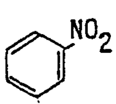 | $CH_3OOC$ | $CH_3$ | $(CH_2)_3N(C_2H_5)_2$ | H | H |

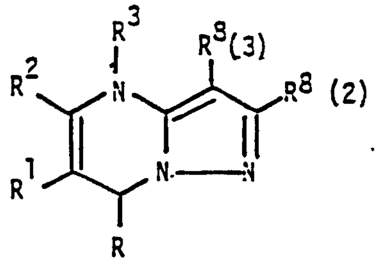

| No. | R | $R^1$ | $R^2$ | $R^3$ | $R^8$ (2) | $R^8$ (3) |
|---|---|---|---|---|---|---|
| 111 | 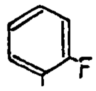 | $(CH_3)_2CHOOC$ | $CH_3$ | H | H | 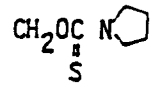 |
| 112 | 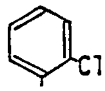 | $C_2H_5OOC$ | $CH_3$ | H | H | $CON(C_2H_5)_2$ |
| 113 | 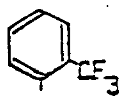 | $(CH_3)_2CHOOC$ | $CH_3$ | H | $CH_2CH=CH_2$ | H |
| 114 | 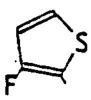 | $(CH_3)_2CHOOC$ | $CH_3$ | H | H | H |
| 115 | 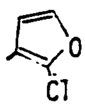 | $C_2H_5OOC$ | $CH_3$ | H | H | H |
| 116 | 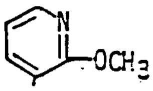 | $(CH_3)_2CHOOC$ | $CH_3$ | H | H | H |
| 117 | 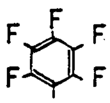 | $(CH_3)_2CHOOC$ | $CH_3$ | H | H | H |
| 118 | 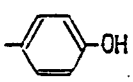 | $CH_3OOCCH_2CHOOC$ 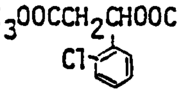 | $CH_3$ | H | H | H |
| 119 | 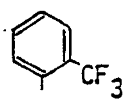 | 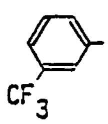 $-(CH_2)_5OOC$ | $CH_3$ | H | H | H |
| 120 | 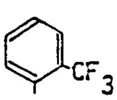 | $C_2H_5OOC$ | 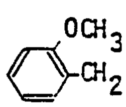 | H | H | H |
| 121 | 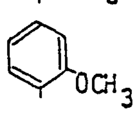 | $CH_3OOC$ | 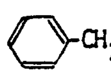 $-CH_2CH_2$ | H | H | H |

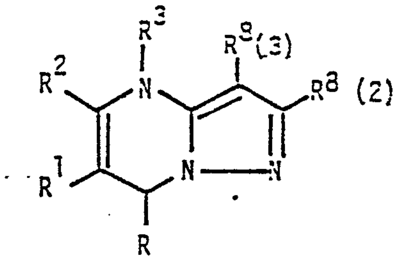

| No. | R | $R^1$ | $R^2$ | $R^3$ | $R^8$ (2) | $R^8$ (3) |
|---|---|---|---|---|---|---|
| 122 | 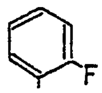 | $(CH_3)_2CHOOC$ | $CH_3$ | H | H | F |
| 123 | 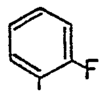 | $(CH_3)_2CHOOC$ | $CH_2N(C_2H_5)_2$ | H | H | H |
| 124 | 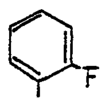 | $(CH_3)_2CHOOC$ | $CH_3$ | H | H | $CH_2OCOCH_3$ |
| 125 | 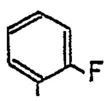 | $(ClCH_2)_2CHOOC$ | $CH_3$ | H | H | H |
| 126 | 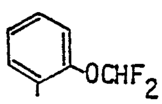 | $(CH_3)_3COOC$ | $CH_3$ | H | H | H |
| 127 | 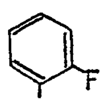 | $(CH_3)_2CHOOC$ | 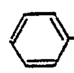 $CH_2OCH_2CH_2$ | H | H | H |
| 128 | 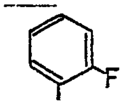 | $(CH_3)_2CHOOC$ | $CH_3$ | H | H | $COCH_3$ |
| 129 | 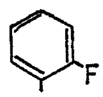 | $(CH_3)_2CHOOC$ | $CH_3$ | H | H | 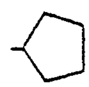 |
| 130 | 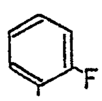 | $C_2H_5OOC$ | $H_2N$ | H | H | H |
| 131 | 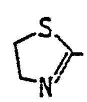 | $CH_3OOC$ | $CH_3$ | H | H | H |
| 132 | 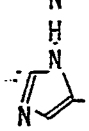 | 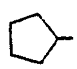 OOC | $CH_3$ | H | H | H |
| 133 | 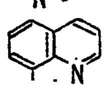 | $(CH_3)_2CHOOC$ | $CH_3$ | H | H | H |

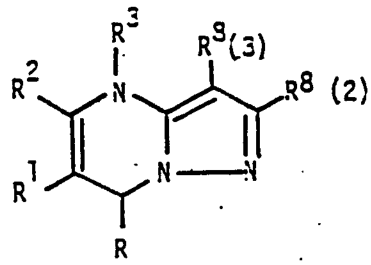

| No. | R | $R^1$ | $R^2$ | $R^3$ | $R^8$ (2) | $R^8$ (3) |
|---|---|---|---|---|---|---|
| 134 | 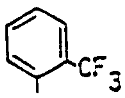 | $C_2H_5OOC$ | Cl | H | H | H |
| 135 | 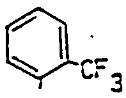 | $C_2H_5OOC$ | $(C_2H_5)_2NCH_2$ | H | H | H |
| 136 | 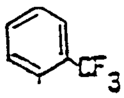 | $C_2H_5OOC$ | $CH_3$ | H | H | F |
| 137 | 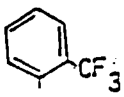 | $C_2H_5OOC$ | $CH_3$ | H | H | $CH_2OH$ |
| 138 | 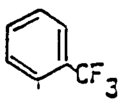 | $C_2H_5OOC$ | $CH_3$ | H | $CH_3$ | $CH_3$ |
| 139 | 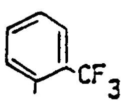 | $C_2H_5OOC$ | $CH_3$ | H | H | $NHCOCH_3$ |
| 140 | 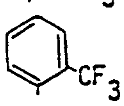 | $C_2H_5OOC$ | $CH_3$ | H | F | H |
| 141 | 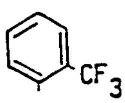 | $C_2H_5OOC$ | $CH_3$ | $CH_3$ | H | H |
| 142 | 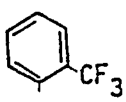 | $C_2H_5OOC$ | $CH_3$ | $CH_2CH_2$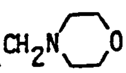 | H | H |
| 143 | 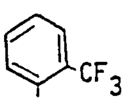 | $C_2H_5OOC$ | $CH_3$ | $CH_2$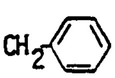 | H | H |
| 144 | 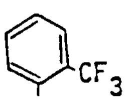 | $C_2H_5OOC$ | 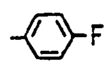 | H | H | H |
| 145 | 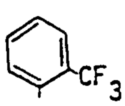 | $H_2NSC$ | $CH_3$ | H | H | H |

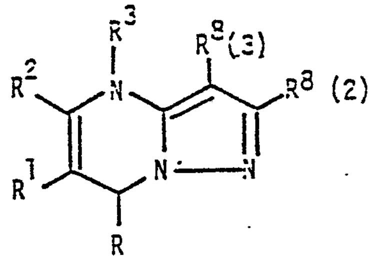

| No. | R | $R^1$ | $R^2$ | $R^3$ | $R^8$ (2) | $R^8$ (3) |
|---|---|---|---|---|---|---|
| 146 | 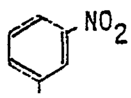 | $(CH_3)_2CHOOC$ | $CH_3$ | H | H | CN |
| 147 | 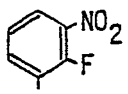 | $(CH_3)_2CHCH_2OOC$ | $CH_3$ | H | H | CN |
| 148 | 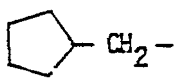 | $CH_3OOC$ | $CH_3$ | H | H | H |
| 149 | 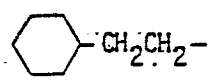 | 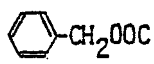 | $CH_3$ | H | H | H |
| 150 | 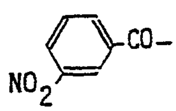 | $CH_3OCH_2CH_2OOC$ | $CH_3$ | H | H | H |
| 151 | 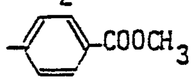 | $CH_3OOC$ | $CH_3$ | H | H | H |
| 152 | 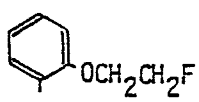 | $(CH_3)_2CHOOC$ | $CH_3$ | H | H | H |
| 153 | 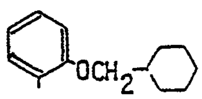 | 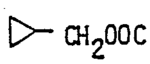 | $CH_3$ | H | H | H |
| 154 | 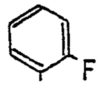 | $(CH_3)_2CHOSC$ | $CH_3$ | H | H | H |
| 155 | 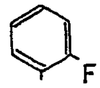 | $(CH_3)_2CHSSC$ | $CH_3$ | H | H | H |
| 156 | 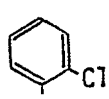 | 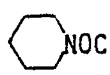 | $CH_3$ | H | H | H |

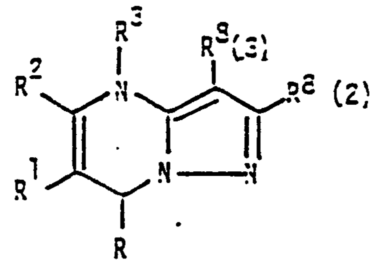

| No. | R | $R^1$ | $R^2$ | $R^3$ | $R^8$ (2) | $R^8$ (3) |
|---|---|---|---|---|---|---|
| 157 | 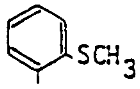 | $(CH_3)_2CHOOC$ | 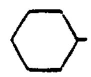 | H | H | H |
| 158 | 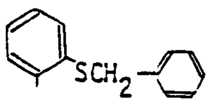 | $CH_3(CH_2)_2OOC$ | 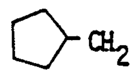 | H | H | H |
| 159 | 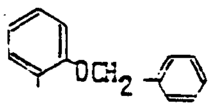 | $CH_3(CH_2)_3OOC$ | $H_2NCH_2CH_2$ | H | H | H |
| 160 | 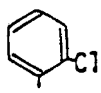 | $CH_3(CH_2)_3OOC$ | $CH_3CONHCH_2CH_2$ | H | H | H |
| 161 | 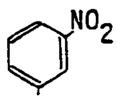 | $(CH_3)_2CHCH_2OOC$ | $HOCH_2CH_2OCH_2$ | H | H | H |
| 162 | 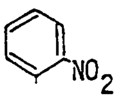 | $C_2H_5OOC$ | $H_2N(CH_2)_2OCH_2$ | H | H | H |
| 163 | 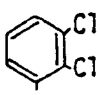 | $CH_3OOC$ | 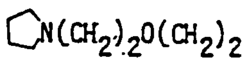 | H | H | H |
| 164 | 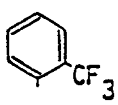 | $(CH_3)_2CHOOC$ | $CH_3$ | $C_3H_7$ | H | H |
| 165 | 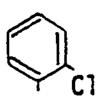 | $(CH_3)_3COOC$ | $CH_3$ | 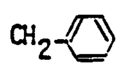 | H | H |
| 166 | 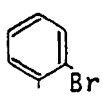 | $C_3H_7OOC$ | $CH_3$ | 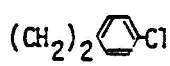 | H | H |
| 167 | 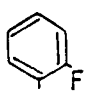 | 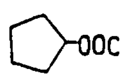 | $CH_3$ | 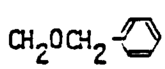 | H | H |
| 168 | 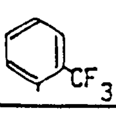 | $CH_3(CH_2)_3OOC$ | $CH_3$ | $COCH_3$ | H | H |

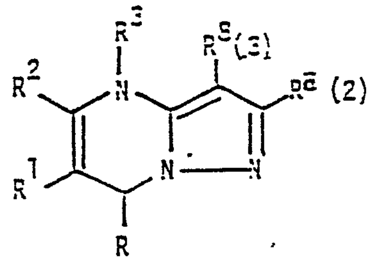

| No. | R | $R^1$ | $R^2$ | $R^3$ | $R^8$ (2) | $R^8$ (3) |
|---|---|---|---|---|---|---|
| 169 | 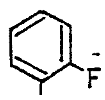 | $(CH_3)_2CHOOC$ | $CH_3$ | H | H | $CH_2F$ |
| 170 | 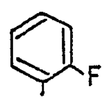 | $(CH_3)_3COOC$ | $CH_3$ | H | H | $CH_2CF_3$ |
| 171 | 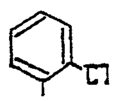 | $(CH_3)_2CHOOC$ | $CH_3$ | H | $CHF_2$ | H |
| 172 | 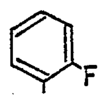 | 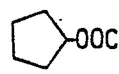-OOC | $CH_3$ | H | H | $CH_2CH=CH_2$ |
| 173 | 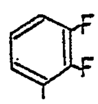 | $(CH_3)_2CHOOC$ | $CH_3$ | H | H | $C\equiv CH$ |
| 174 | 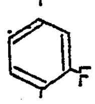 | $C_2H_5OOC$ | $CH_3$ | H | H | $CH_2OCH_2CH_3$ |
| 175 | 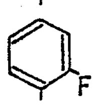 | $(CH_3)_2CHOOC$ | $CH_3$ | H | H | $CH_2$- 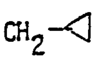 |
| 176 | 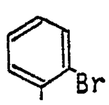 | $CH_3OOC$ | $CH_3$ | H | H | $(CH_2)_3CN$ |
| 177 | 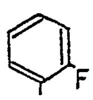 | 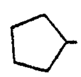-OOC | $CH_3$ | H | H | $CH_2CH_2NO_2$ |
| 178 | 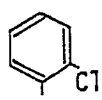 | $CH_3(CH_2)_3OOC$ | $CH_3$ | H | H | $CH_2O\underset{O}{C}NH_2$ |
| 179 | 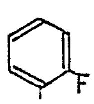 | $(CH_3)_2CHOOC$ | $CH_3$ | H | H | $CH_2O\underset{O}{C}N(CH_3)_2$ |
| 180 | 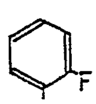 | $(CH_3)_2COOC$ | $CH_3$ | H | H | $CH_2O\underset{S}{C}NH_2$ |

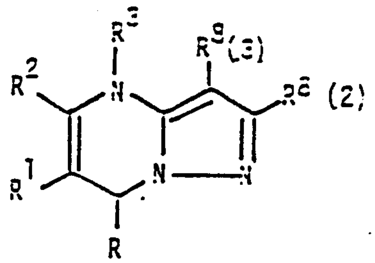

| No. | R | $R^1$ | $R^2$ | $R^3$ | $R^8$ (2) | $R^8$ (3) |
|---|---|---|---|---|---|---|
| 181 | 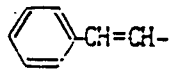 | $(CH_3)_2CHOOC$ | $CH_3$ | H | H | H |
| 182 | 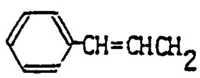 | $(CH_3)_3COOC$ | $CH_3$ | H | H | H |
| 183 | 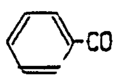 | $C_2H_5OOC$ | $CH_3$ | H | H | H |
| 184 | $CH_3CH=C-$<br>$CH_3$ | $(CH_3)_2CHOOC$ | $CH_3$ | H | H | H |
| 185 | $CH\equiv CCH_2-$ | $(CH_3)_3COOC$ | $CH_3$ | H | H | H |
| 186 | 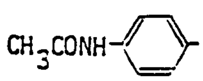 | $C_2H_5OOC$ | $CH_3$ | H | H | H |
| 187 | 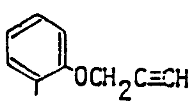 | $(CH_3)_2CHOOC$ | $CH_3$ | H | H | H |
| 188 | 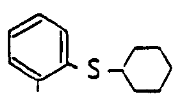 | $C_2H_5OOC$ | $CH_3$ | H | H | H |
| 189 | 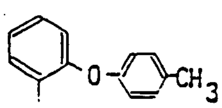 | $CH_3CH_2CH_2OOC$ | $CH_3$ | H | H | H |
| 190 | 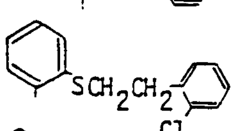 | $CH_3(CH_2)_3OOC$ | $CH_3$ | H | H | H |
| 191 | 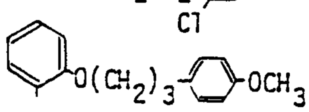 | 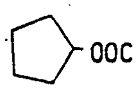 | $CH_3$ | H | H | H |
| 192 | 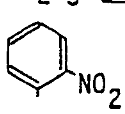 | $(CH_3)_2CHOOC$ | $CH_3$ | H | H | CN |

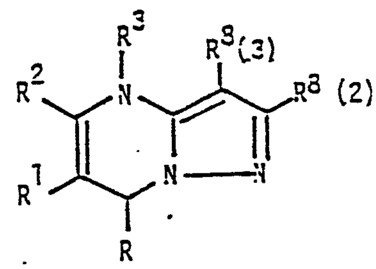

| No. | R | $R^1$ | $R^2$ | $R^3$ | $R^8$ (2) | $R^8$ (3) |
|---|---|---|---|---|---|---|
| 193 | 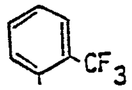 | 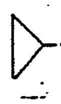 $-CH_2OOC$ | $CH_3$ | H | H | $NHCOCH_3$ |
| 194 | 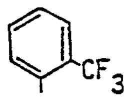 | 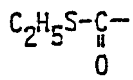 | $CH_3$ | H | H | H |
| 195 | 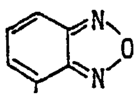 | $O_2N$ | $CH_3$ | H | H | H |
| 196 | 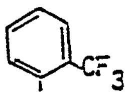 | $C_2H_5OOC$ | $(C_2H_5)_2N$ | H | H | H |

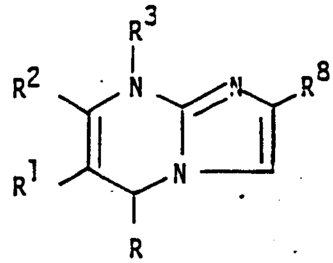

| No. | R | $R^1$ | $R^2$ | $R^3$ | $R^8$ | M.p. (°C) |
|---|---|---|---|---|---|---|
| 197 | 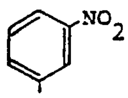 | $C_2H_5OOC$ | $CH_3$ | H | $CH_2OH$ | 220-221(d) |
| 198 | 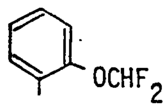 | $(CH_3)_3COOC$ | $CH_3$ | H | H | |
| 199 | 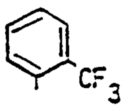 | 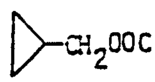 | $CH_3$ | H | H | |
| 200 | 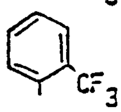 | 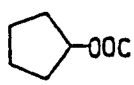 | $CH_3$ | H | H | |
| 201 | 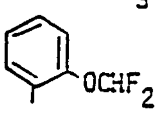 | $C_2H_5OOC$ | $CH_3$ | H | H | |
| 202 | 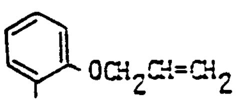 | $C_2H_5OOC$ | $CH_3$ | H | H | |
| 203 | 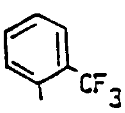 | $FCH_2CH_2OOC$ | $CH_3$ | H | H | |
| 204 | 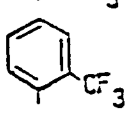 | $ClCH_2CH_2OOC$ | $CH_3$ | H | H | |
| 205 | 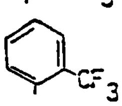 | $CF_3CH_2OOC$ | $CH_3$ | H | H | |
| 206 | 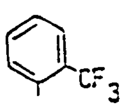 | $CH_3(CH_2)_7OOC$ | $CH_3$ | H | H | |
| 207 | 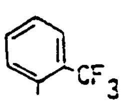 | $CH_3(CH_2)_5OOC$ | $CH_3$ | H | H | |

(d: decomposition)

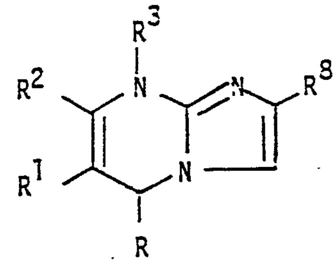

| No. | R | $R^1$ | $R^2$ | $R^3$ | $R^8$ |
|---|---|---|---|---|---|
| 208 | 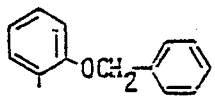 | 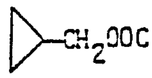 | $CH_3$ | H | H |
| 209 | 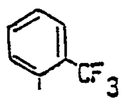 | $CH_2=CHCH_2OOC$ | $CH_3$ | H | H |
| 210 | 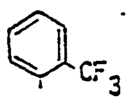 | 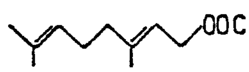 | $CH_3$ | H | H |
| 211 | 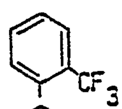 | $O_2N$ | $CH_3$ | H | H |
| 212 | 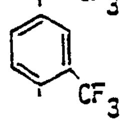 | 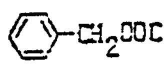 | $CH_3$ | $CH_2OC_2H_5$ | H |
| 213 | 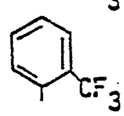 | HOOC | $CH_3$ | $CH_2OC_2H_5$ | H |
| 214 | 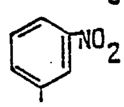 | $C_2H_5OOC$ | $(C_2H_5)_2NCH_2$ | H | H |
| 215 | 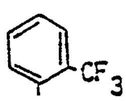 | $CH\equiv CCH_2OOC$ | $CH_3$ | H | H |
| 216 | 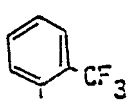 | $CH_3S(CH_2)_2OOC$ | $CH_3$ | H | H |
| 217 | 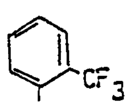 | $CH_3(CH_2)_4OOC$ | $CH_3$ | H | H |
| 218 | 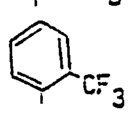 | 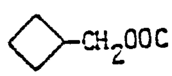 | $CH_3$ | H | H |

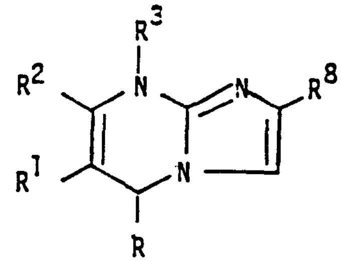

| No. | R | $R^1$ | $R^2$ | $R^3$ | $R^8$ |
|---|---|---|---|---|---|
| 219 | 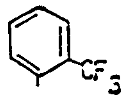 | $CH_3(CH_2)_{21}OOC$ | $CH_3$ | H | H |
| 220 | 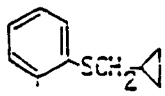 | $C_2H_5OOC$ | $CH_3$ | H | H |
| 221 | 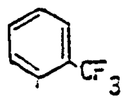 | 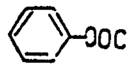 | $CH_3$ | H | H |
| 222 | 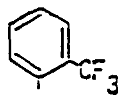 | 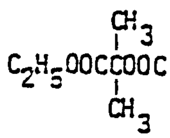 | $CH_3$ | H | H |
| 223 | 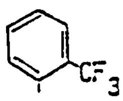 | $CH_3(CH_2)_{15}OOC$ | $CH_3$ | H | H |
| 224 | 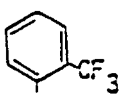 | 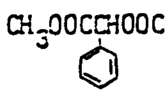 | $CH_3$ | H | H |
| 225 | 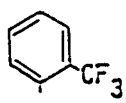 | 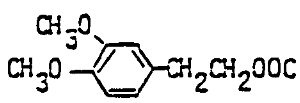 | $CH_3$ | H | H |
| 226 | 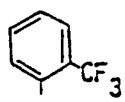 | 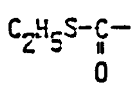 | $CH_3$ | H | H |
| 227 | 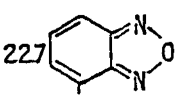 | $O_2N$ | $CH_3$ | H | H |
| 228 | 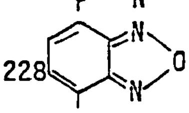 | $C_2H_5OOC$ | $CH_3$ | H | H |
| 229 | 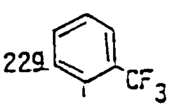 | 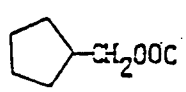 | $CH_3$ | H | H |

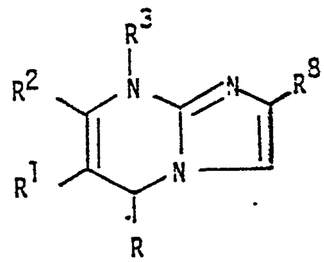

| No. | R | $R^1$ | $R^2$ | $R^3$ | $R^8$ |
|---|---|---|---|---|---|
| 230 | 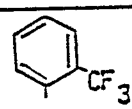 | 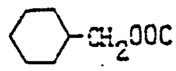 | $CH_3$ | H | H |
| 231 | 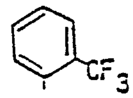 | $(CH_3)_3CCH_2OOC$ | $CH_3$ | H | H |
| 232 | 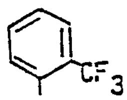 | 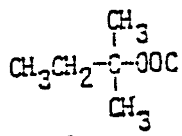 | $CH_3$ | H | H |
| 233 | 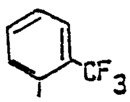 | 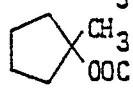 | $CH_3$ | H | H |
| 234 | 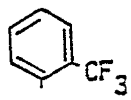 | $C_2H_5OOC$ | $CH_3$ | $CH_3$ | H |
| 235 | 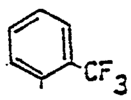 | $C_2H_5OOC$ | $CH_3$ | 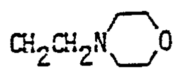 | H |
| 236 | 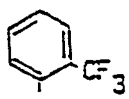 | 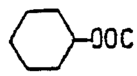 | $CH_3$ | H | H |
| 237 | 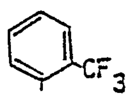 | $H_2NSC$ | $CH_3$ | H | H |
| 238 | 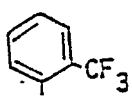 | $C_2H_5OOC$ | $CH_3$ | 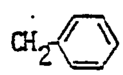 | H |
| 239 | 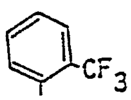 | $C_2H_5OOC$ | 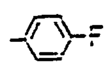 | H | H |
| 240 | 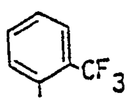 | $C_2H_5OOC$ | Cl | H | H |
| 241 | 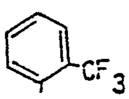 | $C_2H_5OOC$ | $(C_2H_5)_2NCH_2$ | H | H |

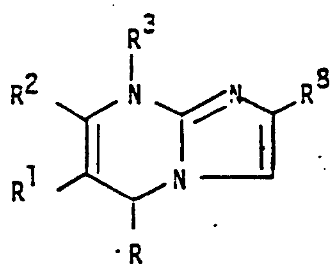

| No. | R | $R^1$ | $R^2$ | $R^3$ | $R^8$ |
|---|---|---|---|---|---|
| 242 | 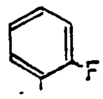 | $(CH_3)_2CHOOC$ | $CH_3$ | H | F |
| 243 | 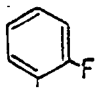 | $C_2H_5OOC$ | 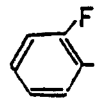 | H | H |
| 244 | 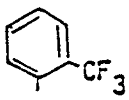 | 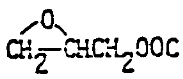 | $CH_3$ | H | H |
| 245 | 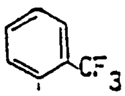 | $C_2H_5OOC$ | $(C_2H_5)_2N$ | H | H |
| 246 | 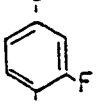 | 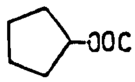 | $CH_3$ | H | H |
| 247 | 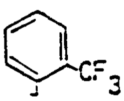 | $(C_2H_5)_2CHOOC$ | $CH_3$ | H | H |
| 248 | 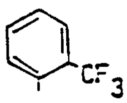 | $(CH_3)_2CHOOC$ | $CH_3$ | $CH_3$ | H |
| 249 | 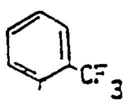 | $(CH_3)_2CHS\underset{O}{\underset{\|}{C}}$ | $CH_3$ | H | H |
| 250 | 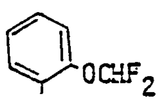 | $CH_3OOC$ | $CH_3$ | H | H |
| 251 | 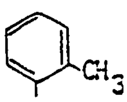 | 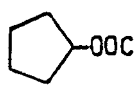 | $CH_3$ | H | H |
| 252 | 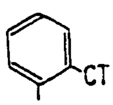 | 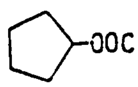 | $CH_3$ | H | H |
| 253 | 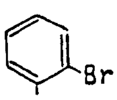 | 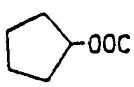 | $CH_3$ | H | H |

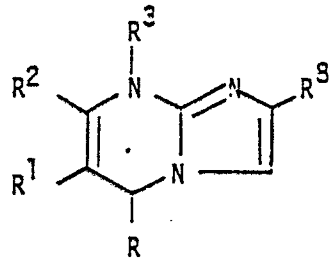

| No. | R | $R^1$ | $R^2$ | $R^3$ | $R^8$ |
|---|---|---|---|---|---|
| 254 | 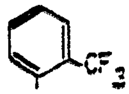 | $(ClCH_2)_2CHOOC$ | $CH_3$ | H | H |
| 255 | 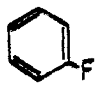 | $ClCH_2CH_2OOC$ | $CH_3$ | H | H |
| 256 | 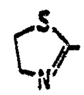 | $CH_3OOC$ | $CH_3$ | H | H |
| 257 | 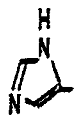 | 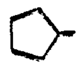 -OOC | $CH_3$ | H | H |
| 258 | 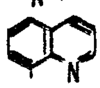 | $(CH_3)_2CHOOC$ | $CH_3$ | H | H |
| 259 | 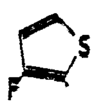 | $(CH_3)_2CHOOC$ | $CH_3$ | H | H |
| 260 | 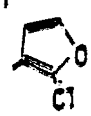 | $C_2H_5OOC$ | $CH_3$ | H | H |
| 261 | 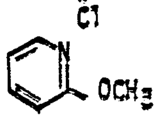 | $(CH_3)_2CHOOC$ | $CH_3$ | H | H |
| 262 | 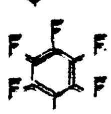 | $(CH_3)_2CHOOC$ | $CH_3$ | H | H |
| 263 | 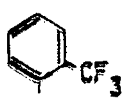 | $(CH_3)_2CHNHOC$ | $CH_3$ | H | H |
| 264 | 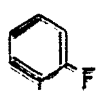 | $(C_2H_5)_2NOC$ | $CH_3$ | H | H |
| 265 | 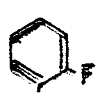 | $H_2NOC$ | $CH_3$ | H | H |

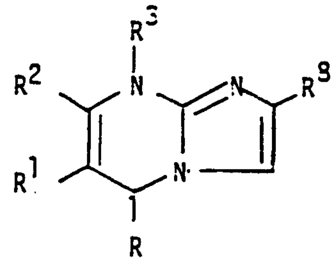

| No. | R | $R^1$ | $R^2$ | $R^3$ | $R^8$ |
|---|---|---|---|---|---|
| 266 | 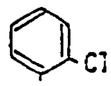 | 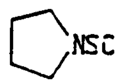 | $CH_3$ | H | H |
| 267 | 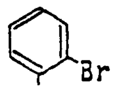 | $(CH_3)_3CNHSC$ | $CH_3$ | H | H |
| 268 | 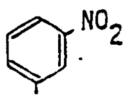 | 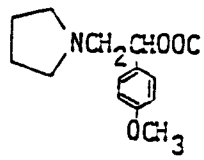 | $CH_3$ | H | H |
| 269 | 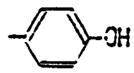 | 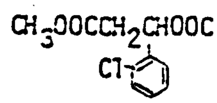 | $CH_3$ | H | H |
| 270 | 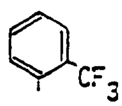 | 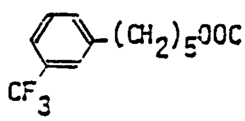 | $CH_3$ | H | H |
| 271 | 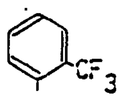 | $C_2H_5OOC$ | 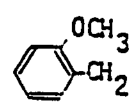 | H | H |
| 272 | 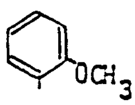 | $CH_3OOC$ | 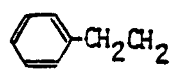 | H | H |
| 273 | 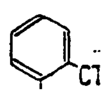 | $(CH_3)_2CHOOC$ | $CH_3$ | $CH_2CH_2Cl$ | H |
| 274 | 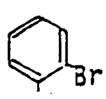 | $(CH_3)_3COOC$ | $CH_3$ | $(CH_2)_3OSO_2CH_3$ | H |
| 275 | 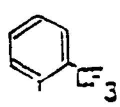 | 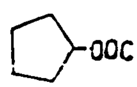 | $CH_3$ | 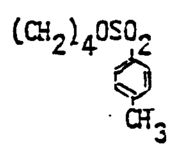 | H |
| 276 | 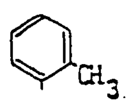 | $C_2H_5OOC$ | $CH_3$ | $CH_2CH_2OH$ | H |

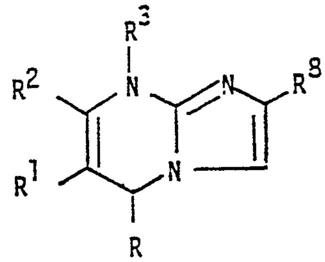

| No. | R | $R^1$ | $R^2$ | $R^3$ | $R^8$ |
|---|---|---|---|---|---|
| 277 | 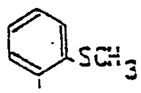 | $(CH_3)_2CHOOC$ | 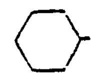 | H | H |
| 278 | 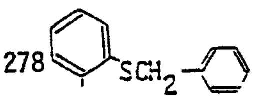 | $CH_3(CH_2)_2OOC$ | 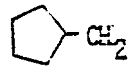 | H | H |
| 279 | 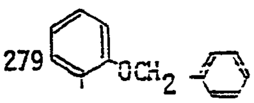 | $CH_3(CH_2)_3OOC$ | $H_2NCH_2CH_2$ | H | H |
| 280 | 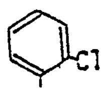 | $CH_3(CH_2)_3OOC$ | $CH_3CONHCH_2CH_2$ | H | H |
| 281 | 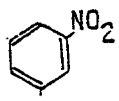 | $(CH_3)_2CHCH_2OOC$ | $HOCH_2CH_2OCH_2$ | H | H |
| 282 | 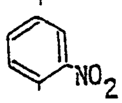 | $C_2H_5OOC$ | $H_2N(CH_2)_2OCH_2$ | H | H |
| 283 | 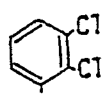 | $CH_3OOC$ | 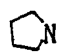$N(CH_2)_2O(CH_2)_2$ | H | H |
| 284 | 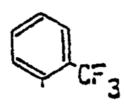 | $(CH_3)_2CHOOC$ | $CH_3$ | $C_3H_7$ | H |
| 285 | 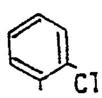 | $(CH_3)_3COOC$ | $CH_3$ | $CH_2$-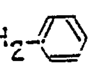 | H |
| 286 | 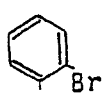 | $C_3H_7OOC$ | $CH_3$ | $(CH_2)_2$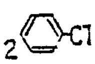 | H |
| 287 | 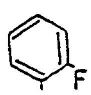 | 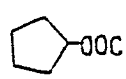OOC | $CH_3$ | $CH_2OCH_2$-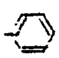 | H |
| 288 | 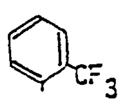 | $CH_3(CH_2)_3OOC$ | $CH_3$ | $COCH_3$ | H |

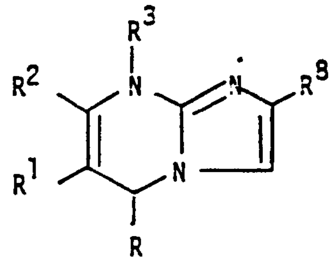

| No. | R | $R^1$ | $R^2$ | $R^3$ | $R^8$ |
|---|---|---|---|---|---|
| 289 | 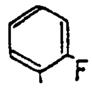 | $(CH_3)_2CHOOC$ | $CH_3$ | 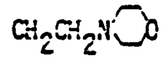 | H |
| 290 | 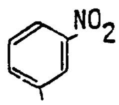 | $CH_3OOC$ | $CH_3$ | $(CH_2)_3NC_2H_5$ | H |
| 291 | 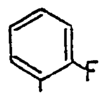 | $(CH_3)_2CHOOC$ | $CH_2N(C_2H_5)_2$ | H | H |
| 292 | 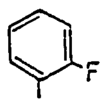 | 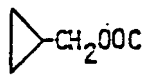 | $CH_3$ | H | H |
| 293 | 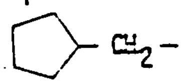 | $CH_3OOC$ | $CH_3$ | H | H |
| 294 | 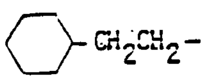 | 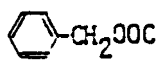 | $CH_3$ | H | H |
| 295 | 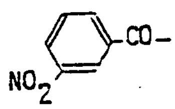 | $CH_3OCH_2CH_2OOC$ | $CH_3$ | H | H |
| 296 | 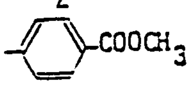 | $CH_3OOC$ | $CH_3$ | H | H |
| 297 | 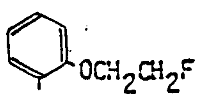 | $(CH_3)_2CHOOC$ | $CH_3$ | H | H |
| 298 | 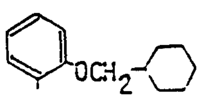 | 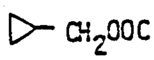 | $CH_3$ | H | H |
| 299 | 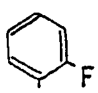 | $(CH_3)_2CHOSC$ | $CH_3$ | H | H |
| 300 | 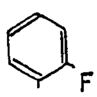 | $(CH_3)_2CHSSC$ | $CH_3$ | H | H |

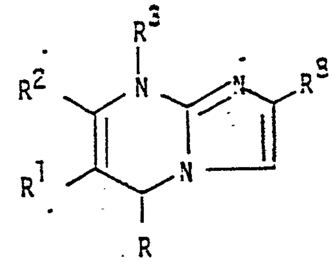

| No. | R | $R^1$ | $R^2$ | $R^3$ | $R^8$ |
|---|---|---|---|---|---|
| 301 | 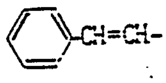 | $(CH_3)_2CHOOC$ | $CH_3$ | H | H |
| 302 | 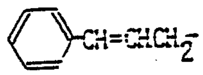 | $(CH_3)_3COOC$ | $CH_3$ | H | H |
| 303 | 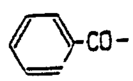 | $C_2H_5OOC$ | $CH_3$ | H | H |
| 304 | $CH_3CH{=}C(CH_3)-$ | $(CH_3)_2CHOOC$ | $CH_3$ | H | H |
| 305 | $CH{\equiv}CCH_2-$ | $(CH_3)_3COOC$ | $CH_3$ | H | H |
| 306 | 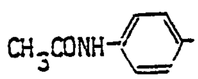 | $C_2H_5OOC$ | $CH_3$ | H | H |
| 307 | 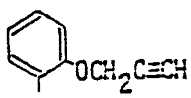 | $(CH_3)_2CHOOC$ | $CH_3$ | H | H |
| 308 | 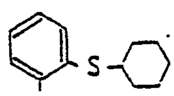 | $C_2H_5OOC$ | $CH_3$ | H | H |
| 309 | 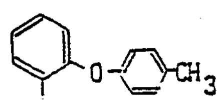 | $CH_3CH_2CH_2OOC$ | $CH_3$ | H | H |
| 310 | 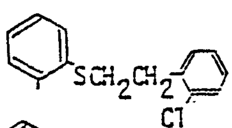 | $CH_3(CH_2)_3OOC$ | $CH_3$ | H | H |
| 311 | 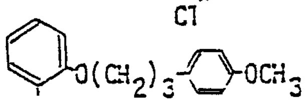 | 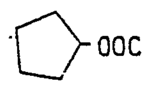 | $CH_3$ | H | H |
| 312 | 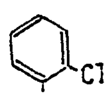 | 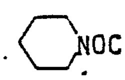 | $CH_3$ | H | H |

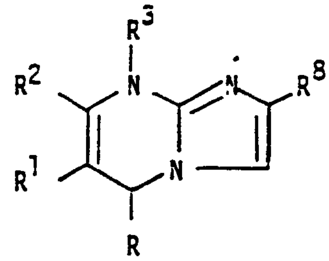

| No. | R | $R^1$ | $R^2$ | $R^3$ | $R^8$ |
|---|---|---|---|---|---|
| 313 | 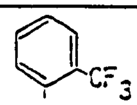 | $(CH_3)_2CHOOC$ | $CH_3$ | $COOC_2H_5$ | H |
| 314 | 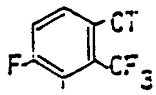 | $C_2H_5OOC$ | $CH_3$ | H | H |
| 315 | 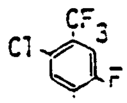 | $(CH_3)_2CHOOC$ | $CH_3$ | H | H |
| 316 | 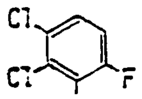 | $C_2H_5OOC$ | $CH_3$ | H | H |
| 317 | 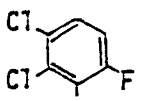 | $(CH_3)_2CHOOC$ | $CH_3$ | H | H |
| 318 | 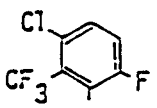 | $(CH_3)_2CHOOC$ | $CH_3$ | H | H |
| 319 | 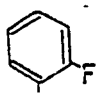 | $C_2H_5OOC$ | $H_2N$ | H | H |
| 320 | 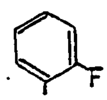 | $(CH_3)_2CHOOC$ | 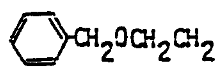 | H | H |
| 321 | 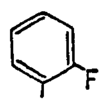 | $(CH_3)_2CHOOC$ | 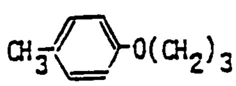 | H | H |
| 322 | 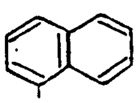 | $(CH_3)_2CHOOC$ | $CH_3$ | H | H |
| 323 | 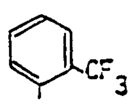 | $ClCH_2CH_2CH_2CH_2OOC$ | $CH_3$ | H | H |
| 324 | 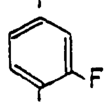 | $(ClCH_2)_2CHOOC$ | $CH_3$ | H | H |

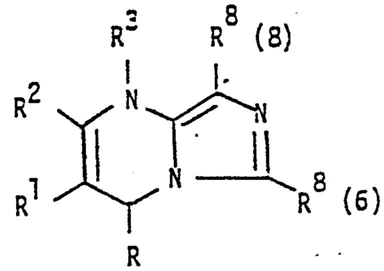

| No. | R | $R^1$ | $R^2$ | $R^3$ | $R^8(6)$ | $R^8(8)$ | M.p. (°C) |
|---|---|---|---|---|---|---|---|
| 325 | 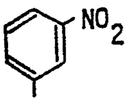 | $C_2H_5OOC$ | $CH_3$ | H | H | $COOC_2H_5$ | 160-162 |
| 326 | 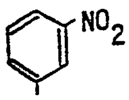 | $C_2H_5OOC$ | $CH_3$ | H | $CH_2N(C_2H_5)_2$ | $COOC_2H_5$ | $HCl\cdot 3/2\ H_2O$ 193-194(d) |
| 327 | 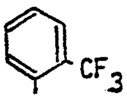 | $C_2H_5OOC$ | $CH_3$ | H | H | $COOC_2H_5$ | 192-193 |
| 328 | 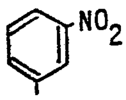 | $C_2H_5OOC$ | $CH_3$ | H | H | COOH | 222(d) |
| 329 | 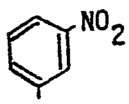 | $C_2H_5OOC$ | $CH_3$ | H | $CH_2N(C_2H_5)_2$ | H | 198-200(d) |
| 330 | 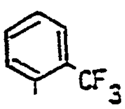 | 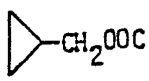 | $CH_3$ | H | H | H | |
| 331 | 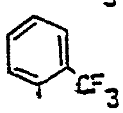 | 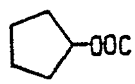 | $CH_3$ | H | H | H | |
| 332 | 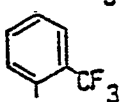 | $O_2N$ | $CH_3$ | H | H | H | |
| 333 | 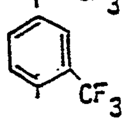 | 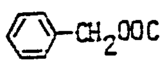 | $CH_3$ | $CH_2OC_2H_5$ | H | H | |
| 334 | 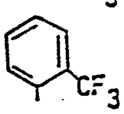 | HOOC | $CH_3$ | $CH_2OC_2H_5$ | H | H | |
| 335 | 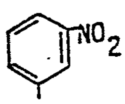 | $C_2H_5OOC$ | $(C_2H_5)_2NCH_2$ | H | H | H | |
| 336 | 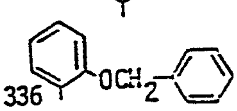 | 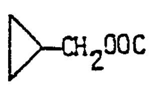 | $CH_3$ | H | H | H | |

(d: decomposition)

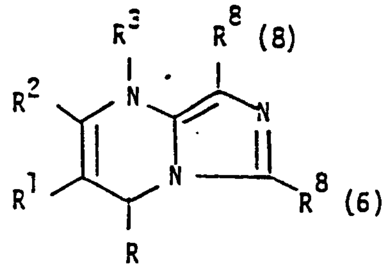

| No. | R | $R^1$ | $R^2$ | $R^3$ | $R^8(6)$ | $R^8(8)$ |
|---|---|---|---|---|---|---|
| 337 | 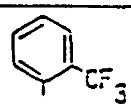 | $CH_3(CH_2)_7OOC$ | $CH_3$ | H | H | H |
| 338 | 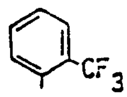 | $CH_3(CH_2)_5OOC$ | $CH_3$ | H | H | H |
| 339 | 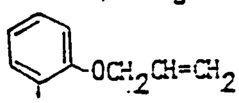 | $C_2H_5OOC$ | $CH_3$ | H | H | H |
| 340 | 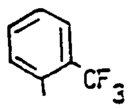 | $FCH_2CH_2OOC$ | $CH_3$ | H | H | H |
| 341 | 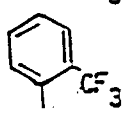 | $CH_3OOC$ | $CH_3$ | H | H | COOH |
| 342 | 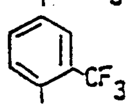 | $O_2N$ | $CH_3$ | H | H | $COOC_2H_5$ |
| 343 | 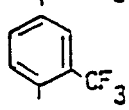 | $ClCH_2CH_2OOC$ | $CH_3$ | H | H | H |
| 344 | 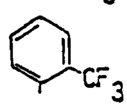 | $CF_3CH_2OOC$ | $CH_3$ | H | H | H |
| 345 | 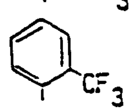 | $CH_2=CHCH_2OOC$ | $CH_3$ | H | H | H |
| 346 | 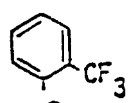 | 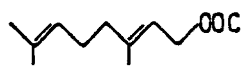 | $CH_3$ | H | H | H |
| 347 | 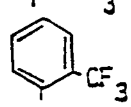 | $CH\equiv CCH_2OOC$ | $CH_3$ | H | H | H |
| 348 | 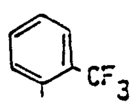 | $CH_3S(CH_2)_2OOC$ | $CH_3$ | H | H | H |

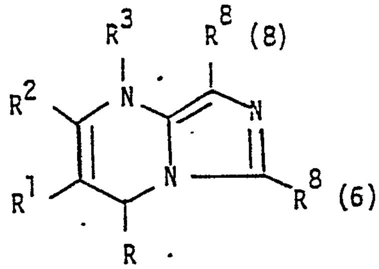

| No. | R | $R^1$ | $R^2$ | $R^3$ | $R^8(6)$ | $R^8(8)$ |
|---|---|---|---|---|---|---|
| 349 | 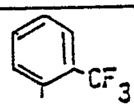 | 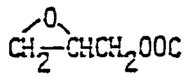 | $CH_3$ | H | H | H |
| 350 | 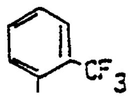 | $CH_3(CH_2)_4OOC$ | $CH_3$ | H | H | H |
| 351 | 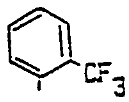 | 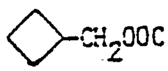 | $CH_3$ | H | H | H |
| 352 | 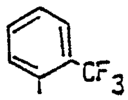 | 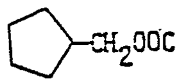 | $CH_3$ | H | H | H |
| 353 | 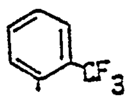 | $CH_3(CH_2)_{21}OOC$ | $CH_3$ | H | H | H |
| 354 | 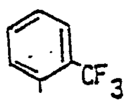 | 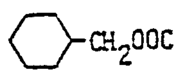 | $CH_3$ | H | H | H |
| 355 | 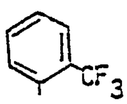 | $(CH_3)_3CCH_2OOC$ | $CH_3$ | H | H | H |
| 356 | 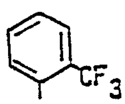 | 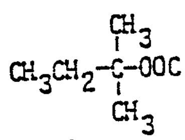 | $CH_3$ | H | H | H |
| 357 | 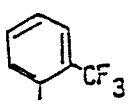 | 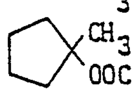 | $CH_3$ | H | H | H |
| 358 | 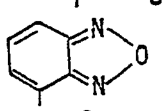 | $C_2H_5OOC$ | $CH_3$ | H | H | H |
| 359 | 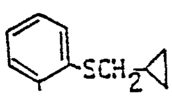 | $C_2H_5OOC$ | $CH_3$ | H | H | H |
| 360 | 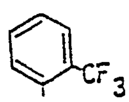 | 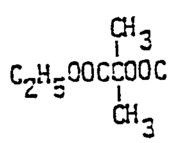 | $CH_3$ | H | H | H |

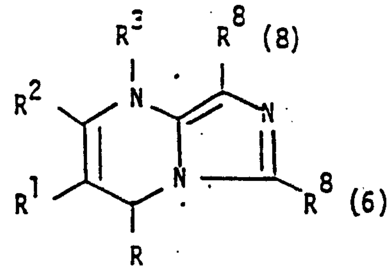

| No. | R | $R^1$ | $R^2$ | $R^3$ | $R^8(6)$ | $R^8(8)$ |
|---|---|---|---|---|---|---|
| 361 | 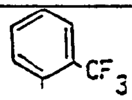 | 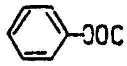OOC | $CH_3$ | H | H | H |
| 362 | 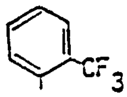 | $CH_3(CH_2)_{15}OOC$ | $CH_3$ | H | H | H |
| 363 | 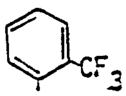 | $CH_3OOCCHOOC$ 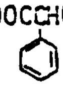 | $CH_3$ | H | H | H |
| 364 | 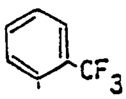 | 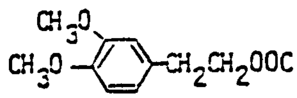 | $CH_3$ | H | H | H |
| 365 | 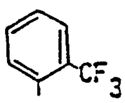 | 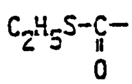 | $CH_3$ | H | H | H |
| 366 | 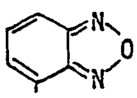 | $O_2N$ | $CH_3$ | H | H | H |
| 367 | 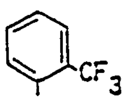 | $C_2H_5OOC$ | Cl | H | H | H |
| 368 | 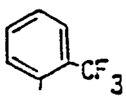 | $C_2H_5OOC$ | $(C_2H_5)_2NCH_2$ | H | H | H |
| 369 | 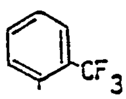 | $C_2H_5OOC$ | $CH_3$ | H | F | H |
| 370 | 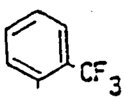 | $C_2H_5OOC$ | $CH_3$ | $CH_3$ | H | H |
| 371 | 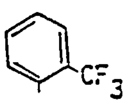 | $C_2H_5OOC$ | $CH_3$ | 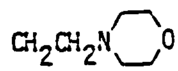 | H | H |
| 372 | 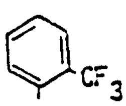 | 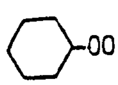OOC | $CH_3$ | H | H | H |

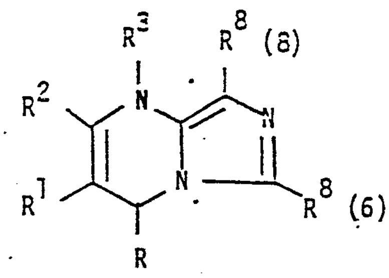

| NO. | R | $R^1$ | $R^2$ | $R^3$ | $R^8(6)$ | $R^8(8)$ |
|---|---|---|---|---|---|---|
| 373 | 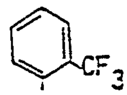 | $C_2H_5OOC$ | $(C_2H_5)_2N$ | H | H | H |
| 374 | 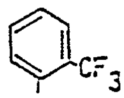 | $C_2H_5OOC$ | $CH_3$ | H | $CH_3$ | H |
| 375 | 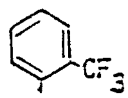 | $C_2H_5OOC$ | $CH_3$ | $CH_2$ 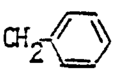 | H | H |
| 376 | 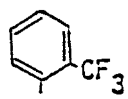 | $C_2H_5OOC$ | 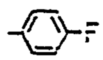 | H | H | H |
| 377 | 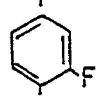 | 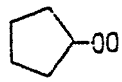 OOC | $CH_3$ | H | H | H |
| 378 | 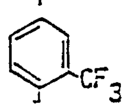 | $(C_2H_5)_2CHOOC$ | $CH_3$ | H | H | H |
| 379 | 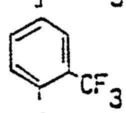 | $(CH_3)_2CHOOC$ | $CH_3$ | $CH_3$ | H | H |
| 380 | 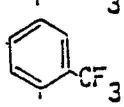 | $H_2NSC$ | $CH_3$ | H | H | H |
| 381 | 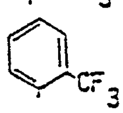 | $(CH_3)_2CHSC(=O)$ | $CH_3$ | H | H | H |
| 382 | 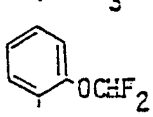 | $CH_3OOC$ | $CH_3$ | H | H | H |
| 383 | 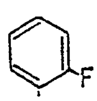 | $C_2H_5OOC$ | 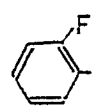 | H | H | H |
| 384 | 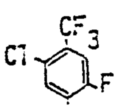 | $(CH_3)_2CHOOC$ | $CH_3$ | H | H | H |

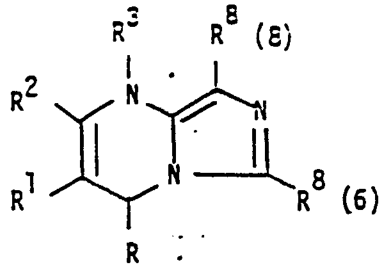

| No. | R | $R^1$ | $R^2$ | $R^3$ | $R^8(6)$ | $R^8(8)$ |
|---|---|---|---|---|---|---|
| 385 | 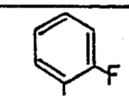 | $(CH_3)_2CHOOC$ | $CH_2N(C_2H_5)_2$ | H | H | H |
| 386 | 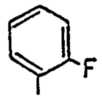 | 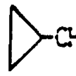$-CH_2OOC$ | $CH_3$ | H | H | H |
| 387 | 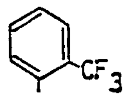 | $(CH_3)_2CHOOC$ | $CH_3$ | $COOC_2H_5$ | H | H |
| 388 | 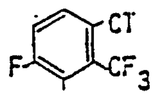 | $C_2H_5OOC$ | $CH_3$ | H | H | H |
| 389 | 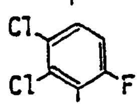 | $C_2H_5OOC$ | $CH_3$ | H | H | H |
| 390 | 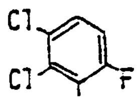 | $(CH_3)_2CHOOC$ | $CH_3$ | H | H | H |
| 391 | 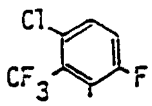 | $(CH_3)_2CHOOC$ | $CH_3$ | H | H | H |
| 392 | 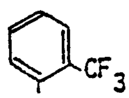 | $(ClCH_2)_2CHOOC$ | $CH_3$ | H | H | H |
| 393 | 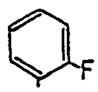 | $ClCH_2CH_2OOC$ | $CH_3$ | H | H | H |
| 394 | 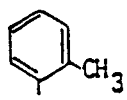 | 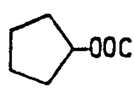$-OOC$ | $CH_3$ | H | H | H |
| 395 | 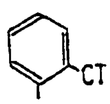 | 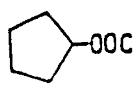$-OOC$ | $CH_3$ | H | H | H |
| 396 | 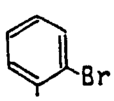 | 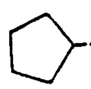$-OOC$ | $CH_3$ | H | H | H |

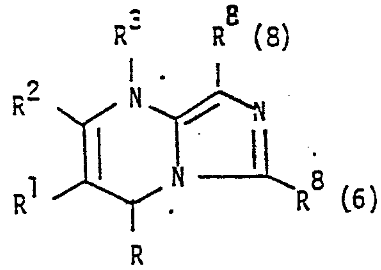

| No. | R | $R^1$ | $R^2$ | $R^3$ | $R^8(6)$ | $R^8(8)$ |
|---|---|---|---|---|---|---|
| 397 | 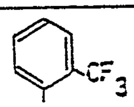 | $ClCH_2CH_2CH_2CH_2OOC$ | $CH_3$ | H | H | H |
| 398 | 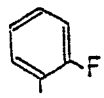 | $(ClCH_2)_2CHOOC$ | $CH_3$ | H | H | H |
| 399 | 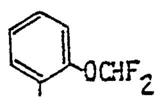 | $(CH_3)_3COOC$ | $CH_3$ | H | H | H |
| 400 | 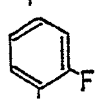 | $C_2H_5OOC$ | $H_2N$ | H | H | H |
| 401 | 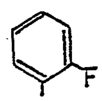 | $(CH_3)_2CHOOC$ | 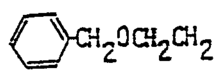 | H | H | H |
| 402 | 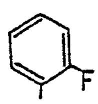 | $(CH_3)_2CHOOC$ | 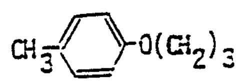 | H | H | H |
| 403 | 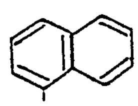 | $(CH_3)_2CHOOC$ | $CH_3$ | H | H | H |
| 404 | 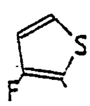 | $(CH_3)_2CHOOC$ | $CH_3$ | H | H | H |
| 405 | 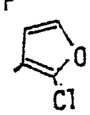 | $C_2H_5OOC$ | $CH_3$ | H | H | H |
| 406 | 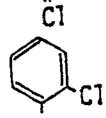 | 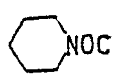 | $CH_3$ | H | H | H |
| 407 | 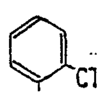 | $(CH_3)_2CHOOC$ | $CH_3$ | $CH_2CH_2Cl$ | H | H |
| 408 | 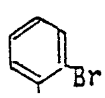 | $(CH_3)_3COOC$ | $CH_3$ | $(CH_2)_3OSO_2CH_3$ | H | H |

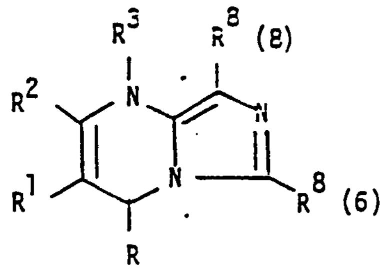

| No. | R | $R^1$ | $R^2$ | $R^3$ | $R^8(6)$ | $R^8(8)$ |
|---|---|---|---|---|---|---|
| 409 | 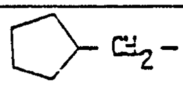 | $CH_3OOC$ | $CH_3$ | H | H | H |
| 410 | 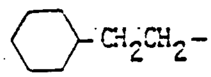 | 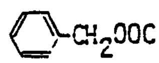 | $CH_3$ | H | H | H |
| 411 | 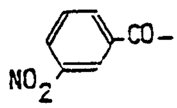 | $CH_3OCH_2CH_2OOC$ | $CH_3$ | H | H | H |
| 412 | 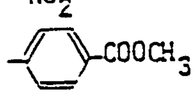 | $CH_3OOC$ | $CH_3$ | H | H | H |
| 413 | 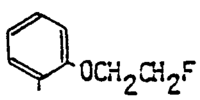 | $(CH_3)_2CHOOC$ | $CH_3$ | H | H | H |
| 414 | 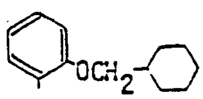 | 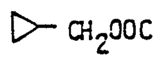 | $CH_3$ | H | H | H |
| 415 | 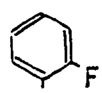 | $(CH_3)_2CHOSC$ | $CH_3$ | H | H | H |
| 416 | 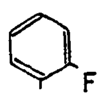 | $(CH_3)_2CHSSC$ | $CH_3$ | H | H | H |
| 417 | 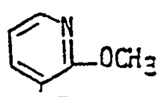 | $(CH_3)_2CHOOC$ | $CH_3$ | H | H | H |
| 418 | 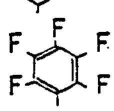 | $(CH_3)_2CHOOC$ | $CH_3$ | H | H | H |
| 419 | 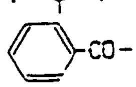 | $C_2H_5OOC$ | $CH_3$ | H | H | H |
| 420 | $CH_3CH=C(CH_3)-$ | $(CH_3)_2CHOOC$ | $CH_3$ | H | H | H |
| 421 | $CH\equiv CCH_2-$ | $(CH_3)_3COOC$ | $CH_3$ | H | H | H |

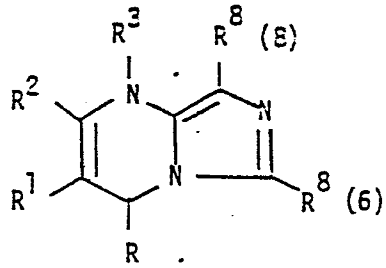

| No. | R | $R^1$ | $R^2$ | $R^3$ | $R^8(6)$ | $R^8(8)$ |
|---|---|---|---|---|---|---|
| 422 | 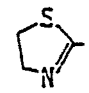 | $CH_3OOC$ | $CH_3$ | H | H | H |
| 423 | 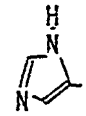 | 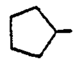-OOC | $CH_3$ | H | H | H |
| 424 | 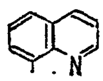 | $(CH_3)_2CHOOC$ | $CH_3$ | H | H | H |
| 425 | 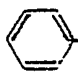CH=CH- | $(CH_3)_2CHOOC$ | $CH_3$ | H | H | H |
| 426 | 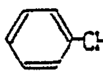CH=CHCH$_2$- | $(CH_3)_3COOC$ | $CH_3$ | H | H | H |
| 427 | 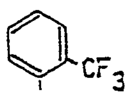 | $(CH_3)_2CHNHOC$ | $CH_3$ | H | H | H |
| 428 | 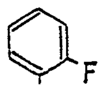 | $(C_2H_5)_2NOC$ | $CH_3$ | H | H | H |
| 429 | 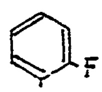 | $H_2NSC$ | $CH_3$ | H | H | H |
| 430 | 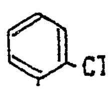 | 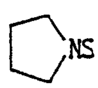NSC | $CH_3$ | H | H | H |
| 431 | 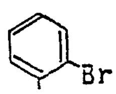 | $(CH_3)_3CNHSC$ | $CH_3$ | H | H | H |
| 432 | 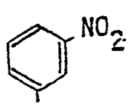 | 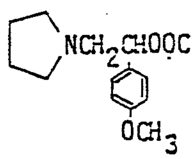 | $CH_3$ | H | H | H |

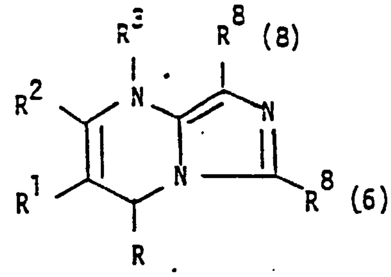

| No. | R | $R^1$ | $R^2$ | $R^3$ | $R^8(6)$ | $R^8(8)$ |
|---|---|---|---|---|---|---|
| 433 | 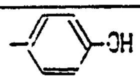 | $CH_3OOCCH_2CHOOC$ 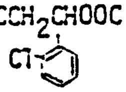 | $CH_3$ | H | H | H |
| 434 | 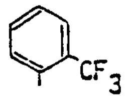 | 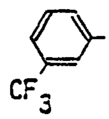 $-(CH_2)_5OOC$ | $CH_3$ | H | H | H |
| 435 | 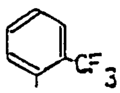 | $C_2H_5OOC$ | 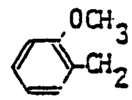 | H | H | H |
| 436 | 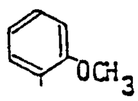 | $CH_3OOC$ | 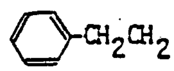 | H | H | H |
| 437 | 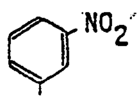 | $(CH_3)_2CHOOC$ | $CH_3$ | H | H | CN |
| 438 | 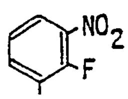 | $(CH_3)_2CHCH_2OOC$ | $CH_3$ | H | H | CN |
| 439 | 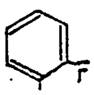 | $(CH_3)_2CHOOC$ | $CH_3$ | H | H | 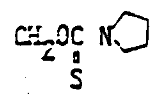 |
| 440 | 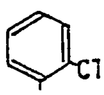 | $C_2H_5OOC$ | $CH_3$ | H | H | $CON(C_2H_5)_2$ |
| 441 | 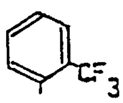 | $(CH_3)_2CHOOC$ | $CH_3$ | H | $CH_2CH=CH_2$ | H |
| 442 | 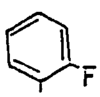 | $(CH_3)_3COOC$ | $CH_3$ | H | H | Br |
| 443 | 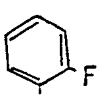 | $(CH_3)_3COOC$ | $CH_3$ | H | $CH_3$ | H |
| 444 | 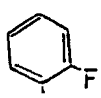 | $(CH_3)_3COOC$ | $CH_3$ | H | H | Cl |

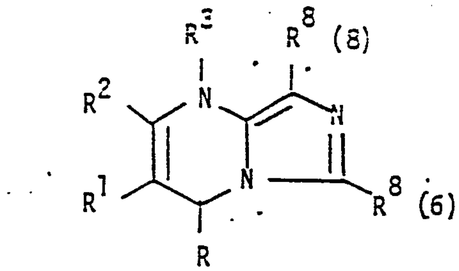

| No. | R | $R^1$ | $R^2$ | $R^3$ | $R^8(6)$ | $R^8(8)$ |
|---|---|---|---|---|---|---|
| 445 | 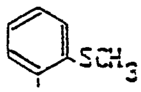 | $(CH_3)_2CHOOC$ | 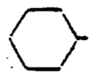 | H | H | H |
| 446 | 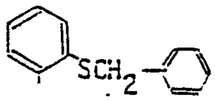 | $CH_3(CH_2)_2OOC$ | 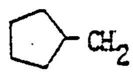 | H | H | H |
| 447 | 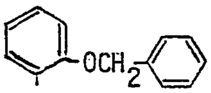 | $CH_3(CH_2)_3OOC$ | $H_2NCH_2CH_2$ | H | H | H |
| 448 | 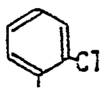 | $CH_3(CH_2)_3OOC$ | $CH_3CONHCH_2CH_2$ | H | H | H |
| 449 | 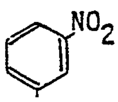 | $(CH_3)_2CHCH_2OOC$ | $HOCH_2CH_2OCH_2$ | H | H | H |
| 450 | 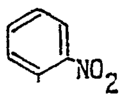 | $C_2H_5OOC$ | $H_2N(CH_2)_2OCH_2$ | H | H | H |
| 451 | 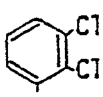 | $CH_3OOC$ | 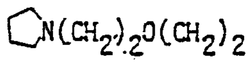 | H | H | H |
| 452 | 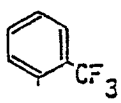 | $(CH_3)_2CHOOC$ | $CH_3$ | $C_3H_7$ | H | H |
| 453 | 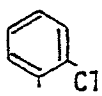 | $(CH_3)_3COOC$ | $CH_3$ | 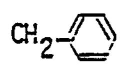 | H | H |
| 454 | 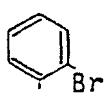 | $C_3H_7OOC$ | $CH_3$ | 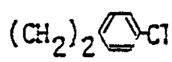 | H | H |
| 455 | 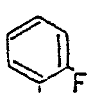 | 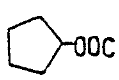 | $CH_3$ | 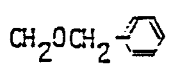 | H | H |
| 456 | 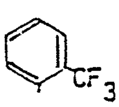 | $CH_3(CH_2)_3OOC$ | $CH_3$ | $COCH_3$ | H | H |

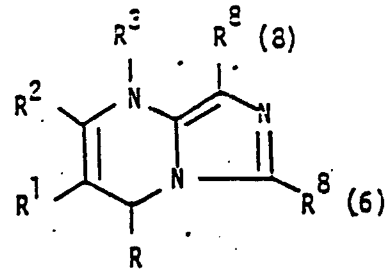

| No. | R | $R^1$ | $R^2$ | $R^3$ | $R^8$(6) | $R^8$(8) |
|---|---|---|---|---|---|---|
| 457 | 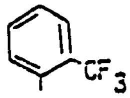 | 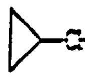$-CH_2OOC$ | $CH_3$ | H | H | $NH_2$ |
| 458 | 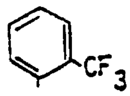 | $C_2H_5OOC$ | $CH_3$ | H | H | $NO_2$ |
| 459 | 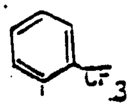 | $C_2H_5OOC$ | $CH_3$ | H | H | $CONH_2$ |
| 460 | 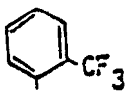 | 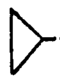$-CH_2OOC$ | $CH_3$ | H | H | $NHCOCH_3$ |
| 461 | 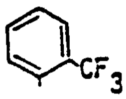 | $C_2H_5OOC$ | $CH_3$ | H | H | F |
| 462 | 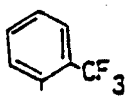 | $C_2H_5OOC$ | $CH_3$ | H | H | $CH_2OH$ |
| 463 | 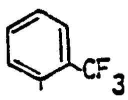 | $C_2H_5OOC$ | $CH_3$ | H | H | $C_3H_7$ |
| 464 | 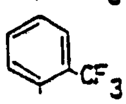 | $C_2H_5OOC$ | $CH_3$ | H | H | $CH_3$ |
| 465 | 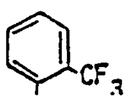 | $C_2H_5OOC$ | $CH_3$ | H | H | $NHCOCH_3$ |
| 466 | 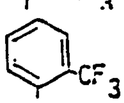 | $C_2H_5OOC$ | $CH_3$ | H | $CH_3$ | $CH_3$ |
| 467 | 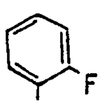 | $(CH_3)_2CHOOC$ | $CH_3$ | H | H | CN |
| 468 | 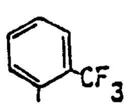 | $(CH_3)_2CHOOC$ | $CH_3$ | H | H | Cl |

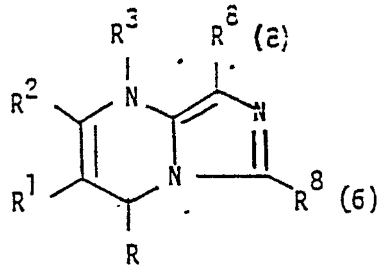

| No. | R | $R^1$ | $R^2$ | $R^3$ | $R^8(6)$ | $R^8(8)$ |
|---|---|---|---|---|---|---|
| 469 | 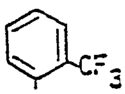 | $C_2H_5OOC$ | $CH_3$ | H | H | Cl |
| 470 | 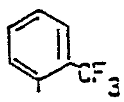 | 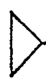 $-CH_2OOC$ | $CH_3$ | H | H | CN |
| 471 | 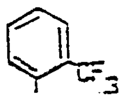 | $C_2H_5OOC$ | $CH_3$ | H | 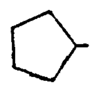 | H |
| 472 | 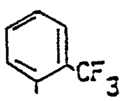 | $C_2H_5OOC$ | $CH_3$ | H | H | $CH_2N(C_2H_5)_2$ |
| 473 | 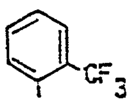 | $CH_3OOC$ | $CH_3$ | H | H | Br |
| 474 | 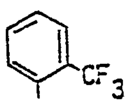 | $CH_3OOC$ | $CH_3$ | H | H | $NO_2$ |
| 475 | 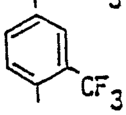 | $CH_3OOC$ | $CH_3$ | H | H | CN |
| 476 | 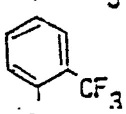 | $CH_3OOC$ | $CH_3$ | H | H | $CONH_2$ |
| 477 | 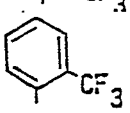 | $C_2H_5OOC$ | $CH_3$ | H | H | CO- 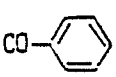 |
| 478 | 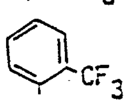 | 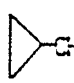 $-CH_2OOC$ | $CH_3$ | H | H | $NO_2$ |
| 479 | 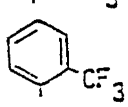 | $C_2H_5OOC$ | $CH_3$ | H | H | Br |
| 480 | 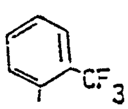 | $C_2H_5OOC$ | $CH_3$ | H | H | CN |

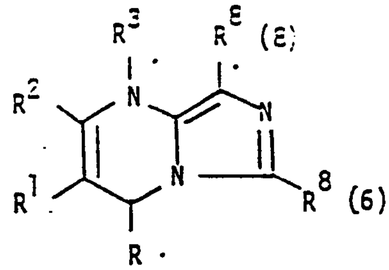

| No. | R | $R^1$ | $R^2$ | $R^3$ | $R^8(6)$ | $R^8(8)$ |
|---|---|---|---|---|---|---|
| 481 | 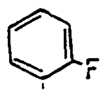 | $(CH_3)_2CHOOC$ | $CH_3$ | H | H | $CH_3$ |
| 482 | 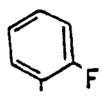 | $(CH_3)_2CHOOC$ | $CH_3$ | H | H | $CH(CH_3)_2$ |
| 483 | 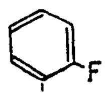 | $(CH_3)_2CHOOC$ | $CH_3$ | H | F | H |
| 484 | 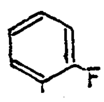 | $(CH_3)_2CHOOC$ | $CH_3$ | H | H | F |
| 485 | 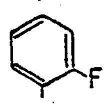 | $(CH_3)_2CHOOC$ | $CH_3$ | H | H | $NO_2$ |
| 486 | 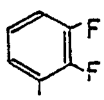 | $(CH_3)_2CHOOC$ | $CH_3$ | H | H | CN |
| 487 | 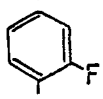 | $(CH_3)_2CHOOC$ | $CH_3$ | H | H | $CH_2OH$ |
| 488 | 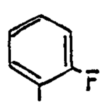 | $(CH_3)_2CHOOC$ | $CH_3$ | H | H | $CH_2OCOCH_3$ |
| 489 | 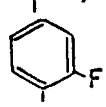 | $(CH_3)_2CHOOC$ | $CH_3$ | H | H | COOH |
| 490 | 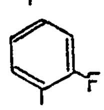 | $(CH_3)_2CHOOC$ | $CH_3$ | H | H | $COCH_3$ |
| 491 | 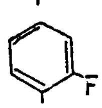 | $(CH_3)_2CHOOC$ | $CH_3$ | H | H | 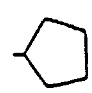 |
| 492 | 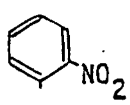 | $(CH_3)_2CHOOC$ | $CH_3$ | H | H | CN |

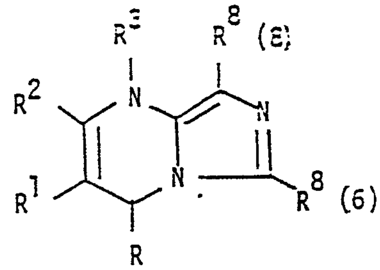

| No. | R | $R^1$ | $R^2$ | $R^3$ | $R^8(6)$ | $R^8(8)$ |
|---|---|---|---|---|---|---|
| 493 | 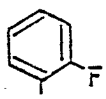 | $(CH_3)_2CHOOC$ | $CH_3$ | H | H | $CH_2F$ |
| 494 | 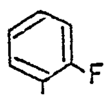 | $(CH_3)_3COOC$ | $CH_3$ | H | H | $CH_2CF_3$ |
| 495 | 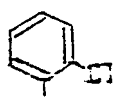 | $(CH_3)_2CHOOC$ | $CH_3$ | H | H | H |
| 496 | 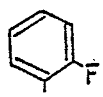 | 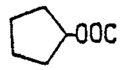OOC | $CH_3$ | H | H | $CH_2CH=CH_2$ |
| 497 | 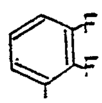 | $(CH_3)_2CHOOC$ | $CH_3$ | H | H | $C\equiv CH$ |
| 498 | 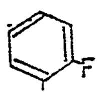 | $C_2H_5OOC$ | $CH_3$ | H | H | $CH_2OCH_2CH_3$ |
| 499 | 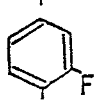 | $(CH_3)_2CHOOC$ | $CH_3$ | H | H | $CH_2$–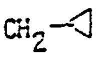 |
| 500 | 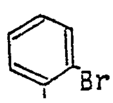 | $CH_3OOC$ | $CH_3$ | H | H | $(CH_2)_3CN$ |
| 501 | 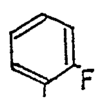 | 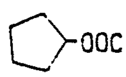OOC | $CH_3$ | H | H | $CH_2CH_2NO_2$ |
| 502 | 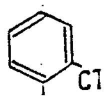 | $CH_3(CH_2)_3OOC$ | $CH_3$ | H | H | $CH_2OCNH_2$ (C=O) |
| 503 | 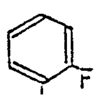 | $(CH_3)_2CHOOC$ | $CH_3$ | H | H | $CH_2OCN(CH_3)_2$ (C=O) |
| 504 | 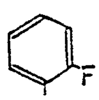 | $(CH_3)_2COOC$ | $CH_3$ | H | H | $CH_2OCNH_2$ (C=S) |

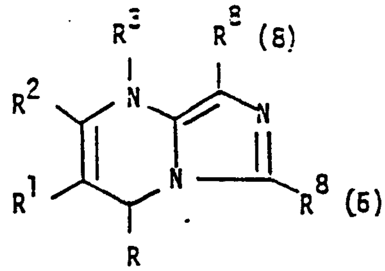

| No. | R | $R^1$ | $R^2$ | $R^3$ | $R^8(6)$ | $R^8(8)$ |
|---|---|---|---|---|---|---|
| 505 | 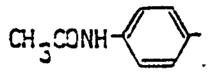 | $C_2H_5OOC$ | $CH_3$ | H | H | H |
| 506 | 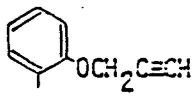 | $(CH_3)_2CHOOC$ | $CH_3$ | H | H | H |
| 507 | 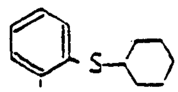 | $C_2H_5OOC$ | $CH_3$ | H | H | H |
| 508 | 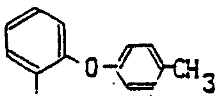 | $CH_3CH_2CH_2OOC$ | $CH_3$ | H | H | H |
| 509 | 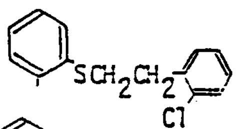 | $CH_3(CH_2)_3OOC$ | $CH_3$ | H | H | H |
| 510 | 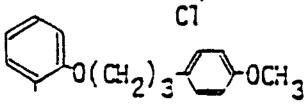 | 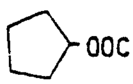 | $CH_3$ | H | H | H |
| 511 | 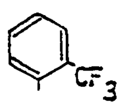 | 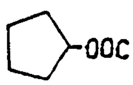 | $CH_3$ | 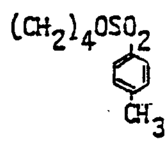 | H | H |
| 512 | 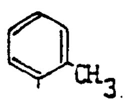 | $C_2H_5OOC$ | $CH_3$ | $CH_2CH_2OH$ | H | H |
| 513 | 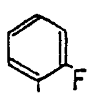 | $(CH_3)_2CHOOC$ | $CH_3$ | 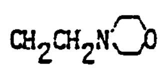 | H | H |
| 514 | 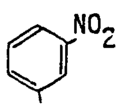 | $CH_3OOC$ | $CH_3$ | $(CH_2)_3NC_2H_5$ | H | H |

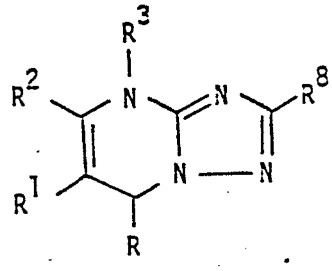

| No. | R | $R^1$ | $R^2$ | $R^3$ | $R^8$ | M.p. (°C) |
|---|---|---|---|---|---|---|
| 515 | 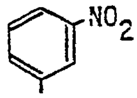 | $C_2H_5OOC$ | $CH_3$ | H | 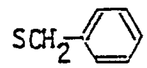 | 208-210 |
| 516 | 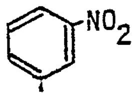 | $C_2H_5OOC$ | 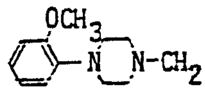 | H | H | HCl 122-123(d) |
| 517 | 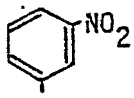 | $C_2H_5OOC$ | 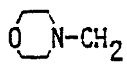 | H | H | HCl 244(d) |
| 518 | 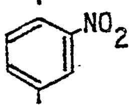 | $C_2H_5OOC$ | $(CH_3)_2CHNHCH_2$ | H | H | 55- 57 |
| 519 | 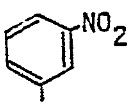 | $C_2H_5OOC$ | $(C_2H_5)_2NCH_2CH_2$ | H | H | HCl·1/2 $H_2O$ 209-211(d) |
| 520 | 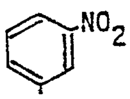 | $C_2H_5OOC$ | 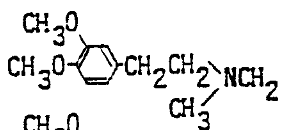 | H | H | 122-124 |
| 521 | 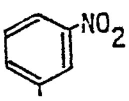 | $C_2H_5OOC$ | 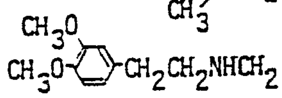 | H | H | 200-201 |
| 522 | 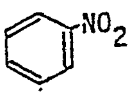 | $C_2H_5OOC$ | 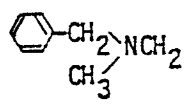 | H | H | 135-137 |
| 523 | 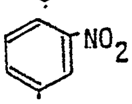 | $C_2H_5OOC$ | $(CH_3)_2NCH_2$ | H | H | HCl 229-230(d) |
| 524 | 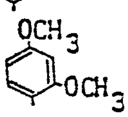 | $CH_3OOC$ | $(C_2H_5)_2NCH_2$ | H | H | HCl 206-208 |
| 525 | 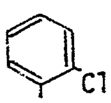 | $CH_3OOC$ | $(C_2H_5)_2NCH_2$ | H | H | HCl 217-218 |

(d: decomposition)

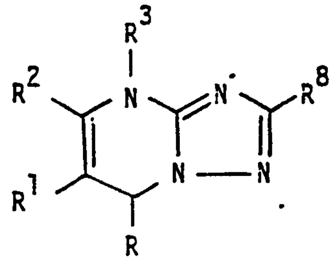

| No. | R | $R^1$ | $R^2$ | $R^3$ | $R^8$ | M.p. (°C) |
|---|---|---|---|---|---|---|
| 526 | $CH_3$ | $C_2H_5OOC$ | $(C_2H_5)_2NCH_2$ | H | H | 100-102 |
| 527 | 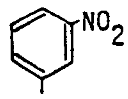 | $C_2H_5OOC$ | $(C_2H_5)_2NCH_2$ | H | 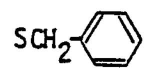 | HCl 155-157(d) |
| 528 | 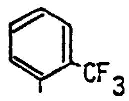 | $C_2H_5OOC$ | $(C_2H_5)_2NCH_2$ | H | H | HCl 206-207(d) |
| 529 | 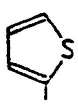 | $CH_3OOC$ | $(C_2H_5)_2NCH_2$ | H | H | HCl 186-188(d) |
| 530 | 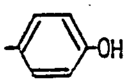 | $C_2H_5OOC$ | $(C_2H_5)_2NCH_2$ | H | H | HCl $H_2O$ 167-169(d) |
| 531 | 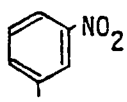 | $C_2H_5OOC$ | 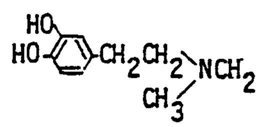 | H | H | HCl·1/2 $H_2C$ 179-181(d) |
| 532 | 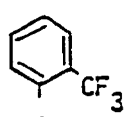 | 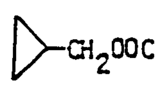 | $CH_3$ | H | H | |
| 533 | 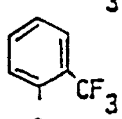 | 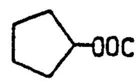 | $CH_3$ | H | H | |
| 534 | 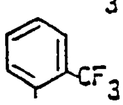 | $C_2H_5OOC$ | $CH_3$ | H | Cl | |
| 535 | 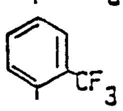 | 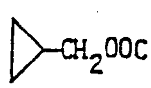 | $CH_3$ | H | CN | |
| 536 | 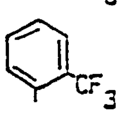 | $C_2H_5OOC$ | $CH_3$ | H | 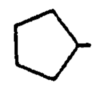 | |
| 537 | 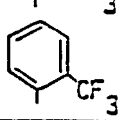 | $O_2N$ | $CH_3$ | H | H | |

(d: decomposition)

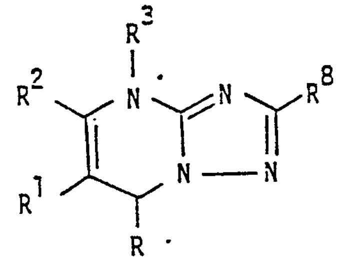

| No. | R | $R^1$ | $R^2$ | $R^3$ | $R^8$ |
|---|---|---|---|---|---|
| 538 | 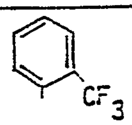 | 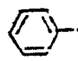$CH_2OOC$ | $CH_3$ | $CH_2OC_2H_5$ | H |
| 539 | 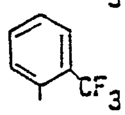 | HOOC | $CH_3$ | $CH_2OC_2H_5$ | H |
| 540 | 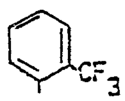 | $C_2H_5OOC$ | $CH_3$ | H | $CH_2N(C_2H_5)_2$ |
| 541 | 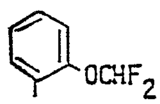 | $C_2H_5OOC$ | $CH_3$ | H | H |
| 542 | 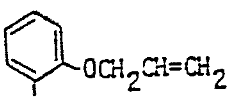 | $C_2H_5OOC$ | $CH_3$ | H | H |
| 543 | 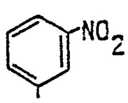 | $C_2H_5OOC$ | $CH_3$ | H | $COOC_2H_5$ |
| 544 | 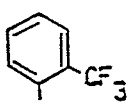 | $CH_3OOC$ | $CH_3$ | H | Br |
| 545 | 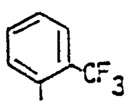 | $O_2N$ | $CH_3$ | H | $COOC_2H_5$ |
| 546 | 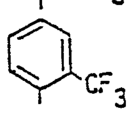 | $ClCH_2CH_2OOC$ | $CH_3$ | H | H |
| 547 | 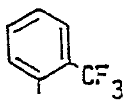 | $C_2H_5OOC$ | $CH_3$ | H | CO-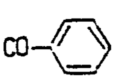 |
| 548 | 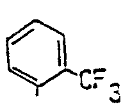 | $CF_3CH_2OOC$ | $CH_3$ | H | H |

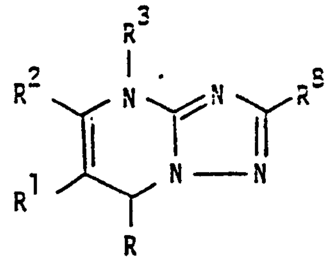

| No. | R | $R^1$ | $R^2$ | $R^3$ | $R^8$ |
|---|---|---|---|---|---|
| 549 | 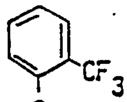 | $CH_3OOC$ | $CH_3$ | H | $NO_2$ |
| 550 | 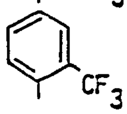 | $CH_3OOC$ | $CH_3$ | H | CN |
| 551 | 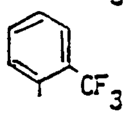 | $CH_3OOC$ | $CH_3$ | H | $CONH_2$ |
| 552 | 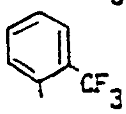 | $FCH_2CH_2OOC$ | $CH_3$ | H | H |
| 553 | 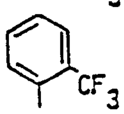 | $CH_3OOC$ | $CH_3$ | H | COOH |
| 554 | 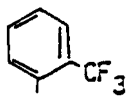 | $CH_3(CH_2)_4OOC$ | $CH_3$ | H | H |
| 555 | 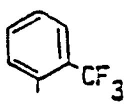 | 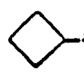$-CH_2OOC$ | $CH_3$ | H | H |
| 556 | 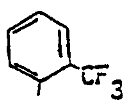 | $C_2H_5OOC$ | $CH_3$ | H | CN |
| 557 | 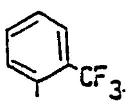 | 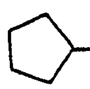$-CH_2OOC$ | $CH_3$ | H | H |
| 558 | 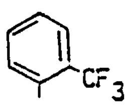 | 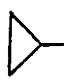$-CH_2OOC$ | $CH_3$ | H | $NH_2$ |
| 559 | 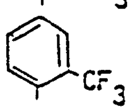 | 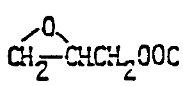 | $CH_3$ | H | H |
| 560 | 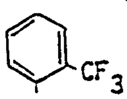 | 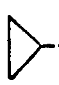$-CH_2OOC$ | $CH_3$ | H | $NHCOCH_3$ |

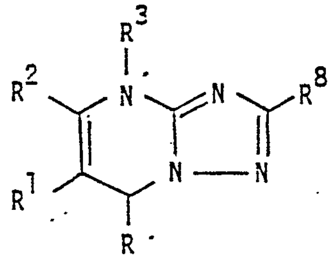

| No. | R | $R^1$ | $R^2$ | $R^3$ | $R^8$ |
|---|---|---|---|---|---|
| 561 | 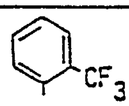 | $C_2H_5OOC$ | $CH_3$ | H | Br |
| 562 | 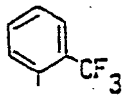 | $CH_3(CH_2)_7OOC$ | $CH_3$ | H | H |
| 563 | 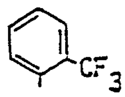 | $CH_3(CH_2)_5OOC$ | $CH_3$ | H | H |
| 564 | 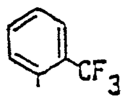 | $C_2H_5OOC$ | $CH_3$ | H | $COOC_2H_5$ |
| 565 | 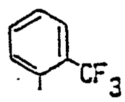 | $CH_2{=}CHCH_2OOC$ | $CH_3$ | H | H |
| 566 | 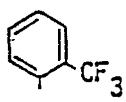 | 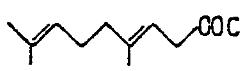 | $CH_3$ | H | H |
| 567 | 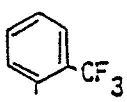 | $CH{\equiv}CCH_2OOC$ | $CH_3$ | H | H |
| 568 | 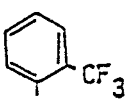 | $CH_3S(CH_2)_2OOC$ | $CH_3$ | H | H |
| 569 | 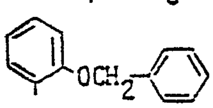 | 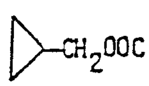 | $CH_3$ | H | H |
| 570 | 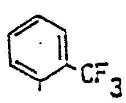 | 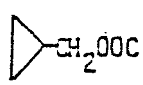 | $CH_3$ | H | $NO_2$ |
| 571 | 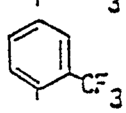 | $CH_3(CH_2)_{21}OOC$ | $CH_3$ | H | H |
| 572 | 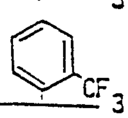 | 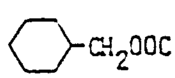 | $CH_3$ | H | H |

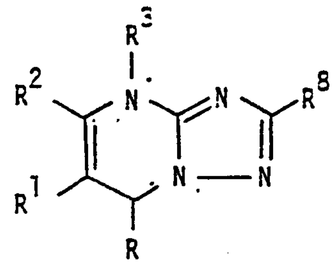

| No. | R | $R^1$ | $R^2$ | $R^3$ | $R^8$ |
|---|---|---|---|---|---|
| 573 | 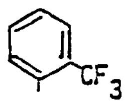 | $(CH_3)_3CCH_2OOC$ | $CH_3$ | H | H |
| 574 | 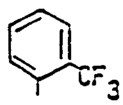 | 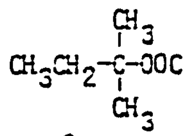 | $CH_3$ | H | H |
| 575 | 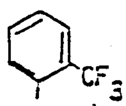 | 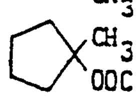 | $CH_3$ | H | H |
| 576 | 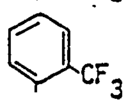 | $C_2H_5OOC$ | $CH_3$ | H | $NO_2$ |
| 577 | 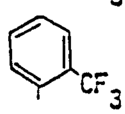 | $C_2H_5OOC$ | $CH_3$ | H | $CONH_2$ |
| 578 | 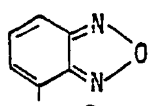 | $C_2H_5OOC$ | $CH_3$ | H | H |
| 579 | 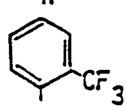 | $CH_3(CH_2)_{15}OOC$ | $CH_3$ | H | H |
| 580 | 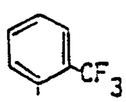 | $CH_3OOCCHOOC$ 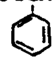 | $CH_3$ | H | H |
| 581 | 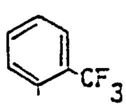 | 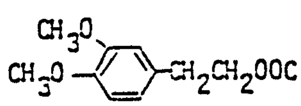 | $CH_3$ | H | H |
| 582 | 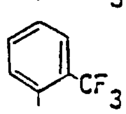 | 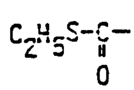 | $CH_3$ | H | H |
| 583 | 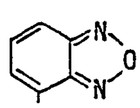 | $O_2N$ | $CH_3$ | H | H |
| 584 | 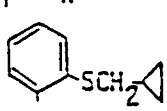 | $C_2H_5OOC$ | $CH_3$ | H | H |

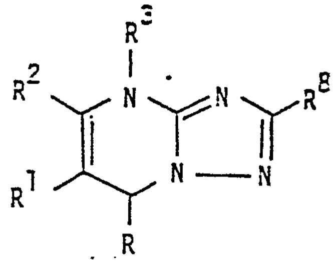

| No. | R | $R^1$ | $R^2$ | $R^3$ | $R^8$ |
|---|---|---|---|---|---|
| 585 | 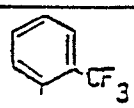 | 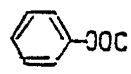 | $CH_3$ | H | H |
| 586 | 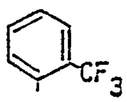 | $C_2H_5OOCC(CH_3)_2OOC$ | $CH_3$ | H | H |
| 587 | 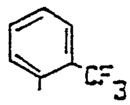 | $(CH_3)_2CHOOC$ | $CH_3$ | $CH_3$ | H |
| 588 | 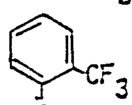 | $(C_2H_5)_2CHOOC$ | $CH_3$ | H | H |
| 589 | 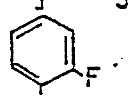 | 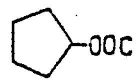 | $CH_3$ | H | H |
| 590 | 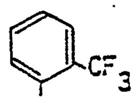 | $ClCH_2CH_2CH_2CH_2OOC$ | $CH_3$ | H | H |
| 591 | 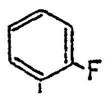 | $(CH_3)_2CHOOC$ | $CH_3$ | H | COOH |
| 592 | 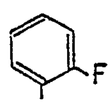 | $(ClCH_2)_2CHOOC$ | $CH_3$ | H | H |
| 593 | 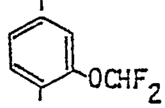 | $(CH_3)_3COOC$ | $CH_3$ | H | H |
| 594 | 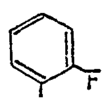 | $(CH_3)_2CHOOC$ | 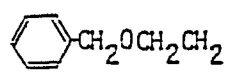 | H | H |
| 595 | 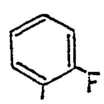 | $(CH_3)_2CHOOC$ | 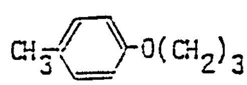 | H | H |
| 596 | 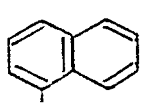 | $(CH_3)_2CHOOC$ | $CH_3$ | H | H |

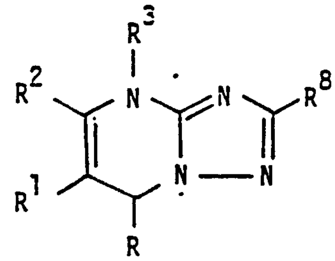

| No. | R | $R^1$ | $R^2$ | $R^3$ | $R^8$ |
|---|---|---|---|---|---|
| 597 | 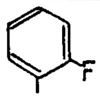 | $(CH_3)_2CHOOC$ | $CH_3$ | H | $COCH_3$ |
| 598 | 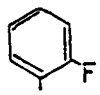 | $(CH_3)_2CHOOC$ | $CH_3$ | H | 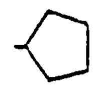 |
| 599 | 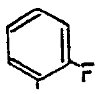 | $C_2H_5OOC$ | $H_2N$ | H | H |
| 600 | 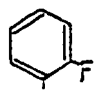 | $(CH_3)_2CHOOC$ | $CH_3$ | H | 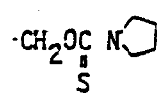 |
| 601 | 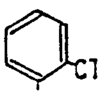 | $C_2H_5OOC$ | $CH_3$ | H | $-CON(C_2H_5)_2$ |
| 602 | 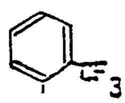 | $(CH_3)_2CHOOC$ | $CH_3$ | H | $CH_2CH=CH_2$ |
| 603 | 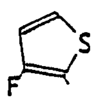 | $(CH_3)_2CHOOC$ | $CH_3$ | H | H |
| 604 | 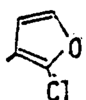 | $C_2H_5OOC$ | $CH_3$ | H | H |
| 605 | 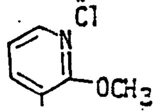 | $(CH_3)_2CHOOC$ | $CH_3$ | H | H |
| 606 | 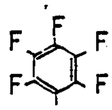 | $(CH_3)_2CHOOC$ | $CH_3$ | H | H |
| 607 | 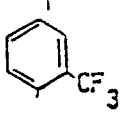 | 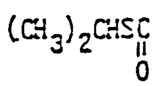 | $CH_3$ | H | H |
| 608 | 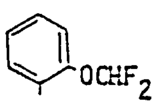 | $CH_3OOC$ | $CH_3$ | H | H |

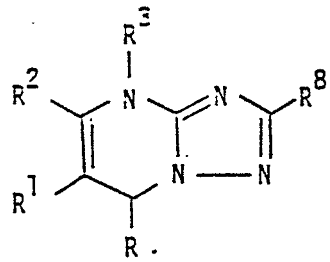

| No. | R | $R^1$ | $R^2$ | $R^3$ | $R^8$ |
|---|---|---|---|---|---|
| 609 | 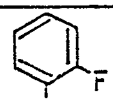 | $(CH_3)_2CHOOC$ | $CH_3$ | H | $-CH_2F$ |
| 610 | 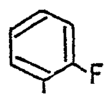 | $(CH_3)_3COOC$ | $CH_3$ | H | $-CH_2CF_3$ |
| 611 | 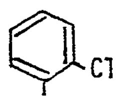 | $(CH_3)_2CHOOC$ | $CH_3$ | H | $CHF_2$ |
| 612 | 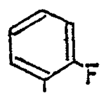 | 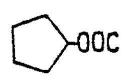OOC | $CH_3$ | H | $CH_2CH{=}CH_2$ |
| 613 | 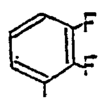 | $(CH_3)_2CHOOC$ | $CH_3$ | H | $C{\equiv}CH$ |
| 614 | 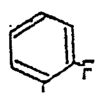 | $C_2H_5OOC$ | $CH_3$ | H | $CH_2OCH_2CH_3$ |
| 615 | 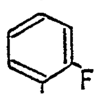 | $(CH_3)_2CHOOC$ | $CH_3$ | H | $CH_2-$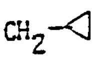 |
| 616 | 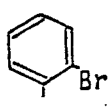 | $CH_3OOC$ | $CH_3$ | H | $(CH_2)_3CN$ |
| 617 | 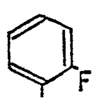 | 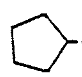OOC | $CH_3$ | H | $CH_2CH_2NO_2$ |
| 618 | 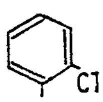 | $CH_3(CH_2)_3OOC$ | $CH_3$ | H | $CH_2O\underset{\underset{O}{\|}}{C}NH_2$ |
| 619 | 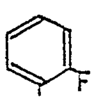 | $(CH_3)_2CHOOC$ | $CH_3$ | H | $CH_2O\underset{\underset{O}{\|}}{C}N(CH_3)_2$ |
| 620 | 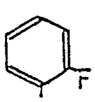 | $(CH_3)_2COOC$ | $CH_3$ | H | $CH_2O\underset{\underset{S}{\|}}{C}NH_2$ |

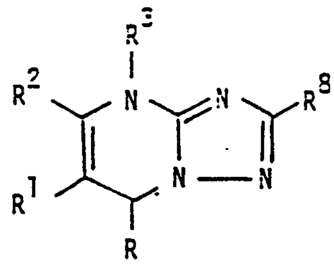

| No. | R | $R^1$ | $R^2$ | $R^3$ | $R^8$ |
|---|---|---|---|---|---|
| 621 | 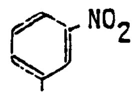 | $(CH_3)_2CHOOC$ | $CH_3$ | H | CN |
| 622 | 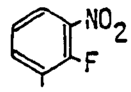 | $(CH_3)_2CHCH_2OOC$ | $CH_3$ | H | CN |
| 623 | 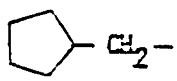 | $CH_3OOC$ | $CH_3$ | H | H |
| 624 | 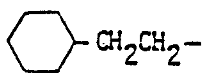 | 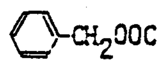 | $CH_3$ | H | H |
| 625 | 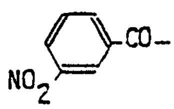 | $CH_3OCH_2CH_2OOC$ | $CH_3$ | H | H |
| 626 | 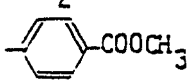 | $CH_3OOC$ | $CH_3$ | H | H |
| 627 | 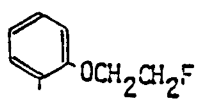 | $(CH_3)_2CHOOC$ | $CH_3$ | H | H |
| 628 | 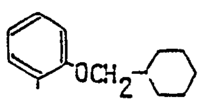 | 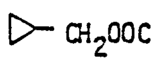 | $CH_3$ | H | H |
| 629 | 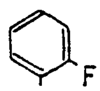 | $(CH_3)_2CHOSC$ | $CH_3$ | H | H |
| 630 | 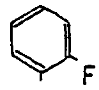 | $(CH_3)_2CHSSC$ | $CH_3$ | H | H |
| 631 | 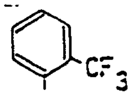 | $(CH_3)_2CHNHOC$ | $CH_3$ | H | H |
| 632 | 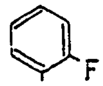 | $(C_2H_5)_2NOC$ | $CH_3$ | H | H |

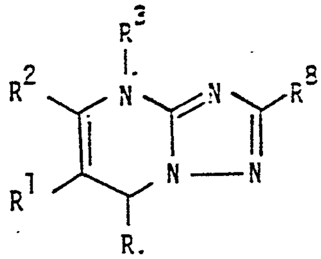

| No. | R | $R^1$ | $R^2$ | $R^3$ | $R^8$ |
|---|---|---|---|---|---|
| 633 | 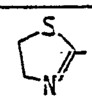 | $CH_3OOC$ | $CH_3$ | H | H |
| 634 | 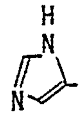 | 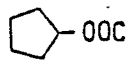 | $CH_3$ | H | H |
| 635 | 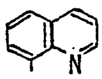 | $(CH_3)_2CHOOC$ | $CH_3$ | H | H |
| 636 | 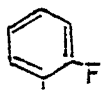 | $(CH_3)_3COOC$ | $CH_3$ | H | Br |
| 637 | 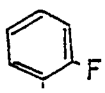 | $(CH_3)_3COOC$ | $CH_3$ | H | $CH_3$ |
| 638 | 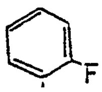 | $(CH_3)_3COOC$ | $CH_3$ | H | Cl |
| 639 | 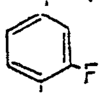 | $(CH_3)_2CHOOC$ | $CH_3$ | H | $CH_3$ |
| 640 | 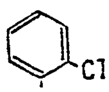 | 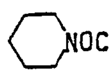 | $CH_3$ | H | H |
| 641 | 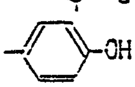 | $CH_3OOCCH_2CHOOC$ 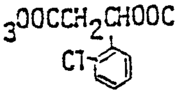 | $CH_3$ | H | H |
| 642 | 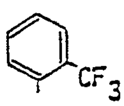 | 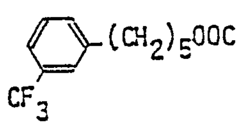 | $CH_3$ | H | H |
| 643 | 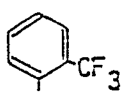 | $C_2H_5OOC$ | 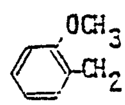 | H | H |
| 644 | 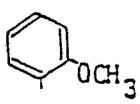 | $CH_3OOC$ | 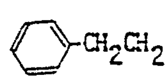 | H | H |

0217142

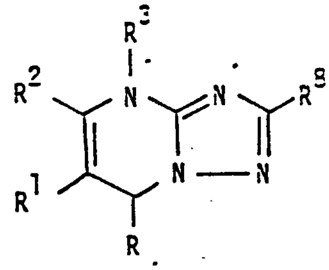

| No. | R | $R^1$ | $R^2$ | $R^3$ | $R^8$ |
|---|---|---|---|---|---|
| 645 | 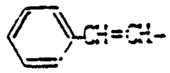 | $(CH_3)_2CHOOC$ | $CH_3$ | H | H |
| 646 | 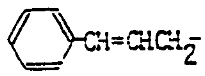 | $(CH_3)_3COOC$ | $CH_3$ | H | H |
| 647 | 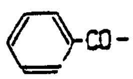 | $C_2H_5OOC$ | $CH_3$ | H | H |
| 648 | $CH_3CH=C(CH_3)-$ | $(CH_3)_2CHOOC$ | $CH_3$ | H | H |
| 649 | $CH\equiv CCH_2-$ | $(CH_3)_3COOC$ | $CH_3$ | H | H |
| 650 | 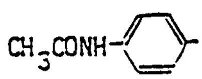 | $C_2H_5OOC$ | $CH_3$ | H | H |
| 651 | 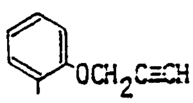 | $(CH_3)_2CHOOC$ | $CH_3$ | H | H |
| 652 | 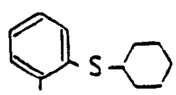 | $C_2H_5OOC$ | $CH_3$ | H | H |
| 653 | 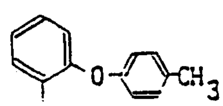 | $CH_3CH_2CH_2OOC$ | $CH_3$ | H | H |
| 654 | 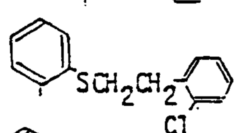 | $CH_3(CH_2)_3OOC$ | $CH_3$ | H | H |
| 655 | 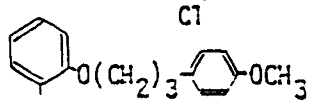 | 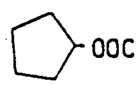 | $CH_3$ | H | H |
| 656 | 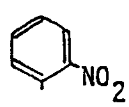 | $(CH_3)_2CHOOC$ | $CH_3$ | H | CN |

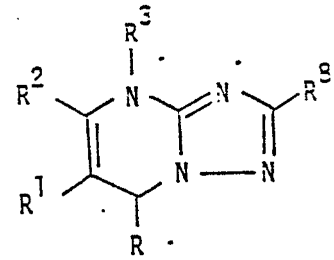

| No. | R | $R^1$ | $R^2$ | $R^3$ | $R^8$ |
|---|---|---|---|---|---|
| 657 | 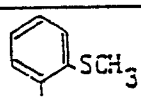 | $(CH_3)_2CHOOC$ | 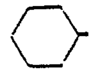 | H | H |
| 658 | 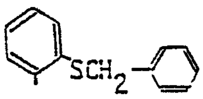 | $CH_3(CH_2)_2OOC$ | 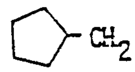 | H | H |
| 659 | 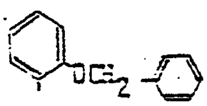 | $CH_3(CH_2)_3OOC$ | $H_2NCH_2CH_2$ | H | H |
| 660 | 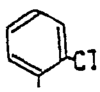 | $CH_3(CH_2)_3OOC$ | $CH_3CONHCH_2CH_2$ | H | H |
| 661 | 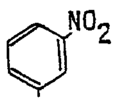 | $(CH_3)_2CHCH_2OOC$ | $HOCH_2CH_2OCH_2$ | H | H |
| 662 | 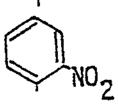 | $C_2H_5OOC$ | $H_2N(CH_2)_2OCH_2$ | H | H |
| 663 | 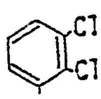 | $CH_3OOC$ | 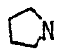 $(CH_2)_2O(CH_2)_2$ | H | H |
| 664 | 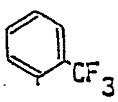 | $(CH_3)_2CHOOC$ | $CH_3$ | $C_3H_7$ | H |
| 665 | 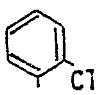 | $(CH_3)_3COOC$ | $CH_3$ | $CH_2$- 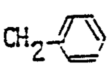 | H |
| 666 | 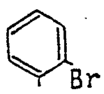 | $C_3H_7OOC$ | $CH_3$ | $(CH_2)_2$ 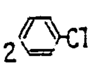 Cl | H |
| 667 | 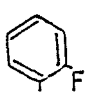 | 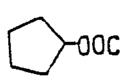 OOC | $CH_3$ | $CH_2OCH_2$- 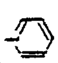 | H |
| 668 | 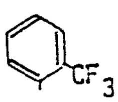 | $CH_3(CH_2)_3OOC$ | $CH_3$ | $COCH_3$ | H |

0217142

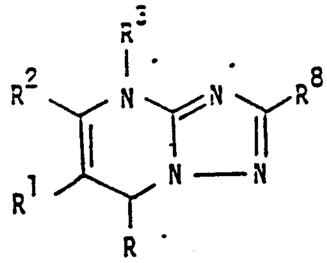

| No. | R | $R^1$ | $R^2$ | $R^3$ | $R^8$ |
|---|---|---|---|---|---|
| 669 | 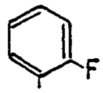 | $(CH_3)_2CHOOC$ | $CH_3$ | H | $NO_2$ |
| 670 | 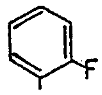 | $(CH_3)_2CHOOC$ | $CH_2N(C_2H_5)_2$ | H | H |
| 671 | 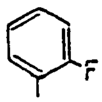 | 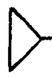 $-CH_2OOC$ | $CH_3$ | H | H |
| 672 | 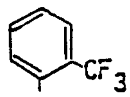 | $(CH_3)_2CHOOC$ | $CH_3$ | $COOC_2H_5$ | H |
| 673 | 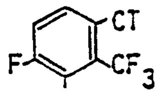 | $C_2H_5OOC$ | $CH_3$ | H | H |
| 674 | 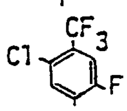 | $(CH_3)_2CHOOC$ | $CH_3$ | H | H |
| 675 | 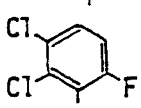 | $C_2H_5OOC$ | $CH_3$ | H | H |
| 676 | 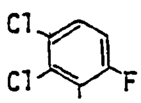 | $(CH_3)_2CHOOC$ | $CH_3$ | H | H |
| 677 | 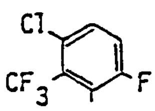 | $(CH_3)_2CHOOC$ | $CH_3$ | H | H |
| 678 | 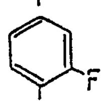 | $(CH_3)_2CHOOC$ | $CH_3$ | H | $CH_2OH$ |
| 679 | 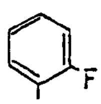 | $(CH_3)_2CHOOC$ | $CH_3$ | H | $CH_2OCOCH_3$ |
| 680 | 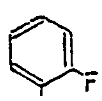 | $(CH_3)_2CHOOC$ | $CH_3$ | H | F |

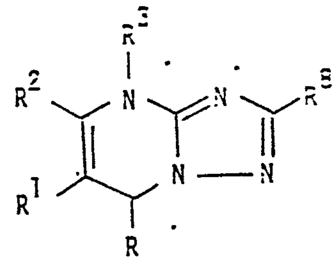

| No. | R | $R^1$ | $R^2$ | $R^3$ | $R^8$ |
|---|---|---|---|---|---|
| 681 | 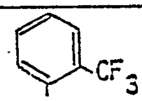 | $(ClCH_2)_2CHOOC$ | $CH_3$ | H | H |
| 682 | 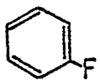 | $ClCH_2CH_2OOC$ | $CH_3$ | H | H |
| 683 | 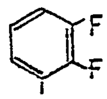 | $(CH_3)_2CHOOC$ | $CH_3$ | H | CN |
| 684 | 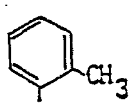 | 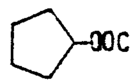-OOC | $CH_3$ | H | H |
| 685 | 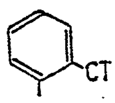 | 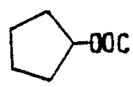-OOC | $CH_3$ | H | H |
| 686 | 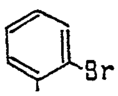 | 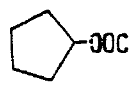-OOC | $CH_3$ | H | H |
| 687 | 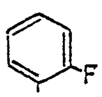 | $C_2H_5OOC$ | 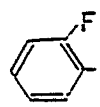 | H | H |
| 688 | 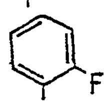 | $(CH_3)_2CHOOC$ | $CH_3$ | H | CN |
| 689 | 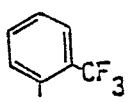 | $(CH_3)_2CHOOC$ | $CH_3$ | H | Cl |
| 690 | 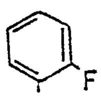 | $(CH_3)_2CHOOC$ | $CH_3$ | H | $CH(CH_3)_2$ |
| 691 | 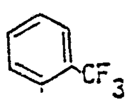 | $C_2H_5OOC$ | $CH_3$ | H | F |
| 692 | 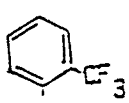 | $C_2H_5OOC$ | $CH_3$ | H | $CH_2OH$ |

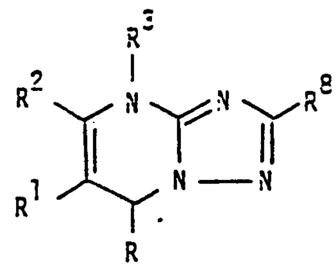

| No. | R | R1 | R2 | R3 | R8 |
|---|---|---|---|---|---|
| 693 | 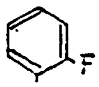 | $H_2NSC$ | $CH_3$ | H | H |
| 694 | 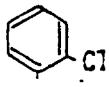 | 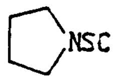 | $CH_3$ | H | H |
| 695 | 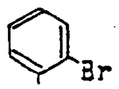 | $(CH_3)_3CNHSC$ | $CH_3$ | H | H |
| 696 | 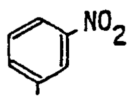 | 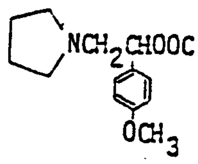 | $CH_3$ | H | H |
| 697 | 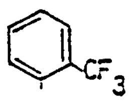 | $C_2H_5OOC$ | $CH_3$ | H | $C_3H_7$ |
| 698 | 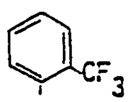 | $C_2H_5OOC$ | $(C_2H_5)_2N$ | H | H |
| 699 | 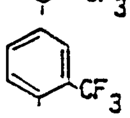 | $C_2H_5OOC$ | $CH_3$ | H | $CH_3$ |
| 700 | 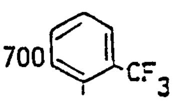 | $C_2H_5OOC$ | $CH_3$ | 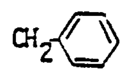 | H |
| 701 | 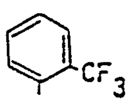 | $C_2H_5OOC$ | 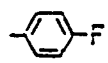 | H | H |
| 702 | 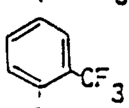 | $H_2NSC$ | $CH_3$ | H | H |
| 703 | 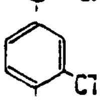 | $(CH_3)_2CHOOC$ | $CH_3$ | $CH_2CH_2Cl$ | H |
| 704 | 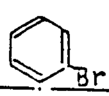 | $(CH_3)_3COOC$ | $CH_3$ | $(CH_2)_3OSO_2CH_3$ | H |

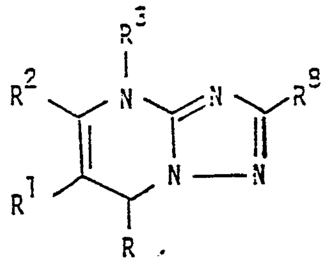

| No. | R | $R^1$ | $R^2$ | $R^3$ | $R^8$ |
|---|---|---|---|---|---|
| 705 | 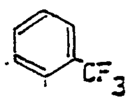 | 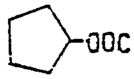 | $CH_3$ | 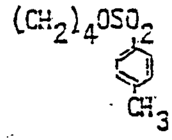 | H |
| 706 | 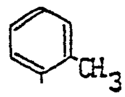 | $C_2H_5OOC$ | $CH_3$ | $CH_2CH_2OH$ | H |
| 707 | 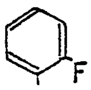 | $(CH_3)_2CHOOC$ | $CH_3$ | 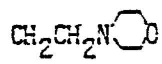 | H |
| 708 | 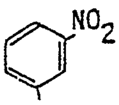 | $CH_3OOC$ | $CH_3$ | $(CH_2)_3NC_2H_5$ | H |
| 709 | 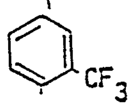 | $C_2H_5OOC$ | $CH_3$ | $CH_3$ | H |
| 710 | 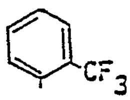 | $C_2H_5OOC$ | $CH_3$ | 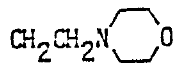 | H |
| 711 | 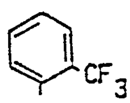 | 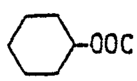 | $CH_3$ | H | H |
| 712 | 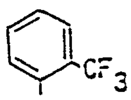 | $C_2H_5OOC$ | Cl | H | H |
| 713 | 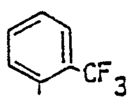 | $C_2H_5OOC$ | $CH_3$ | H | $NHCOCH_3$ |

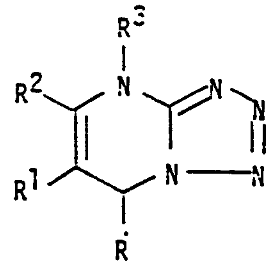

| No. | R | $R^1$ | $R^2$ | $R^3$ |
|---|---|---|---|---|
| 714 | 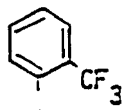 | 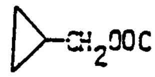 | $CH_3$ | H |
| 715 | 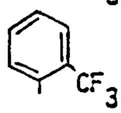 | 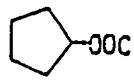 | $CH_3$ | H |
| 716 | 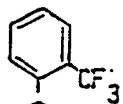 | $O_2N$ | $CH_3$ | H |
| 717 | 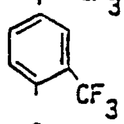 | 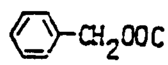 | $CH_3$ | $CH_2OC_2H_5$ |
| 718 | 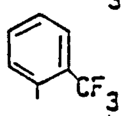 | HOOC | $CH_3$ | $CH_2OC_2H_5$ |
| 719 | 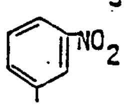 | $CH_3OOC$ | $CH_3$ | $(CH_2)_3N(C_2H_5)_2$ |
| 720 | 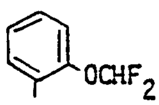 | $C_2H_5OOC$ | $CH_3$ | H |
| 721 | 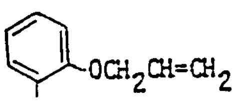 | $C_2H_5OOC$ | $CH_3$ | H |
| 722 | 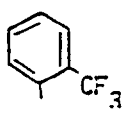 | $FCH_2CH_2OOC$ | $CH_3$ | H |
| 723 | 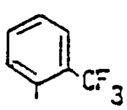 | $ClCH_2CH_2OOC$ | $CH_3$ | H |
| 724 | 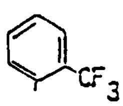 | $CF_3CH_2OOC$ | $CH_3$ | H |

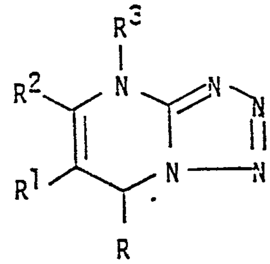

| No. | R | $R^1$ | $R^2$ | $R^3$ |
|---|---|---|---|---|
| 725 | 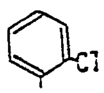 | $CH_3(CH_2)_3OOC$ | $CH_3CONHCH_2CH_2$ | H |
| 726 | 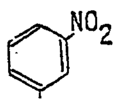 | $(CH_3)_2CHCH_2OOC$ | $HOCH_2CH_2OCH_2$ | H |
| 727 | 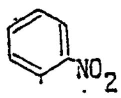 | $C_2H_5OOC$ | $H_2N(CH_2)_2OCH_2$ | H |
| 728 | 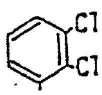 | $CH_3OOC$ | 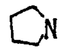$(CH_2)_2O(CH_2)_2$ | H |
| 729 | 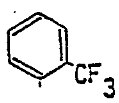 | $(CH_3)_2CHOOC$ | $CH_3$ | $C_3H_7$ |
| 730 | 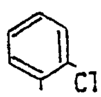 | $(CH_3)_3COOC$ | $CH_3$ | $CH_2$-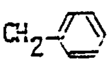 |
| 731 | 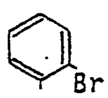 | $C_3H_7OOC$ | $CH_3$ | $(CH_2)_2$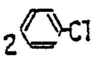 |
| 732 | 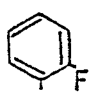 | 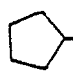-OOC | $CH_3$ | $CH_2OCH_2$-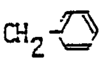 |
| 733 | 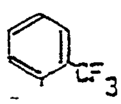 | $CH_3(CH_2)_3OOC$ | $CH_3$ | $COCH_3$ |
| 734 | 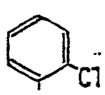 | $(CH_3)_2CHOOC$ | $CH_3$ | $CH_2CH_2Cl$ |
| 735 | 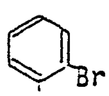 | $(CH_3)_3COOC$ | $CH_3$ | $(CH_2)_3OSO_2CH_3$ |

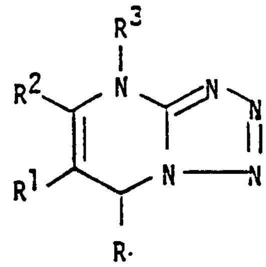

| No. | R | $R^1$ | $R^2$ | $R^3$ |
|---|---|---|---|---|
| 736 | 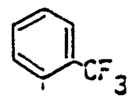 | 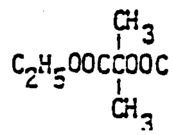 | $CH_3$ | H |
| 737 | 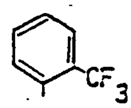 | $CH_3(CH_2)_{15}OOC$ | $CH_3$ | H |
| 738 | 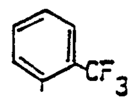 | 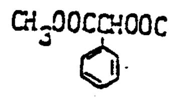 | $CH_3$ | H |
| 739 | 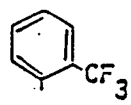 | 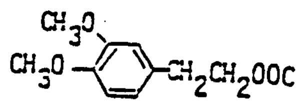 | $CH_3$ | H |
| 740 | 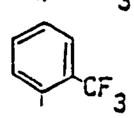 | 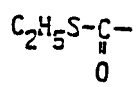 | $CH_3$ | H |
| 741 | 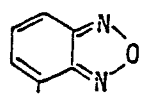 | $O_2N$ | $CH_3$ | H |
| 742 | 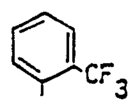 | $C_2H_5OOC$ | Cl | H |
| 743 | 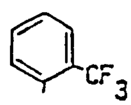 | $C_2H_5OOC$ | $(C_2H_5)_2NCH_2$ | H |
| 744 | 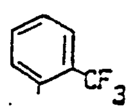 | $CH_3(CH_2)_{21}OOC$ | $CH_3$ | H |
| 745 | 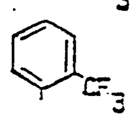 | 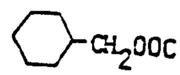 | $CH_3$ | H |
| 746 | 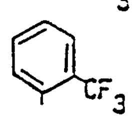 | $(CH_3)_3CCH_2OOC$ | $CH_3$ | H |

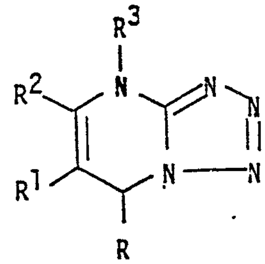

| No. | R | $R^1$ | $R^2$ | $R^3$ |
|---|---|---|---|---|
| 747 | 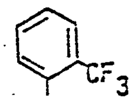 | 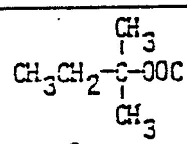 | $CH_3$ | H |
| 748 | 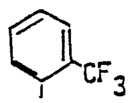 | 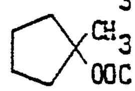 | $CH_3$ | H |
| 749 | 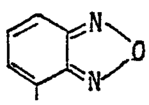 | $C_2H_5OOC$ | $CH_3$ | H |
| 750 | 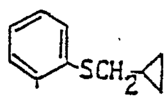 | $C_2H_5OOC$ | $CH_3$ | H |
| 751 | 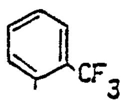 | 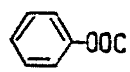 | $CH_3$ | H |
| 752 | 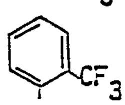 | $C_2H_5OOC$ | $(C_2H_5)_2N$ | H |
| 753 | 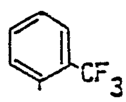 | $C_2H_5OOC$ | $CH_3$ | $CH_3$ |
| 754 | 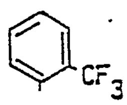 | $C_2H_5OOC$ | $CH_3$ | 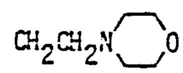 |
| 755 | 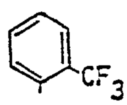 | 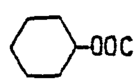 | $CH_3$ | H |
| 756 | 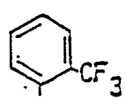 | $C_2H_5OOC$ | $CH_3$ | 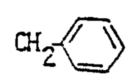 |
| 757 | 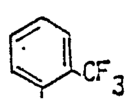 | $C_2H_5OOC$ | 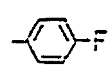 | H |

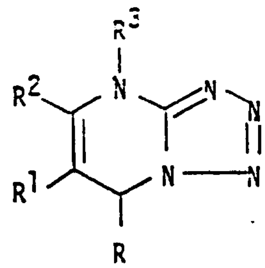

| No. | R | $R^1$ | $R^2$ | $R^3$ |
|---|---|---|---|---|
| 758 | 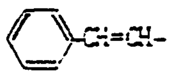 | $(CH_3)_2CHOOC$ | $CH_3$ | H |
| 759 | 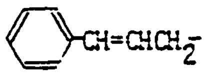 | $(CH_3)_3COOC$ | $CH_3$ | H |
| 760 | 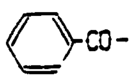 | $C_2H_5OOC$ | $CH_3$ | H |
| 761 | $CH_3CH=C(CH_3)-$ | $(CH_3)_2CHOOC$ | $CH_3$ | H |
| 762 | $CH\equiv CCH_2-$ | $(CH_3)_3COOC$ | $CH_3$ | H |
| 763 | 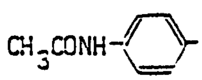 | $C_2H_5OOC$ | $CH_3$ | H |
| 764 | 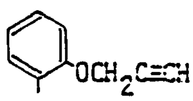 | $(CH_3)_2CHOOC$ | $CH_3$ | H |
| 765 | 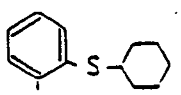 | $C_2H_5OOC$ | $CH_3$ | H |
| 766 | 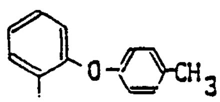 | $CH_3CH_2CH_2OOC$ | $CH_3$ | H |
| 767 | 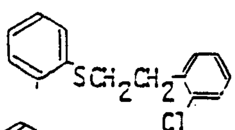 | $CH_3(CH_2)_3OOC$ | $CH_3$ | H |
| 768 | 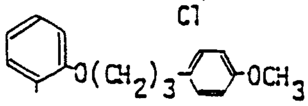 | 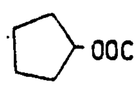 | $CH_3$ | H |

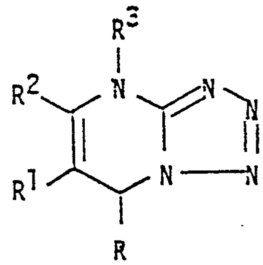

| No. | R | $R^1$ | $R^2$ | $R^3$ |
|---|---|---|---|---|
| 769 | 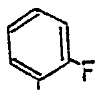 | $C_2H_5OOC$ | $H_2N$ | H |
| 770 | 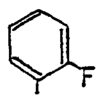 | $(CH_3)_2CHOOC$ | 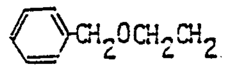 | H |
| 771 | 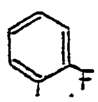 | $(CH_3)_2CHOOC$ | 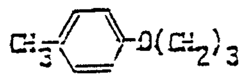 | H |
| 772 | 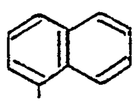 | $(CH_3)_2CHOOC$ | $CH_3$ | H |
| 773 | 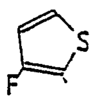 | $(CH_3)_2CHOOC$ | $CH_3$ | H |
| 774 | 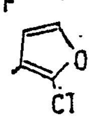 | $C_2H_5OOC$ | $CH_3$ | H |
| 775 | 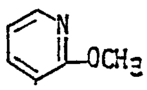 | $(CH_3)_2CHOOC$ | $CH_3$ | H |
| 776 | 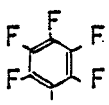 | $(CH_3)_2CHOOC$ | $CH_3$ | H |
| 777 | 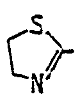 | $CH_3OOC$ | $CH_3$ | H |
| 778 | 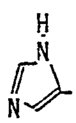 | 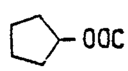 | $CH_3$ | H |
| 779 | 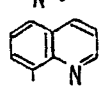 | $(CH_3)_2CHOOC$ | $CH_3$ | H |

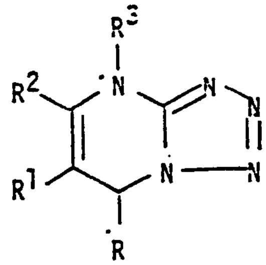

| No. | R | $R^1$ | $R^2$ | $R^3$ |
|---|---|---|---|---|
| 780 | 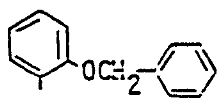 | 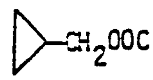 | $CH_3$ | H |
| 781 | 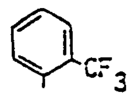 | $CH_3(CH_2)_7OOC$ | $CH_3$ | H |
| 782 | 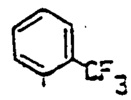 | $CH_3(CH_2)_5OOC$ | $CH_3$ | H |
| 783 | 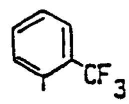 | $CH_2{=}CHCH_2OOC$ | $CH_3$ | H |
| 784 | 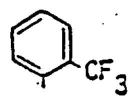 | 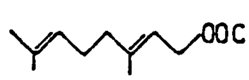 | $CH_3$ | H |
| 785 | 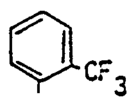 | $CH{\equiv}CCH_2OOC$ | $CH_3$ | H |
| 786 | 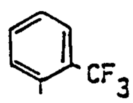 | $CH_3S(CH_2)_2OOC$ | $CH_3$ | H |
| 787 | 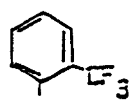 | $CH_3(CH_2)_4OOC$ | $CH_3$ | H |
| 788 | 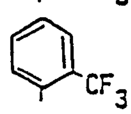 | 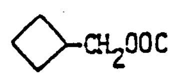 | $CH_3$ | H |
| 789 | 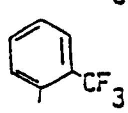 | 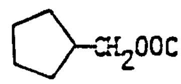 | $CH_3$ | H |
| 790 | 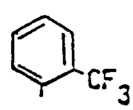 | 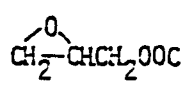 | $CH_3$ | H |

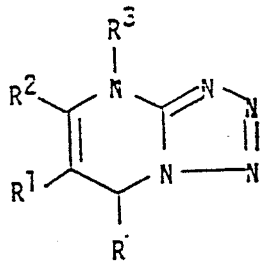

| No. | R | $R^1$ | $R^2$ | $R^3$ |
| --- | --- | --- | --- | --- |
| 791 | 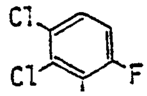 | $C_2H_5OOC$ | $CH_3$ | H |
| 792 | 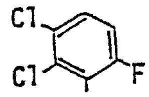 | $(CH_3)_2CHOOC$ | $CH_3$ | H |
| 793 | 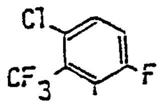 | $(CH_3)_2CHOOC$ | $CH_3$ | H |
| 794 | 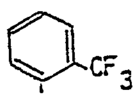 | $(ClCH_2)_2CHOOC$ | $CH_3$ | H |
| 795 | 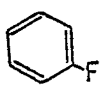 | $ClCH_2CH_2OOC$ | $CH_3$ | H |
| 796 | 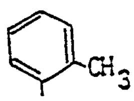 | 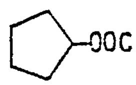 | $CH_3$ | H |
| 797 | 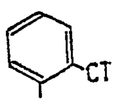 | 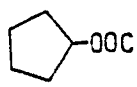 | $CH_3$ | H |
| 798 | 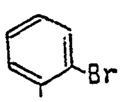 | 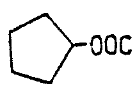 | $CH_3$ | H |
| 799 | 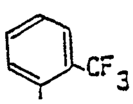 | $ClCH_2CH_2CH_2CH_2OOC$ | $CH_3$ | H |
| 800 | 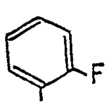 | $(ClCH_2)_2CHOOC$ | $CH_3$ | H |
| 801 | 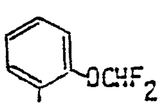 | $(CH_3)_3COOC$ | $CH_3$ | H |

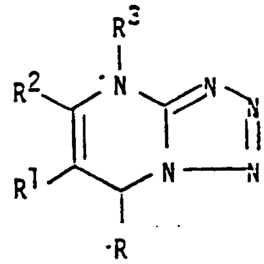

| No. | R | $R^1$ | $R^2$ | $R^3$ |
|---|---|---|---|---|
| 802 | 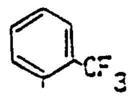 | $H_2NSC$ | $CH_3$ | H |
| 803 | 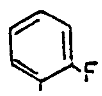 | 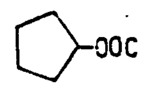 | $CH_3$ | H |
| 804 | 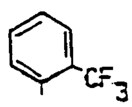 | $(CH_3)_2CHOOC$ | $CH_3$ | $CH_3$ |
| 805 | 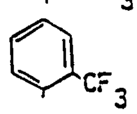 | $(CH_3)_2CHS\underset{O}{\underset{\|}{C}}$ | $CH_3$ | H |
| 806 | 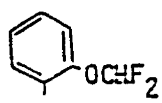 | $CH_3OOC$ | $CH_3$ | H |
| 807 | 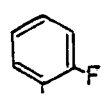 | $C_2H_5OOC$ | 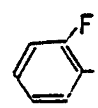 | H |
| 808 | 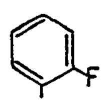 | $(CH_3)_2CHOOC$ | $CH_2N(C_2H_5)_2$ | H |
| 809 | 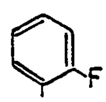 | 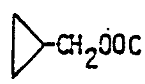 | $CH_3$ | H |
| 810 | 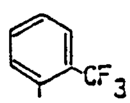 | $(CH_3)_2CHOOC$ | $CH_3$ | $COOC_2H_5$ |
| 811 | 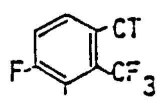 | $C_2H_5OOC$ | $CH_3$ | H |
| 812 | 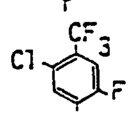 | $(CH_3)_2CHOOC$ | $CH_3$ | H |

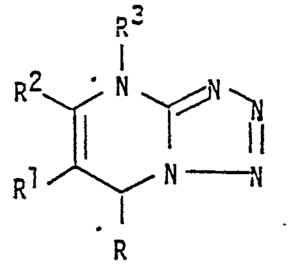

| No. | R | $R^1$ | $R^2$ | $R^3$ |
|---|---|---|---|---|
| 813 | 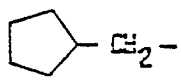 | $CH_3OOC$ | $CH_3$ | H |
| 814 | 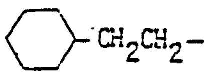 | 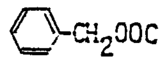 | $CH_3$ | H |
| 815 | 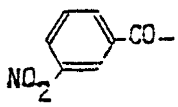 | $CH_3OCH_2CH_2OOC$ | $CH_3$ | H |
| 816 | 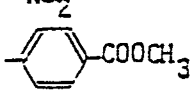 | $CH_3OOC$ | $CH_3$ | H |
| 817 | 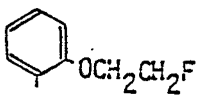 | $(CH_3)_2CHOOC$ | $CH_3$ | H |
| 818 | 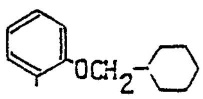 | 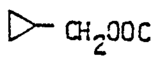 | $CH_3$ | H |
| 819 | 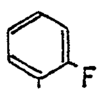 | $(CH_3)_2CHOSC$ | $CH_3$ | H |
| 820 | 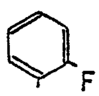 | $(CH_3)_2CHSSC$ | $CH_3$ | H |
| 821 | 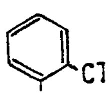 | 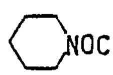 | $CH_3$ | H |
| 822 | 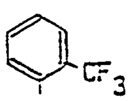 | $(CH_3)_2CHNHOC$ | $CH_3$ | H |
| 823 | 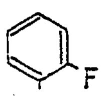 | $(C_2H_5)_2NOC$ | $CH_3$ | H |

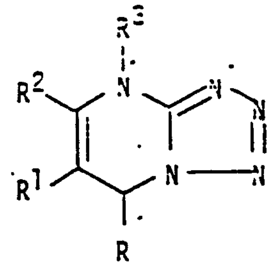

| No. | R | $R^1$ | $R^2$ | $R^3$ |
|---|---|---|---|---|
| 824 | 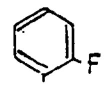 | $H_2NSC$ | $CH_3$ | H |
| 825 | 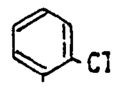 | 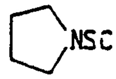 | $CH_3$ | H |
| 826 | 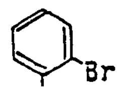 | $(CH_3)_3CNHSC$ | $CH_3$ | H |
| 827 | 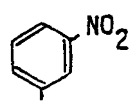 | 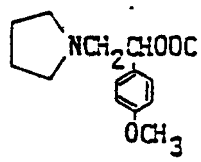 | $CH_3$ | H |
| 828 | 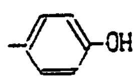 | 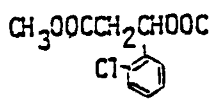 | $CH_3$ | H |
| 829 | 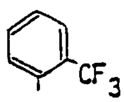 | 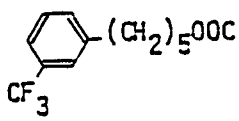 | $CH_3$ | H |
| 830 | 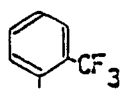 | $C_2H_5OOC$ | 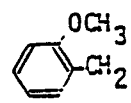 | H |
| 831 | 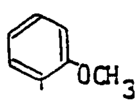 | $CH_3OOC$ | 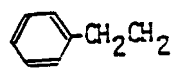 | H |
| 832 | 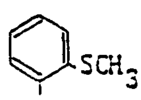 | $(CH_3)_2CHOOC$ | 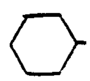 | H |
| 833 | 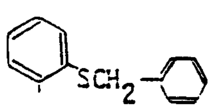 | $CH_3(CH_2)_2OOC$ | 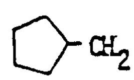 | H |
| 834 | 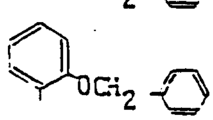 | $CH_3(CH_2)_3OOC$ | $H_2NCH_2CH_2$ | H |

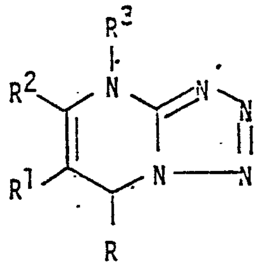

| No. | R | $R^1$ | $R^2$ | $R^3$ |
|---|---|---|---|---|
| 835 | 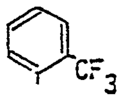 | 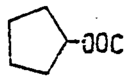 | $CH_3$ | 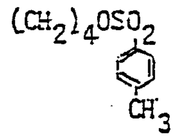 |
| 836 | 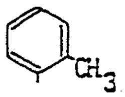 | $C_2H_5OOC$ | $CH_3$ | $CH_2CH_2OH$ |
| 837 | 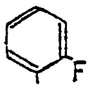 | $(CH_3)_2CHOOC$ | $CH_3$ | 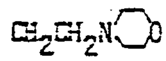 |

0217142

What is claimed is:

1. A polyazaheterocycle compound of the formula:

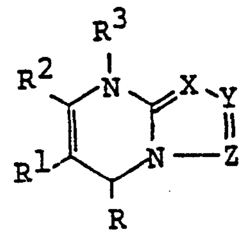

or a pharmaceutically acceptable salt thereof, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and $-X-R^4$ (wherein X is oxygen or sulfur and $R^4$ is $C_{1-8}$ alkyl, $C_{2-10}$ alkenyl, $C_{2-10}$ alkynyl, haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl, $C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) heteroaryl, (9) substituted heteroaryl, (10) aralkyl, (11) substituted aralkyl, (12) arylalkenyl, (13) substituted arylalkenyl, (14) cycloalkylalkyl, (15) cycloalkylalkenyl, (16) cycloalkylalkynyl, (17) heteroarylalkyl, (18) substituted heteroarylalkyl, (19) benzoyl, (20) substituted benzoyl,

$R^1$ is (1) hydrogen, (2) nitro, (3) cyano, (4) acyl,

(5) carbamoyl, (6) mono or di-substituted carbamoyl, (7) thiocarbamoyl, (8) mono or di-substituted thiocarbamoyl, (9) $C_{1-8}$ alkylthiocarbonyl, (10) $C_{1-8}$ alkoxythiocarbonyl, (11) $C_{1-8}$ alkyldithiocarbonyl, or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl-$C_{1-8}$ alkyl, haloalkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, heteroaryl-$C_{1-8}$ alkyl, alkylthio-$C_{1-8}$ alkyl, alkoxycarbonyl-$C_{1-8}$ alkyl, 2,3-epoxypropyl, $-(CH_2)m-$ 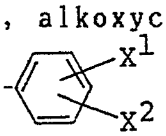

(wherein $X^1$ and $X^2$ are same or different and each is hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or $-(CH_2)pCH(CH_2)nR^7$ (with $R^6$ on the CH)

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$

alkoxy, heteroaryl or $C_{1-8}$ alkoxycarbonyl, and each of n and p is 0 or 1 to 4) ;

$R^2$ is (1) halogen, (2) cyano, (3) $C_{1-8}$ alkyl, (4) haloalkyl, (5) hydroxyalkyl, (6) acyloxyalkyl, (7) alkoxyalkyl, (8) dialkoxyalkyl, (9) aminoalkyl, (10) acylaminoalkyl, (11) formyl, (12) carbamoyloxyalkyl, (13) thiocarbamoyloxyalkyl, (14) amino, (15) substituted amino, (16) aralkyloxyalkyl, (17) aryloxyalkyl, (18) hydroxyiminomethyl, (19) hydroxyalkoxyalkyl, (20) aminoalkoxyalkyl, (21) substituted aminoalkoxyalkyl, (22) $C_{3-8}$ cycloalkyl, (23) cycloalkylalkyl, (24) phenyl, (25) substituted phenyl by at least one substituent selected from halogen, $C_{1-8}$ alkyl, $C_{1-8}$ alkoxy, nitro, cyano, hydroxy, amino and trifluoromethyl, (26) aralkyl, or (27) substituted aralkyl by at least one substituent selected from halogen, $C_{1-8}$ alkyl, $C_{1-8}$ alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl;

and at least one of X, Y and Z is N, and other(s) is (are) C-$R^8$, wherein $R^8$ is (1) hydrogen, (2) halogen, (3)nitro, (4) amino, (5) acylamino, (6) $C_{1-8}$ alkyl, (7) $C_{3-8}$ cycloalkyl, (8) cyano, (9) carboxyl, (10) alkoxycarbonyl, (11) carbamoyl, (12) mono or di-substituted carbamoyl, (13) formyl, (14) hydroxyalkyl, (15) aminoalkyl, (16) acyl, (17) haloalkyl, (18) $C_{2-10}$ alkenyl, (19) $C_{2-10}$ alkynyl, (20) cycloalkylalkyl, (21) alkoxyalkyl, (22) acyloxyalkyl, (23) cyanoalkyl, (24) nitroalkyl, (25) carbamoyloxyalkyl, (26) thiocarbamoyloxyalkyl, (27) aralkyloxy, (28) aralkylthio, (29) alkylthio, (30) $C_{1-8}$ alkoxy, or (31) aralkyl; with the following provisos: (1) when two of X, Y and Z are C-$R^8$, two of $R^8$ are the same or different and each is as defined above, (2) when X and Z are N, $R^2$ is not halogen, amino or substituted amino, (3) when only X is N, $R^8$ is not nitro, amino, $C_{1-8}$ alkyl, carboxyl, formyl, hydroxylalkyl, aminoalkyl, alkoxyalkyl, acyloxyalkyl, nitroalkyl, aralkyloxy, aralkylthio, alkylthio or $C_{1-8}$ alkoxy, and (4) each of R, $R^1$, $R^2$, $R^3$ and $R^8$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and -X-$R^4$ (wherein X is oxygen or sulfur, and $R^4$ is $C_{1-4}$ alkyl, aralkyl), $C_{3-8}$ cycloalkyl, furyl,

thienyl or pyridyl;

$R^1$: hydrogen, cyano, acyl, carbamoyl or $-COOR^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$ alkyl, heteroaryl-$C_{1-4}$ alkyl, $-(CH_2)m-$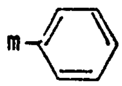 (wherein m is 1 to 4) or $-(CH_2)pCH(CH_2)nR^7$ (wherein $R^6$ is phenyl
$|$
$R^6$

or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen;

$R^8$: hydrogen.

2. The compound of claim 1 represented by the formula:

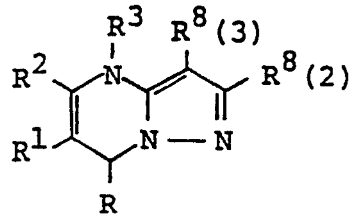

or a pharmaceutically acceptable salt thereof, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and $-X-R^4$ (wherein X is oxygen or

sulfur and $R^4$ is $C_{1-8}$ alkyl, $C_{2-10}$ alkenyl, $C_{2-10}$ alkynyl, haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl, $C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) heteroaryl, (9) substituted heteroaryl, (10) aralkyl, (11) substituted aralkyl, (12) arylalkenyl, (13) substituted arylalkenyl, (14) cycloalkylalkyl, (15) cycloalkylalkenyl, (16) cycloalkylalkynyl, (17) heteroarylalkyl, (18) substituted heteroarylalkyl, (19) benzoyl, (20) substituted benzoyl, $R^1$ is (1) hydrogen, (2) nitro, (3) cyano, (4) acyl, (5) carbamoyl, (6) mono or di-substituted carbamoyl, (7) thiocarbamoyl, (8) mono or di-substituted thiocarbamoyl, (9) $C_{1-8}$ alkylthiocarbonyl, (10) $C_{1-8}$ alkoxythiocarbonyl, (11) $C_{1-8}$ alkyldithiocarbonyl, or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl-$C_{1-8}$ alkyl, haloalkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, hetero-

aryl-$C_{1-8}$ alkyl, alkylthio-$C_{1-8}$ alkyl, alkoxycarbonyl-$C_{1-8}$ alkyl, 2,3-epoxypropyl, $-(CH_2)_m-$ 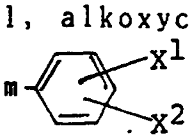

(wherein $X^1$ and $X^2$ are same or different and each is hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or $-(CH_2)_pCH(R^6)(CH_2)_nR^7$

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$ alkoxy, heteroaryl or $C_{1-8}$ alkoxycarbonyl, and each of n and p is 0 or 1 to 4) ;

$R^2$ is (1) halogen, (2) cyano, (3) $C_{1-8}$ alkyl, (4) haloalkyl, (5) hydroxyalkyl, (6) acyloxyalkyl, (7) alkoxyalkyl, (8) dialkoxyalkyl, (9) aminoalkyl, (10) acylaminoalkyl, (11) formyl, (12) carbamoyloxyalkyl, (13) thiocarbamoyloxyalkyl, (14) amino, (15) substituted amino, (16) aralkyloxyalkyl, (17) aryloxyalkyl, (18) hydroxyiminomethyl, (19) hydroxyalkoxyalkyl, (20) aminoalkoxyalkyl, (21) substituted aminoalkoxyalkyl, (22) $C_{3-8}$ cycloalkyl, (23) cycloalkylalkyl, (24) phenyl, (25) substituted

phenyl by at least one substituent selected from halogen, $C_{1-8}$ alkyl, $C_{1-8}$ alkoxy, nitro, cyano, hydroxy, amino and trifluoromethyl, (26) aralkyl, or (27) substituted aralkyl by at least one substituent selected from halogen, $C_{1-8}$ alkyl, $C_{1-8}$ alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl;

and $R^8(2)$ and $R^8(3)$ are the same or different and each is (1) hydrogen, (2) halogen, (3) nitro, (4) amino, (5) acylamino, (6) $C_{1-8}$ alkyl, (7) $C_{3-8}$ cycloalkyl, (8) cyano, (9) carboxyl, (10) alkoxycarbonyl, (11) carbamoyl, (12) mono or di-substituted carbamoyl, (13) formyl, (14) hydroxyalkyl, (15) aminoalkyl, (16) acyl, (17) haloalkyl, (18) $C_{2-10}$ alkenyl, (19) $C_{2-10}$ alkynyl, (20) cycloalkylalkyl, (21) alkoxyalkyl, (22) acyloxyalkyl, (23) cyanoalkyl, (24) nitroalkyl, (25) carbamoyloxyalkyl, (26) thiocarbamoyloxyalkyl, (27) aralkyloxy, (28) aralkylthio, (29) alkylthio, (30) $C_{1-8}$ alkoxy, or (31) aralkyl;

with the following proviso that each of R, $R^1$, $R^2$, $R^3$,

$R^8(2)$ and $R^8(3)$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and $-X-R^4$ (wherein X is oxygen or sulfur, and $R^4$ is $C_{1-4}$ alkyl, aralkyl), $C_{3-8}$ cycloalkyl, furyl, thienyl or pyridyl;

$R^1$: hydrogen, cyano, acyl, carbamoyl or $-COOR^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$ alkyl, heteroaryl-$C_{1-4}$ alkyl, $-(CH_2)m-$ 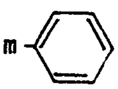 (wherein m is 1 to 4) or $-(CH_2)p\underset{R^6}{\underset{|}{CH}}(CH_2)nR^7$ (wherein $R^6$ is phenyl or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen;

$R^8(2)$ : hydrogen;

$R^8(3)$ : hydrogen.

3. The compound of claim 1 represented by the formula:

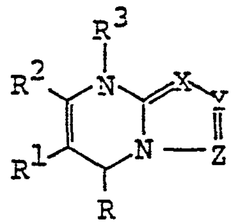

or a pharmaceutically acceptable salt thereof, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and -X-$R^4$ (wherein X is oxygen or sulfur and $R^4$ is $C_{1-8}$ alkyl, $C_{2-10}$ alkenyl, $C_{2-10}$ alkynyl, haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl, $C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) heteroaryl, (9) substituted heteroaryl, (10) aralkyl, (11) substituted aralkyl, (12) arylalkenyl, (13) substituted arylalkenyl, (14) cycloalkylalkyl, (15) cycloalkylalkenyl, (16) cycloalkylalkynyl, (17) heteroarylalkyl, (18) substituted heteroarylalkyl, (19) benzoyl, (20) substituted benzoyl,

$R^1$ is (1) hydrogen, (2) nitro, (3) cyano, (4) acyl, (5) carbamoyl, (6) mono or di-substituted carbamoyl, (7) thiocarbamoyl, (8) mono or di-substituted thiocarba-

moyl, (9) $C_{1-8}$ alkylthiocarbonyl, (10) $C_{1-8}$ alkoxythiocarbonyl, (11) $C_{1-8}$ alkyldithiocarbonyl, or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl-$C_{1-8}$ alkyl, haloalkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, heteroaryl-$C_{1-8}$ alkyl, alkylthio-$C_{1-8}$ alkyl, alkoxycarbonyl-$C_{1-8}$ alkyl, 2,3-epoxypropyl, $-(CH_2)m-$ 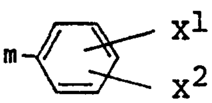

(wherein $X^1$ and $X^2$ are same or different and each is hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or $-(CH_2)pCH(CH_2)nR^7$ (with $R^6$ on the CH)

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$ alkoxy, heteroaryl or $C_{1-8}$ alkoxycarbonyl, and each of n and p is 0 or 1 to 4) ;

$R^2$ is (1) halogen, (2) cyano, (3) $C_{1-8}$ alkyl, (4) haloalkyl, (5) hydroxyalkyl, (6) acyloxyalkyl, (7) alkoxyalkyl, (8) dialkoxyalkyl, (9) aminoalkyl, (10) acylaminoalkyl, (11) formyl, (12) carbamoyloxyalkyl, (13) thiocarbamoyloxyalkyl, (14) amino, (15) substituted amino, (16) aralkyloxyalkyl, (17) aryloxyalkyl, (18) hydroxyiminomethyl, (19) hydroxyalkoxyalkyl, (20) aminoalkoxyalkyl, (21) substituted aminoalkoxyalkyl, (22) $C_{3-8}$ cycloalkyl, (23) cycloalkylalkyl, (24) phenyl, (25) substituted phenyl by at least one substituent selected from halogen, $C_{1-8}$ alkyl, $C_{1-8}$ alkoxy, nitro, cyano, hydroxy, amino and trifluoromethyl, (26) aralkyl, or (27) substituted aralkyl by at least one substituent selected from halogen, $C_{1-8}$ alkyl, $C_{1-8}$ alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl;

and one of X and Y is N, and the other and Z are C-$R^8$, wherein $R^8$ is (1) hydrogen, (2) halogen, (3) nitro, (4)

amino, (5) acylamino, (6) $C_{1-8}$ alkyl, (7) $C_{3-8}$ cycloalkyl, (8) cyano, (9) carboxyl, (10) alkoxycarbonyl, (11) carbamoyl, (12) mono or di-substituted carbamoyl, (13) formyl, (14) hydroxyalkyl, (15) aminoalkyl, (16) acyl, (17) haloalkyl, (18) $C_{2-10}$ alkenyl, (19) $C_{2-10}$ alkynyl, (20) cycloalkylalkyl, (21) alkoxyalkyl, (22) acyloxyalkyl, (23) cyanoalkyl, (24) nitroalkyl, (25) carbamoyloxyalkyl, (26) thiocarbamoyloxyalkyl, (27) aralkyloxy, (28) aralkylthio, (29) alkylthio, (30) $C_{1-8}$ alkoxy, or (31) aralkyl; with the following provisos: (1) two of $R^8$ are the same or different and each is as defined above, (2) when X is N, $R^8$ is not nitro, amino, $C_{1-8}$ alkyl, carboxyl, formyl, hydroxylalkyl, aminoalkyl, alkoxyalkyl, acyloxyalkyl, nitroalkyl, aralkyloxy, aralkylthio, alkylthio or $C_{1-8}$ alkoxy, and (3) each of R, $R^1$, $R^2$, $R^3$ and $R^8$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and -X-$R^4$ (wherein X is oxygen or sulfur, and $R^4$ is $C_{1-4}$ alkyl, aralkyl), $C_{3-8}$ cycloalkyl, furyl, thienyl or pyridyl;

$R^1$: hydrogen, cyano, acyl, carbamoyl or -COO$R^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$

alkyl, heteroaryl-$C_{1-4}$ alkyl, $-(CH_2)_m-$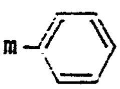 (wherein m is 1 to 4) or $-(CH_2)_p\underset{R^6}{\underset{|}{C}}H(CH_2)_nR^7$ (wherein $R^6$ is phenyl or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen;

$R^8$: hydrogen.

4. The compound of claim 1 represented by the formula:

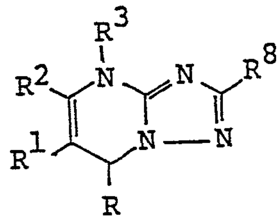

or a pharmaceutically acceptable salt thereof, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and $-X-R^4$ (wherein X is oxygen or sulfur and $R^4$ is $C_{1-8}$ alkyl, $C_{2-10}$ alkenyl, $C_{2-10}$ alkynyl, haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl,

$C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) heteroaryl, (9) substituted heteroaryl, (10) aralkyl, (11) substituted aralkyl, (12) arylalkenyl, (13) substituted arylalkenyl, (14) cycloalkylalkyl, (15) cycloalkylalkenyl, (16) cycloalkylalkynyl, (17) heteroarylalkyl, (18) substituted heteroarylalkyl, (19) benzoyl, (20) substituted benzoyl, $R^1$ is (1) hydrogen, (2) nitro, (3) cyano, (4) acyl, (5) carbamoyl, (6) mono or di-substituted carbamoyl, (7) thiocarbamoyl, (8) mono or di-substituted thiocarbamoyl, (9) $C_{1-8}$ alkylthiocarbonyl, (10) $C_{1-8}$ alkoxythiocarbonyl, (11) $C_{1-8}$ alkyldithiocarbonyl, or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl-$C_{1-8}$ alkyl, haloalkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, heteroaryl-$C_{1-8}$ alkyl, alkylthio-$C_{1-8}$ alkyl, alkoxycarbonyl-$C_{1-8}$ alkyl, 2,3-epoxypropyl, $-(CH_2)m-$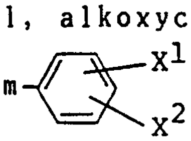

(wherein $X^1$ and $X^2$ are same or different and each is

hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or $-(CH_2)_p\underset{\displaystyle R^6}{\underset{|}{CH}}(CH_2)_nR^7$

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$ alkoxy, heteroaryl or $C_{1-8}$ alkoxycarbonyl, and each of n and p is 0 or 1 to 4) ;

$R^2$ is (1) cyano, (2) $C_{1-8}$ alkyl, (3) haloalkyl, (4) hydroxyalkyl, (5) acyloxyalkyl, (6) alkoxyalkyl, (7) dialkoxyalkyl, (8) aminoalkyl, (9) acylaminoalkyl, (10) formyl, (11) carbamoyloxyalkyl, (12) thiocarbamoyloxyalkyl, (13) aralkyloxyalkyl, (14) aryloxyalkyl, (15) hydroxyiminomethyl, (16) hydroxyalkoxyalkyl, (17) aminoalkoxyalkyl, (18) substituted aminoalkoxyalkyl, (19) $C_{3-8}$ cycloalkyl, (20) cycloalkylalkyl, (21) phenyl, (22) substituted phenyl by at least one substituent selected from halogen, $C_{1-8}$ alkyl, $C_{1-8}$ alkoxy, nitro, cyano, hydroxy, amino and trifluoromethyl, (23) aralkyl, or (24) substituted aralkyl by at least one substituent selected from halogen, alkyl, alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl;

$R^8$ is (1) hydrogen, (2) halogen, (3) nitro, (4) amino, (5) acylamino, (6) $C_{1-8}$ alkyl, (7) $C_{3-8}$ cycloalkyl, (8) cyano, (9) carboxyl, (10) alkoxycarbonyl, (11) carbamoyl, (12) mono or di-substituted carbamoyl, (13) formyl, (14) hydroxyalkyl, (15) aminoalkyl, (16) acyl, (17) haloalkyl, (18) $C_{2-10}$ alkenyl, (19) $C_{2-10}$ alkynyl, (20) cycloalkylalkyl, (21) alkoxyalkyl, (22) acyloxyalkyl, (23) cyanoalkyl, (24) nitroalkyl, (25) carbamoyloxyalkyl, (26) thiocarbamoyloxyalkyl, (27) aralkyloxy, (28) aralkylthio, (29) alkylthio, (30) $C_{1-8}$ alkoxy, or (31) aralkyl;

with the following proviso that each of R, $R^1$, $R^2$, $R^3$ and $R^7$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and $-X-R^4$ (wherein X is oxygen or sulfur, and $R^4$ is $C_{1-4}$ alkyl, aralkyl), $C_{3-8}$ cycloalkyl, furyl,

thienyl or pyridyl;

$R^1$: hydrogen, cyano, acyl, carbamoyl or $-COOR^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$ alkyl, heteroaryl-$C_{1-4}$ alkyl, $-(CH_2)m-$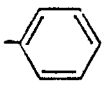 (wherein m is 1 to 4) or $-(CH_2)p\underset{R^6}{\underset{|}{CH}}(CH_2)nR^7$ (wherein $R^6$ is phenyl or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen;

$R^8$: hydrogen.

5. The compound of claim 1 represented by the formula:

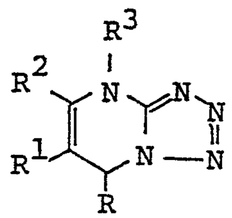

or a pharmaceutically acceptable salt thereof, wherein R is (1) hydrogen, (2) $C_{1-8}$ alkyl, (3) $C_{2-10}$ alkenyl, (4) $C_{2-10}$ alkynyl, (5) aryl, (6) substituted aryl by at least one substituent selected from halogen, amino, nitro, cyano, $C_{1-8}$ alkyl, trifluoromethyl, hydroxy, acylamino, acyl, alkoxycarbonyl and $-X-R^4$ (wherein X is oxygen or

sulfur and $R^4$ is $C_{1-8}$ alkyl, $C_{2-10}$ alkenyl, $C_{2-10}$ alkynyl, haloalkyl, $C_{3-8}$ cycloalkyl, cycloalkylalkyl, phenyl, substituted phenyl by at least one substituent selected from halogen, nitro, cyano, $C_{1-8}$ alkyl, trifluoromethyl, $C_{1-8}$ alkoxy and hydroxy, aralkyl or substituted aralkyl by on the benzene ring at least one substituent selected from halogen, nitro, cyano, $C_{1-8}$ alkyl, trifluoromethyl, $C_{1-8}$ alkoxy and hydroxy), (7) $C_{3-8}$ cycloalkyl, (8) heteroaryl, (9) substituted heteroaryl, (10) aralkyl, (11) substituted aralkyl, (12) arylalkenyl, (13) substituted arylalkenyl, (14) cycloalkylalkyl, (15) cycloalkylalkenyl, (16) cycloalkylalkynyl, (17) heteroarylalkyl, (18) substituted heteroarylalkyl, (19) benzoyl, (20) substituted benzoyl, $R^1$ is (1) hydrogen, (2) nitro, (3) cyano, (4) acyl, (5) carbamoyl, (6) mono or di-substituted carbamoyl, (7) thiocarbamoyl, (8) mono or di-substituted thiocarbamoyl, (9) $C_{1-8}$ alkylthiocarbonyl, (10) $C_{1-8}$ alkoxythiocarbonyl, (11) $C_{1-8}$ alkyldithiocarbonyl, or (12) $-COOR^5$ wherein $R^5$ is hydrogen, $C_{1-25}$ alkyl, $C_{2-10}$ alkenyl, $C_{2-10}$ alkynyl, $C_{3-8}$ cycloalkyl, substituted $C_{3-8}$ cycloalkyl by $C_{1-8}$ alkyl, $C_{3-8}$ cycloalkyl-$C_{1-8}$ alkyl, haloalkyl, $C_{1-8}$ alkoxy-$C_{1-8}$ alkyl, cyano-$C_{1-8}$ alkyl, nitro-$C_{1-8}$ alkyl, hydroxy-$C_{1-8}$ alkyl, acyloxy-$C_{1-8}$ alkyl, amino-$C_{1-8}$ alkyl, substituted amino-$C_{1-8}$ alkyl, amino-$C_{1-8}$ alkoxy-$C_{1-8}$ alkyl, substituted amino-$C_{1-8}$ alkoxy-$C_{1-8}$ alkyl, hetero-

aryl-$C_{1-8}$ alkyl, alkylthio-$C_{1-8}$ alkyl, alkoxycarbonyl-$C_{1-8}$ alkyl, 2,3-epoxypropyl, $-(CH_2)m-$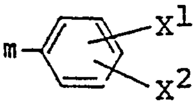

(wherein $X^1$ and $X^2$ are same or different and each is hydrogen, halogen, $C_{1-8}$ alkyl, $C_{1-8}$ alkoxy, nitro, cyano, trifluoromethyl, hydroxy, amino or mono or di-substituted amino, and m is 0 or 1 to 5) or $-(CH_2)pCH(R^6)(CH_2)nR^7$

(wherein $R^6$ is phenyl, substituted phenyl by at least one substituent selected from halogen, $C_{1-8}$ alkyl, $C_{1-8}$ alkoxy, nitro, cyano, trifluoromethyl, hydroxy, amino, acylamino and acyl, heteroaryl or substituted heteroaryl, $R^7$ is cyano, hydroxy, nitro, $C_{1-8}$ alkoxy, amino, substituted amino, acyloxy, amino-$C_{1-8}$ alkoxy, substituted amino-$C_{1-8}$ alkoxy, heteroaryl or $C_{1-8}$ alkoxycarbonyl, and each of n and p is 0 or 1 to 4) ;

$R^2$ is (1) halogen, (2) cyano, (3) $C_{1-8}$ alkyl, (4) haloalkyl, (5) hydroxyalkyl, (6) acyloxyalkyl, (7) alkoxyalkyl, (8) dialkoxyalkyl, (9) aminoalkyl, (10) acylaminoalkyl, (11) formyl, (12) carbamoyloxyalkyl, (13) thiocarbamoyloxyalkyl, (14) amino, (15) substituted amino, (16) aralkyloxyalkyl, (17) aryloxyalkyl, (18) hydroxyiminomethyl, (19) hydroxyalkoxyalkyl, (20) aminoalkoxyalkyl, (21) substituted aminoalkoxyalkyl, (22) $C_{3-8}$ cycloalkyl, (23) cycloalkylalkyl, (24) phenyl, (25) substituted

phenyl by at least one substituent selected from halogen, $C_{1-8}$ alkyl, $C_{1-8}$ alkoxy, nitro, cyano, hydroxy, amino and trifluoromethyl, (26) aralkyl, or (27) substituted aralkyl by at least one substituent selected from halogen, $C_{1-8}$ alkyl, $C_{1-8}$ alkoxy, nitro and trifluoromethyl;

$R^3$ is (1) hydrogen, (2) $C_{1-8}$ alkyl, (3) haloalkyl, (4) hydroxyalkyl, (5) alkoxyalkyl, (6) alkoxycarbonyl, (7) aminoalkyl, (8) substituted aminoalkyl, (9) acyl, (10) heteroaroyl, (11) arenesulfonyloxyalkyl, (12) alkanesulfonyloxyalkyl, (13) aralkyl, or (14) substituted aralkyl by on the benzene ring at least one substituent selected from halogen, $C_{1-8}$ alkyl, $C_{1-8}$ alkoxy, cyano, nitro and trifluoromethyl;

with the following proviso that each of R, $R^1$, $R^2$ and $R^3$ is not the following substituents at the same time;

R : hydrogen, $C_{1-5}$ alkyl, phenyl, substituted phenyl by one or two substituents selected from halogen, amino, nitro, cyano, $C_{1-4}$ alkyl, trifluoromethyl, hydroxy, acyl and $-X-R^4$ (wherein X is oxygen or sulfur, and $R^4$ is $C_{1-4}$ alkyl, aralkyl), $C_{3-8}$ cycloalkyl, furyl, thienyl or pyridyl;

$R^1$: hydrogen, cyano, acyl, carbamoyl or $-COOR^5$ wherein $R^5$ is hydrogen, $C_{1-4}$ alkyl, $C_{1-4}$ alkoxy-$C_{1-4}$ alkyl, cyano-$C_{1-4}$ alkyl, nitro-$C_{1-4}$ alkyl, substituted amino-$C_{1-4}$ alkyl, heteroaryl-$C_{1-4}$ alkyl, $-(CH_2)m-$ 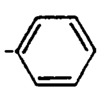 (wherein m

is 1 to 4) or $-(CH_2)_pCH(R^6)(CH_2)_nR^7$ (wherein $R^6$ is phenyl or heteroaryl, $R^7$ is cyano, nitro, $C_{1-4}$ alkoxy, substituted amino or heteroaryl, and each of n and p is 0 or 1 to 4);

$R^2$: cyano, $C_{1-4}$ alkyl, haloalkyl, hydroxyalkyl, acyloxyalkyl, alkoxyalkyl, dialkoxyalkyl, formyl, carbamoyloxyalkyl, thiocarbamoyloxyalkyl or hydroxyiminomethyl;

$R^3$: hydrogen.

6. A compound of claim 1 selected from 6-cyclopropylmethyloxycarbonyl-5-methyl-7-(2-trifluoromethylphenyl)-4,7-dihydropyrazolo[1,5-a]pyrimidine, 3-cyano-6-cyclopropylmethyloxycarbonyl-5-methyl-7-(2-trifluoromethylphenyl)-4,7-dihydropyrazolo[1,5-a]pyrimidine, 3-cyano-6-ethoxycarbonyl-5-methyl-7-(2-trifluoromethylphenyl)-4,7-dihydropyrazolo[1,5-a]pyrimidine, 6-cyclopentyloxycarbonyl-5-methyl-7-(2-trifluoromethylphenyl)-4,7-dihydropyrazolo[1,5-a]pyrimidine, 3-chloro-6-ethoxycarbonyl-5-methyl-7-(2-trifluoromethylphenyl)-4,7-dihydropyrazolo[1,5-a]pyrimidine, 7-(2-difluoromethoxyphenyl)-6-ethoxycarbonyl-5-methyl-4,7-dihydropyrazolo[1,5-a]pyrimidine, 6-[2-(3,4-dimethoxyphenyl)ethoxy]carbonyl-5-methyl-7-(2-trifluoromethylphenyl)-4,7-dihydropyrazolo[1,5-a]pyrimidine, 5-methyl-6-(3-pentyloxycarbonyl)-7-(2-trifluoromethylphenyl)-4,7-dihydropyrazolo[1,5-a]pyrimidine, 6-cyclopentyloxycarbonyl-7-(2-fluorophenyl)-5-methyl-4,7-dihydropyrazolo[1,5-a]pyrimidine, 3-cyano-7-(2-fluorophenyl)-6-isopropoxycarbonyl-5-methyl-4,7-dihydropyrazolo[1,5-a]pyrimidine, 3-cyano-7-(2,3-difluorophenyl)-6-isopropoxycarbonyl-5-methyl-4,7-dihydropyrazolo[1,5-a]pyrimidine, 3-cyano-6-isopropoxycarbonyl-5-methyl-7-(2-nitrophenyl)-4,7-dihydropyrazolo[1,5-a]pyrimidine and 3-cyano-6-isopropoxycarbonyl-5-methyl-7-(3-nitrophenyl)-4,7-dihydropyrazolo[1,5-a]pyrimidine.

7. A method for preparing a polyazaheterocycle compound of the formula:

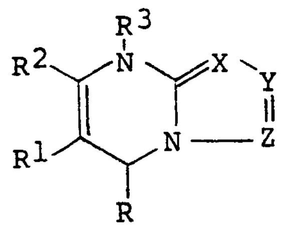

(I)

wherein each of R, $R^1$, $R^2$, $R^3$, X, Y and Z is as defined in claim 1, which comprises by any of the following processes of:

(a) subjecting a compound of the formula:

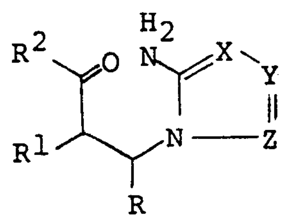

(II)

to dehydration condensation;

(b) subjecting a compound of the formula:

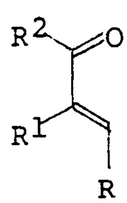

(III)

to condensation with a compound of the formula:

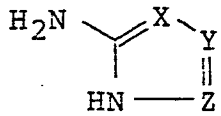

(IV)

(c) subjecting a compound of the formula:

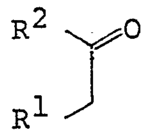

(V)

to dehydration condensation with a compound of the formula:

R-CHO (VI)

and then subjecting the compound obtained to condensation with a compound of the formula (IV);

(d) reacting a compound of the formula:

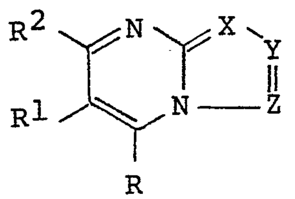

(VII)

with hydrogen gas or a complex metal hydride; and

(e) reacting a compound of the formula:

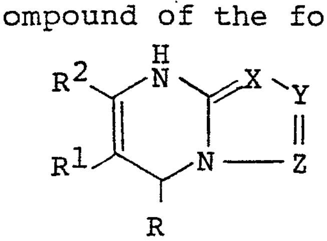

(VIII)

with a compound of the formula:

$R^{3'}$-W (IX)

wherein $R^{3'}$ is other than hydrogen in $R^3$, and W is halogen or active ester group.

8. A pharmaceutical composition comprising a therapeutically effective amount of the compound of claim 1 and a pharmaceutically acceptable carrier thereof.